(19) Europäisches Patentamt
European Patent Office
Office européen des brevets

(11) Numéro de publication : **0 376 776 B1**

# (12) FASCICULE DE BREVET EUROPEEN

(45) Date de publication du fascicule du brevet : **23.09.92 Bulletin 92/39**

(51) Int. Cl.[5] : **A61K 7/13**, C07C 323/23, C07C 319/20

(21) Numéro de dépôt : **89403358.8**

(22) Date de dépôt : **05.12.89**

## (54) Procédé de teinture de fibres kératiniques avec un hydroxyindole, associé à un dérivé quinonique et nouvelles 1,4-benzoquinones.

(30) Priorité : **06.12.88 LU 87403**

(43) Date de publication de la demande : **04.07.90 Bulletin 90/27**

(45) Mention de la délivrance du brevet : **23.09.92 Bulletin 92/39**

(84) Etats contractants désignés : **AT BE CH DE ES FR GB GR IT LI NL**

(56) Documents cités :
**FR-A- 2 234 277**
**FR-A- 2 626 173**
**GB-A- 2 187 456**
**US-A- 3 328 301**
**J. ELECTROCHEM. SOC., vol. 118, no. 1, janvier 1971, pages 57-63, Manchester, New-Hamp., US; J.L. HUNTINGTON et al.: "Theeffect of molecular structure on the electrode kinetics of aminoquinones and quinone thioethers"**
**J. ORG. CHEM., vol. 50, 1985, pages 1963-1969, American Chemical Society, US; K. HAYAKAWA et al.: "A useful synthon approachto bicyclic enols: Acid-catalyzed and base-catalyzed rearrangements of Diels-Alder adducts of 2-methoxy-5-methyl-1,4-benzoquinone"**

(56) Documents cités :
**TETRAHEDRON LETTERS, vol. 25, no. 42, 1984, pages 4833-4836, Pergamon Press Ltd., GB; R.N. WARRENER et al.: "Linearannelation of tricyclic quinones by site selective reaction with dienes"**
**TETRAHEDRON, vol. 43, no. 12, 1987, pages 2749-2754, Pergamon Journals Ltd, GB; A. NAPOLITANO et al.: "A reinvestigation ofthe reactions between 5,6-dihydroxyindoles and quinones"**

(73) Titulaire : **L'OREAL**
**14, Rue Royale**
**F-75008 Paris (FR)**

(72) Inventeur : **De Labbey, Arnaud**
**9, rue Charles Dordain**
**F-93600 Aulnay-sous-Bois (FR)**
Inventeur : **Baudry, Alain**
**19 Villa du Buisson Ardent**
**F-95500 Gonesse (FR)**
Inventeur : **Bauer, Daniel**
**8bis Allée de la Fontaine**
**F-93340 Le Raincy (FR)**
Inventeur : **Bore, Pierre**
**197 avenue Daniel Perdrigé**
**F-93370 Montfermeil (FR)**

(74) Mandataire : **Casalonga, Axel et al**
**BUREAU D.A. CASALONGA - JOSSE**
**Morassistrasse 8**
**W-8000 München 5 (DE)**

EP 0 376 776 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à un nouveau procédé de coloration des fibres kératiniques et plus particulièrement des fibres kératiniques humaines, telles que les cheveux, mettant en oeuvre au moins un hydroxyindole, associé à au moins un dérivé quinonique ainsi qu'à de nouvelles 1,4-benzoquinones.

On a déjà proposé dans le passé de teindre les fibres kératiniques et en particulier les cheveux, à l'aide de dérivés hydroxylés de l'indole et plus particulièrement dans les brevets français de la demanderesse n° 1.133.594, 1.166.172, 2.390.158 et la demande de brevet français 2.536.993 qui proposent des procédés de teinture à l'aide du 5,6-dihydroxyindole en utilisant des cations métalliques jouant le rôle de promoteur de la mélanogénèse.

Le procédé décrit dans la demande de brevet français n° 2.594.331 de la demanderesse met en oeuvre, soit du manganèse sous forme de permanganate, soit un bichromate.

L'utilisation des cations métalliques dans les procédés de teinture pose cependant un certain nombre de problèmes dans la mesure où l'élimination de ces cations métalliques du cheveu est relativement difficile.

Après deux traitements et malgré les rinçages, il reste toujours des traces de métaux sur les cheveux, qui peuvent présenter des inconvénients lorsque les cheveux doivent subir ultérieurement des traitements comme des décolorations ou des permanentes. C'est particulièrement le cas du cuivre et du manganèse.

La demanderesse a décrit, par ailleurs, dans ses demandes de brevets français 2.593.061 et 2.593.062, un procédé de teinture mettant en oeuvre des dérivés d'indole et plus particulièrement le 5,6-dihydroxyindole en association avec un anion minéral et plus particulièrement un iodure.

Dans ce cas, cependant, les procédés nécessitent l'utilisation d'un oxydant minéral constitué notamment par le peroxyde d'hydrogène.

On a également décrit dans la demande EP-A-271186 la teinture des cheveux en utilisant des dérivés d'hydroxyindole et des oxydants, tels que l'acide périodique et les périodates, le permanganate de potassium, l'hypochlorite de sodium, le ferricyanure de potassium, le bichromate de potassium, le persulfate d'ammonium, l'oxyde d'argent, le chlorure ferrique, l'oxyde de plomb, le sulfate de calcium, le réactif de Fenton.

La demanderesse a découvert, ce qui fait l'objet de l'invention, qu'il était possible, de façon surprenante, d'effectuer une teinture à l'aide de dérivés hydroxylés de l'indole, sans utiliser le peroxyde d'hydrogène, l'ammoniaque, un cation métallique, ou un anion minéral, en imprégnant les fibres kératiniques et en particulier les cheveux par le dérivé hydroxylé de l'indole, cette imprégnation étant précédée ou suivie par l'application d'une composition contenant un dérivé quinonique.

La demanderesse a découvert, notamment, qu'il était particulièrement facile de teindre les cheveux naturels dans des tons très divers, des nuances claires aux nuances foncées.

L'invention a donc pour objet un nouveau procédé de teinture des fibres kératiniques et en particulier des fibres kératiniques humaines, mettant en oeuvre un dérivé hydroxylé de l'indole et à titre d'agent oxydant un dérivé quinonique.

D'autres objets de l'invention sont constitués par des dispositifs à plusieurs compartiments utilisés dans le cadre de ce procédé.

D'autres objets de l'invention apparaîtront à la lecture de la description et des exemples qui suivent.

Le procédé de teinture des fibres kératiniques et en particulier des fibres kératiniques humaines, telles que les cheveux, conforme à l'invention, est essentiellement caractérisé par le fait que l'on applique sur ces fibres au moins une composition (A) contenant, dans un milieu approprié pour la teinture, au moins un mono ou dihydroxyindole, l'application de la composition (A) étant précédée ou suivie par l'application d'une composition (B) contenant, dans un milieu approprié pour la teinture, au moins un dérivé quinonique choisi parmi les ortho- ou parabenzoquinones, les ortho- ou parabenzoquinones monoimines ou diimines, les ortho- ou parabenzoquinones sulfonimides, les $\alpha$, $\omega$-alkylène bis-1,4-benzoquinones, les 1,2- ou 1,4-naphtoquinones, les 1,2- ou 1,4-naphtoquinone-monoimines ou diimines; les mono- ou dihydroxyindoles et les dérivés quinoniques étant choisis de façon à ce que la différence de potentiel d'oxydoréduction $\Delta E$ entre le potentiel d'oxydoréduction $E_i$ des mono- ou dihydroxyindoles, déterminé à pH 7 en milieu phosphate sur électrode de carbone vitreux, par voltamétrie, et le potentiel d'oxydoréduction $E_q$ des dérivés quinoniques déterminé à pH 7 en milieu phosphate par polarographie sur électrode de mercure et par rapport à l'électrode au calomel saturé, soit telle que

$$\Delta E = E_i - E_q \leq 320 \text{ mV}.$$

Les mono- ou dihydroxyindoles utilisés conformément à l'invention, répondent plus particulièrement à la formule (I) suivante :

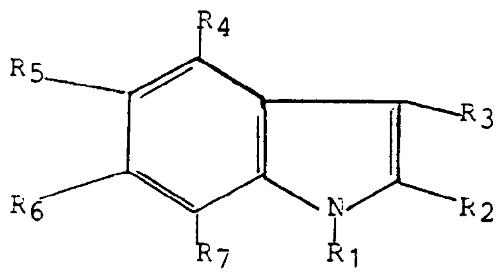

(I)

dans laquelle :

$R_1$ désigne hydrogène ou un groupement alkyle en $C_1$-$C_4$;

$R_2$ et $R_3$, identiques ou différents, représentent un atome d'hydrogène, un groupement alkyle en $C_1$-$C_4$, un groupement carboxyle, un groupement alcoxy ($C_1$-$C_4$) carbonyle, hydroxyalkyle ($C_1$-$C_4$), aminoalkyle ($C_1$-$C_4$);

$R_4$, $R_5$, $R_5$ et $R_7$, indépendamment l'un de l'autre, représentent un atome d'hydrogène, un radical alkyle en $C_1$-$C_4$, amino substitué ou non par un ou deux groupements alkyle ou hydroxyalkyle en $C_1$-$C_4$, acyl ($C_2$-$C_6$) amino, carboxyle, carboxyalkyle en $C_1$-$C_4$, alcoxy($C_1$-$C_4$)carbonyle,

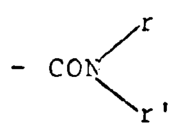

(où r et r' désignent, indépendamment l'un de l'autre, hydrogène ou alkyle en $C_1$-$C_4$), halogène, hydroxyalkyle en $C_1$-$C_4$, amino alkyle en $C_1$-$C_4$, OH ou OZ, Z désignant un radical alkyle, linéaire ou ramifié en $C_1$-$C_{20}$, un groupement aralkyle, un groupement formyle, un groupement acyle en $C_2$-$C_{20}$, linéaire ou ramifié, un groupement alcénoyle en $C_3$-$C_{20}$, linéaire ou ramifié, un groupement $-SiR_{11}R_{12}R_{13}$, un groupement $-P(O)(OR_8)_2$, un groupement $R_8OSO_2$-; les radicaux $R_4$ et $R_5$ ou bien $R_5$ et $R_6$ ou bien $R_5$ et $R_7$ pouvant former, conjointement avec les atomes de carbone auxquels ils sont liés, un cycle contenant éventuellement un groupement carbonyle, un groupement thiocarbonyle, un groupement $>P(O)(OR_8)$ ou un groupement $>CR_9R_{10}$, sous réserve qu'au moins l'un des radicaux $R_4$ à $R_7$ représente un groupement OH,

$R_5$ et $R_9$ représentent un atome d'hydrogène ou un groupement alkyle en $C_1$-$C_4$,

$R_{10}$ représente un groupement alcoxy en $C_1$-$C_4$ ou un groupement mono- ou dialkyl ($C_1$-$C_4$) amino,

$R_{11}$, $R_{12}$ et $R_{13}$, identiques ou différents, représentent des groupements alkyle en $C_1$-$C_4$, linéaires ou ramifiés,

et les sels correspondants de métaux alcalins, alcalino-terreux, d'ammonium et d'amines de ces composés.

Dans la formule précitée, les radicaux alkyle, linéaires ou ramifiés en $C_1$-$C_{20}$, sont particulièrement choisis parmi méthyle, éthyle, propyle, butyle, hexadécyle; le groupement aralkyle est de préférence un groupement benzyle; le groupement acyle en $C_2$-$C_{20}$ est de préférence choisi parmi les groupements acétyle, propionyle, butanoyle, pivaloyle, hexanoyle, myristoyle, hexadecanoyle; le groupement alcénoyle en $C_3$-$C_{20}$, linéaire ou ramifié, est de préférence choisi parmi les radicaux buténoyle, oleyle.

Les composés particulièrement préférés, selon l'invention, sont choisis plus particulièrement parmi :

- le 4-hydroxyindole
- le 4-hydroxy 5-méthoxyindole
- le 4-hydroxy 5-éthoxyindole
- le 5-hydroxyindole
- le 2-carboxy 5-hydroxyindole
- le 5-hydroxy 6-méthoxyindole
- le 6-hydroxyindole
- le 6-hydroxy 7-méthoxyindole
- le 5-méthoxy 6-hydroxyindole
- le 2-carboxy 6-hydroxyindole
- le 2-éthoxycarbonyl 6-hydroxyindole
- le 7-hydroxyindole
- le 2,3-diméthyl 7-hydroxy 4-méthoxyindole

- le 5,6-dihydroxyindole
- le 1-méthyl 5,6-dihydroxyindole
- le 2-méthyl 5,6-dihydroxyindole
- le 3-méthyl 5,6-dihydroxyindole
- le 2,3-diméthyl 5,6-dihydroxyindole
- le (5 ou 6)-acétoxy (6 ou 5)-hydroxyindole
- le 2-éthoxycarbonyl 5,6-dihydroxyindole
- le 2-carboxy 5,6-dihydroxyindole
- le 2,3-diméthyl 5-hydroxy 6-aminoindole
- le 2,3-diméthyl 5-amino 6-hydroxyindole.

Les dérivés quinoniques sont choisis plus particulièrement parmi les composés répondant aux formules (II) et (II') :

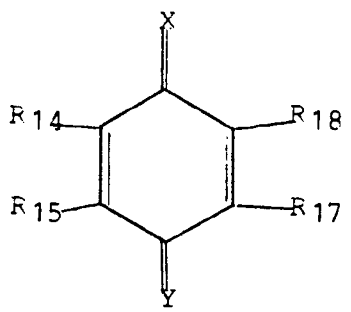

(II)

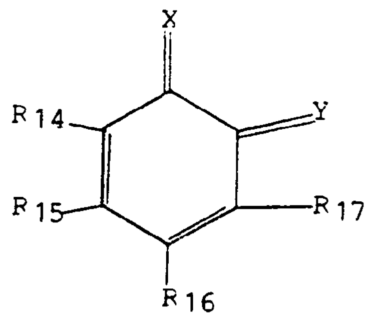

(II')

dans lesquelles :

X désigne oxygène ou un groupement $NR_{19}$;

Y désigne oxygène ou un groupement $NR_{20}$;

$R_{19}$ et $R_{20}$, identiques ou différents, désignant hydrogène, halogène, un radical alkyle en $C_1$-$C_4$, hydroxyalkyle en $C_1$-$C_4$, un radical alkyl ($C_1$-$C_4$)sulfonyle ou un radical phénylsulfonyle;

$R_{14}$, $R_{15}$, $R_{16}$, $R_{17}$ et $R_{18}$ désignent, indépendamment l'un de l'autre, hydrogène, un radical alkyle ($C_1$-$C_4$), un groupement carboxyle, alkyl($C_1$-$C_4$) carbonyle, alcoxy ($C_1$-$C_4$)carbonyle, alcoxy($C_1$-$C_4$) méthyle, alkyl($C_1$-$C_4$)thiométhyle, hydroxyalkyl($C_1$-$C_4$) thiométhyle, hydroxyalkyl($C_1$-$C_4$)sulfinyle,

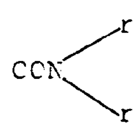

(r et r' désignant, indépendamment l'un de l'autre, hydrogène ou alkyle en $C_1$-$C_4$), carboxyalkyle en $C_1$-$C_4$, halogène, hydroxyalkyle ($C_1$-$C_4$), amino substitué ou non par un ou deux groupes alkyle ou hydroxyalkyle en $C_1$-$C_4$, acyl($C_2$-$C_6$)amino, $SO_3M$, (où M désigne hydrogène, K ou Na), un groupement sulfoxyde, sulfone ou sulfonamide, éventuellement substitué, ou bien un radical $OZ_1$ dans lequel $Z_1$ peut être hydrogène, alkyle ($C_1$-$C_4$), hydroxyalkyle($C_1$-$C_4$), carboxyalkyle($C_1$-$C_4$), phényle éventuellement substitué par alcoxy en $C_1$-$C_4$, ou encore un radical $-SZ_2$ dans lequel $Z_2$ est un groupe alkyle en $C_1$-$C_4$, hydroxyalkyle en $C_1$-$C_4$, dihydroxyalkyle en $C_2$-$C_4$, carboxyalkyle en $C_1$-$C_4$;

$R_{14}$ et $R_{15}$ pouvant former avec les atomes de carbone auxquels ils sont rattachés, le groupement cyclique suivant :

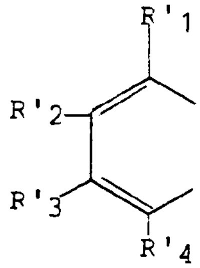

(III)

dans lequel :

$R'_1$, $R'_2$, $R'_3$ et $R'_4$ ont les significations indiquées ci-dessus pour $R_{14}$, $R_{15}$, $R_{16}$, $R_{17}$, $R_{18}$, lorsqu'ils ne forment pas de cycle;

Conformément à l'invention, halogène est choisi de préférence parmi le fluor, le chlore ou le brome.

L'invention a également pour objet de nouvelles 1,4-benzoquinones de formule :

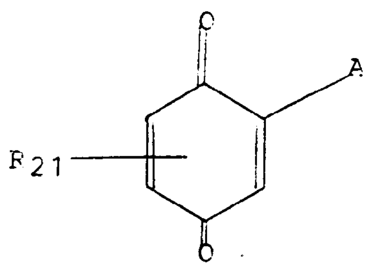

(IV)

dans laquelle A représente les groupements $CH_2SR$ ou SR, dans lesquels R désigne un groupement alkyle en $C_1$-$C_4$ ou hydroxyalkyle en $C_1$-$C_4$; $R_{21}$ représente un atome d'hydrogène, un radical alkyle en $C_1$-$C_4$ ou le groupement SR, R ayant la signification mentionnée ci-dessus, sous réserve que $R_{21}$ représente un atome d'hydrogène lorsque A désigne le groupement $-CH_2SR$,

et leur utilisation dans des compositions tinctoriales pour fibres kératiniques, telles que les cheveux.

Les composés préférés sont choisis, notamment parmi les benzoquinones de formules (II) et (II'), dans lesquelles :

$R_{14}$, $R_{15}$, $R_{16}$, $R_{17}$, $R_{18}$ désignent, indépendamment l'un de l'autre, hydrogène, alkyle inférieur en $C_1$-$C_4$, alcoxy inférieur en $C_1$-$C_4$, halogène, acylamino en $C_2$-$C_6$, $SO_3M$, alcoxy($C_1$-$C_4$)méthyle, carboxy alkyle($C_1$-$C_4$), alcoxy($C_1$-$C_4$)carbonyle, dialkyl($C_1$-$C_4$) amino, $OZ_1$ dans lequel $Z_1$ représente carboxyalkyle ($C_1$-$C_4$), hydroxyalkyle($C_1$-$C_4$), $SZ_2$ dans lequel $Z_2$ représente hydroxyalkyle($C_1$-$C_4$), dihydroxyalkyle($C_1$-$C_4$), carboxyalkyle($C_1$-$C_4$), alkyle($C_1$-$C_4$).

X désigne oxygène ou le groupement $NR_{19}$;

Y désigne oxygène ou le groupement $NR_{20}$,

$R_{19}$ et $R_{20}$, indépendamment l'un de l'autre, désignent hydrogène, halogène, alkyle inférieur en $C_1$-$C_4$, méthylsulfonyle ou phénylsulfonyle;

d'autres composés préférés utilisés conformément à l'invention, répondent aux formules (V) et (VI) ci-après :

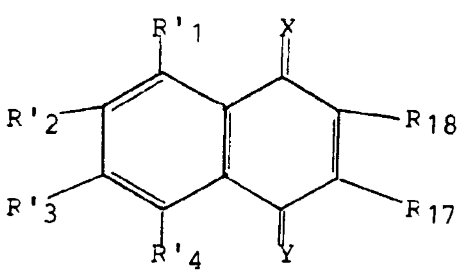

(V)

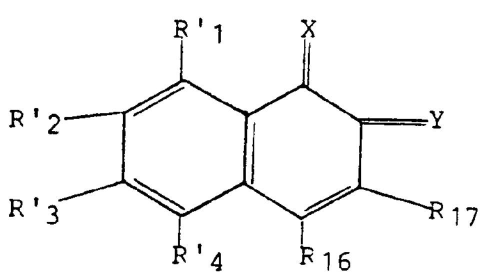

(VI)

dans lesquelles :

$R'_1$, $R'_2$, $R'_3$, $R'_4$, $R_{16}$, $R_{17}$ et $R_{18}$ ont les significations indiquées ci-dessus pour les composés de formules (II), (II') et (III);

X et Y ayant les mêmes significations que celles indiquées ci-dessus.

Les composés particulièrement préférés sont choisis parmi ceux dans lesquels $R'_1$,$R'_2$,$R'_3$,$R'_4$,$R_{16}$, $R_{17}$,$R_{18}$ désignent hydrogène, alkyle en $C_1$-$C_4$, alcoxy en $C_1$-$C_4$, halogène, acyl($C_2$-$C_6$)amino ou $SO_3H$.

Les composés particulièrement préférés utilisables conformément à l'invention, sont en particulier ;

- la 1,4-benzoquinone
- la 2-méthoxy 1,4-benzoquinone
- la 2-méthyl 1,4-benzoquinone
- la 2,6-diméthyl 1,4-benzoquinone
- la 2,3,5-trichloro 6-méthyl 1,4-benzoquinone
- la 2-acétylamino 1,4-benzoquinone
- la 2-acétylamino 3,5-diméthyl 1,4-benzoquinone
- la 2,6-diméthyl 5-acétylamino 1,4-benzoquinone
- la 2-chloro 1,4-benzoquinone
- la tétrachloro 1,2-benzoquinone
- la 2,3-diméthoxy 1,4-benzoquinone
- la 2-β-carboxyéthoxy 1,4-benzoquinone
- la 2-méthoxyméthyl 1,4-benzoquinone
- la 2-β-hydroxyéthyl 1,4-benzoquinone
- la 2-β-hydroxyéthylthio 1,4-benzoquinone
- la 2,5-bis-ß-hydroxyéthylthio 1,4-benzoquinone
- la 2-β, γ-dihydroxypropylthio 1,4-benzoquinone
- la 2-β-carboxyéthylthio 1,4-benzoquinone
- la 2-carboxyméthyl 1,4-benzoquinone
- la 2-β-hydroxyéthylthio 6-méthyl 1,4-benzoquinone
- la 2-méthoxycarbonyl 3-méthoxy 1,4-benzoquinone
- la 2-méthoxycarbonyl 1,4-benzoquinone
- la 2-méthylthio 1,4-benzoquinone
- la 2-diméthylamino 1,4-benzoquinone
- la 2-acétylamino 5-méthoxy 1,4-benzoquinone
- la 2-(β-hydroxyéthylthio)méthyl 1,4-benzoquinone
- la 2-(méthylthio)méthyl 1,4-benzoquinone
- la 4,5-diméthoxy 1,2-benzoquinone
- la 4-méthyl 5-chloro 1,2-benzoquinone
- la 4,5-diméthyl 1,2-benzoquinone
- la 2,3-diméthyl 1,4-benzoquinone
- la 2-β-hydroxyéthoxy 1,4-benzoquinone
- la N-méthyl sulfonyl 1,4-benzoquinone monoimine
- la N-phényl sulfonyl 1,4-benzoquinone monoimine
- la 1,4-naphtoquinone
- la 1,2-naphtoquinone
- l'acide 1,2-naphtoquinone 4-sulfonique
- la 2,3-dichloro 1,4-naphtoquinone.
- la N-2,6-trichloro 1,4-benzoquinoneimine.

Les associations particulièrement préférées, conformément à l'invention, sont choisies parmi l'association du 5,6-dihydroxyindole avec la 1,4-benzoquinone, la 2-méthyl 1,4-benzoquinone, la 2,6-diméthyl 1,4-benzoquinone, la 2-méthoxy 1,4-benzoquinone, la 2-chloro 1,4-benzoquinone, la 2,3,5-trichloro 6-méthyl 1,4-benzoquinone, la 2-acétylamino 1,4-benzoquinone, la 2-acétylamino 3-méthoxy 1,4-benzoquinone, la 2,6-diméthyl 5-acétylamino 1,4-benzoquinone, la 2,3-diméthoxy 1,4-benzoquinone, la 2-méthoxyméthyl 1,4-benzoquinone, la 2-β-hydroxyéthyl 1,4-benzoquinone, la 2-β, γ-dihydroxypropylthio 1,4-benzoquinone, la 2-β-carboxyéthylthio 1,4-benzoquinone, la 2-carboxyméthyl 1,4-benzoquinone, la 1,4-naphtoquinone, la N-2,6-trichloro 1,4-benzoquinonimine, la 1,2-naphtoquinone, l'acide 1,2-naphtoquinone 4-sulfonique.

Dans les conditions habituelles d'une teinture, c'est-à-dire pour des temps de pose de 2 à 30 minutes et une température habituellement supportée par les modèles, par exemple entre 25° et 40°C, la concentration du mono- ou du dihydroxyindole utilisé dans la composition (A) est de préférence comprise entre 0,01 et 0,3 mole/litre.

La concentration en dérivé quinonique est telle qu'elle permet l'oxydation du mono- ou dihydroxy indole dans les conditions habituelles utilisées par le coiffeur lors d'une teinture et elle est de préférence comprise entre 0,005 et 1 mole/litre dans la composition (B).

Le pH de la composition (A) est de préférence compris entre 2 et 10.

Le pH de la composition (B) est de préférence compris entre 2 et 10.

La composition (B) est cependant utilisée de préférence à un pH acide.

Les compositions (A) et (B) peuvent être conditionnées sous des formes habituellement utilisées, notamment dans la teinture des cheveux, en particulier sous forme de lotion plus ou moins épaissie, de gel, d'émulsion éventuellement conditionnée(s) en aérosol.

Le milieu approprié pour la teinture est généralement un milieu aqueux qui peut être constitué par l'eau ou un mélange d'eau et d'un solvant qui, lorsque la composition est appliquée sur les cheveux, doit être cosmétiquement acceptable.

De tels solvants sont choisis plus particulièrement parmi les solvants organiques, tels que les alcools inférieurs en $C_1$-$C_6$ comme l'alcool éthylique, l'alcool propylique ou isopropylique, l'alcool tertiobutylique, l'éthylèneglycol, le propylèneglycol, les éthers nonométhylique, monoéthylique ou monobutylique de l'éthylèneglycol, l'acétate du monoéthyléther de l'éthylèneglycol, les monométhyléthers du propylèneglycol et du dipropylène glycol, le lactate de méthyle.

Les solvants particulièrement préférés sont l'alcool éthylique et le propylèneglycol.

Les solvants, lorsqu'ils sont utilisés, le sont plus particulièrement dans des concentrations comprises entre 10 et 50% pour les alcools inférieurs et pour les concentrations élevées en mono- ou dihydroxyindole.

Les compositions (A) et (B) conformes à l'invention peuvent également être stockées en milieu solvant anhydre. Les solvants sont choisis parmi les solvants définis ci-dessus. On appelle milieu anhydre un milieu contenant moins de 1% d'eau.

Les compositions conformes à l'invention, lorsqu'elles sont utilisées pour la teinture des cheveux, peuvent également contenir tous autres adjuvants habituellement utilisés en cosmétique et plus particulièrement des agents tensio-actifs anioniques, cationiques, non-ioniques, amphotères ou leurs mélanges, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

Les compositions (A) et/ou (B) utilisables dans le procédé conforme à l'invention, peuvent contenir d'autres colorants habituellement utilisés pour la teinture des fibres kératiniques et en particulier des colorants directs, tels que les dérivés nitrés benzéniques, les colorants d'oxydation de type para ou ortho et des coupleurs ou des colorants d'oxydation dits "rapides", c'est-à-dire des molécules à structure benzénique, précurseurs de colorants susceptibles de générer les composés colorés par simple oxydation à l'air pendant le temps de pose sur la chevelure qui est généralement inférieur à 1 heure et en l'absence de tout autre agent oxydant.

Ces compositions peuvent également contenir des colorants quinoniques de la famille des benzoquinones, des benzoquinones imines ou diimines, des naphtoquinones, des naphtoquinone-imines ou naphtoquinonediimines, qui ne répondent pas aux conditions de potentiel définies ci-dessus. Ces colorants sont, dans ce cas, utilisés pour apporter leur propre nuance à la teinture.

En vue de la mise en oeuvre du procédé conforme à l'invention, on peut conditionner les différentes compositions dans un dispositif à plusieurs compartiments encore appelé "kit" ou nécessaire de teinture comportant tous les composants destinés à être appliqués pour une même teinture sur les fibres kératiniques et en particulier les cheveux, en applications successives avec ou sans prémélange.

De tels dispositifs peuvent comporter plus particulièrement un premier compartiment contenant la composition (A) et un second compartiment comportant la composition (B) . Une autre variante peut également consister à stocker la composition (A) ou la composition (B) dans un milieu solvant anhydre et à prévoir un troisième compartiment contenant un milieu aqueux approprié pour la teinture et cosmétiquement acceptable si les compositions sont destinées à être appliquées sur les cheveux. Dans ce cas, on mélange tout juste avant l'emploi le contenu du troisième compartiment dans l'un ou l'autre ou les deux compartiments contenant les compositions anhydres (A) et (B).

Le procédé conforme à l'invention est mis en oeuvre de préférence en appliquant, dans un premier temps, la composition (A) et dans un second temps, la composition (B). Il peut être utilisé, notamment, pour teindre les cheveux humains, naturels ou déjà teints, permanentés ou non, ou défrisés, les cheveux fortement ou légèrement décolorés et éventuellement permanentés.

Dans ce cas, on applique la composition (A) à une température qui peut être supportée par la tête, c'est-à-dire comprise entre 25 et 40°C, pendant 2 à 30 minutes et on fait suivre avec ou sans rinçage intermédiaire cette application par l'application de la composition (B) contenant le dérivé quinonique qui est maintenu au contact des cheveux pendant 5 à 30 minutes, la température de teinture est également comprise entre 25 et

40°C.

Le procédé conforme à l'invention peut également être mis en oeuvre, en vue de nuancer ou rafraîchir une teinture qui aurait été effectuée à l'aide de mono- ou dihydroxyindole, en appliquant la composition (B) plusieurs heures ou jours après la teinture à l'aide des mono- ou dihydroxyindoles.

Il est également possible d'utiliser ce procédé pour la teinture des fourrures ou de la laine dans les conditions habituelles industrielles de température, de temps de contact et de concentration.

Les composés nouveaux de formule (IV) utilisables conformément à l'invention, peuvent être préparés suivant les procédés suivants :

A/ Lorsque (A) désigne le groupement $CH_2SR$ et $R_{21}$ hydrogène (formule IV(A)). Ces composés IV(A) sont préparés à partir des para aminophénols correspondants par oxydation avec le chlorure ferrique ou le sulfate ferrique, suivant le schéma réactionnel I suivant.

SCHEMA REACTIONNEL I

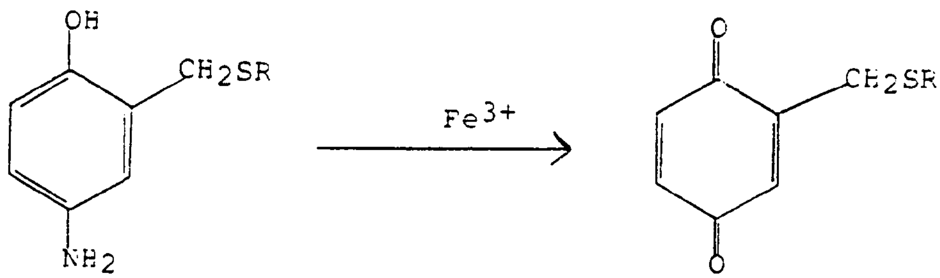


(VII) (IV(A))

Les composés de formule (VII) sont préparés en deux étapes à partir du nitrophénol substitué de formule (IX), dans laquelle Y désigne un atome d'halogène, selon le schéma réactionnel ci-dessous :

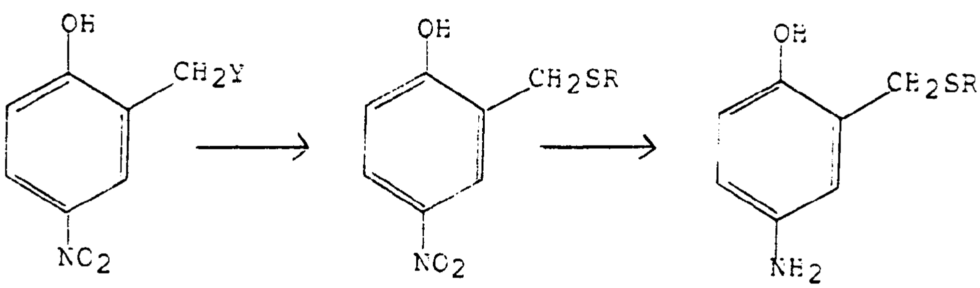

(IX) (VIII) (VII)

Le composé de formule (VII) est obtenu par réduction du composé de formule (VIII) où R a les significations ci-dessus définies. Parmi les méthodes de réduction classiques, on peut citer la réduction par l'hydrosulfite de sodium en milieu alcalin à une température inférieure à 80°C, ou bien une réduction catalytique en milieu hydroalcoolique, sous pression d'hydrogène, en présence d'un catalyseur tel que le Palladium sur charbon ou le Nickel.

Le composé de formule (VIII) peut être préparé par action d'un thiolate de formule :

$$M - S - R$$

dans laquelle M représente un métal alcalin ou alcalino-terreux et R a les significations ci-dessus définies sur le composé de formule (IX).

Le thiolate peut être avantageusement préparé in situ selon le schéma réactionnel :

$$MOH + HSR \rightarrow MSR + H_2O$$

le composé HSR pouvant également servir de solvant.

Parmi les solvants que l'on peut utiliser pour la préparation des composés de formule (VIII), on peut citer, outre le composé HSR, le dioxane, le N,N-diméthyl formamide, la N-méthylpyrrolidone, utilisés seuls ou en mélange. Généralement, la température de la réaction est inférieure à 100°C.

B/ Lorsque (A) désigne le groupement SR, R ayant les significations indiquées ci-dessus et $R_{21}$ étant hy-

drogène ou alkyle (pour les composés de formule (IV) (composé IV(B)).

Ces composés IV(B) sont préparés à partir des composés benzoquinoniques de formule (X) en faisant réagir dans un solvant alcool/eau ou dans l'éthanol, deux moles du dérivé quinonique avec une mole de thiol (RSH) selon le schéma réactionnel II.

SCHEMA REACTIONNEL II

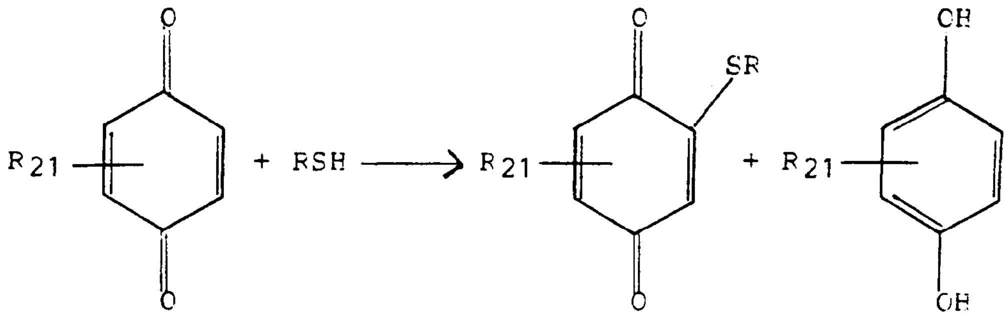

(X) VI(B)

C/ Lorsque (A) et $R_{21}$ désignent simultanément SR, R ayant la signification indiquée pour les composés (IV) (composé IV(C)).

Ces composés sont préparés en faisant réagir 4 moles de p-benzoquinone avec 3 moles de dérivé de formule RSH dans l'éthanol/eau ou l'éthanol absolu, selon le schéma réactionnel III.

SCHEMA REACTIONNEL III

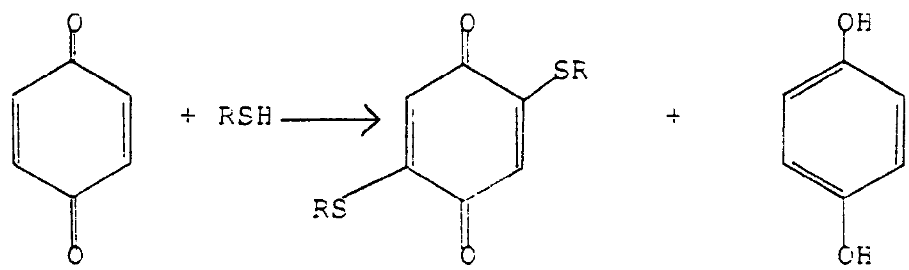

IV(C)

Les exemples suivants sont destinés à illustrer la préparation des composés de formule (IV).

EXEMPLE DE PREPARATION 1

Préparation de la 2-(méthylthio)méthyl 1,4-benzoquinone

(Formule IV(A),R=$CH_3$):

1ère étape :

Préparation du 4-amino 2-[(méthylthio)méthyl]phénol.

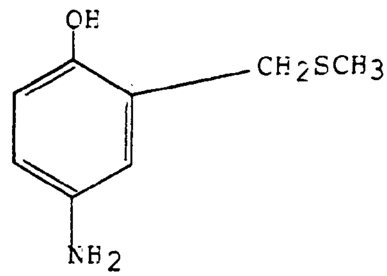

A une solution de 16 g de soude (NaOH) en pastilles dans 135 ml d'eau, on ajoute 0,085 mole (17 g) de 4-nitro 2-[(méthylthio)méthyl]phénol, puis par portions de façon à maintenir la température entre 70°C et 75°C, 55 g d'hydrosulfite de sodium. L'agitation est maintenue 30 minutes à 75°C après la fin de l'addition. Le produit attendu est obtenu, par neutralisation du milieu réactionnel par l'acide acétique, après refroidissement. Après essorage, puis lavage à l'eau et séchage, il est recristallisé de l'éthanol à 96°. Il fond à 166°C.

L'analyse du produit obtenu donne les résultats suivants :

Analyse pour $C_8H_{11}NOS$

| | C | H | N | O | S |
|---|---|---|---|---|---|
| Calculé | 56,79 | 6,55 | 8,28 | 9,45 | 18,91 |
| Trouvé | 56,69 | 6,49 | 8,25 | 9,56 | 18,76 |

2ème étape :

On dissout le 4-amino 2-[(méthylthio)méthyl]phénol (8,45 g, 0,05 mole) dans un mélange acétate d'éthyle (200 ml), eau (200 ml) et acide chlorhydrique concentré (6 ml). On y ajoute une solution de chlorure ferrique (16,25 g dans 125 ml d'eau, 0,1 mole). Au bout d'un quart d'heure, la solution violet noir est extraite 4 fois avec de l'acétate d'éthyle. Les phases organiques sont séchées sur sulfate de sodium et concentrées au quart. La phase organique est traitée au noir végétal, filtrée et le solvant évaporé. On obtient une huile orangée brun qui cristallise dans l'éther diisopropylique. On obtient une poudre orange :

Poids : 3 g
Rendement : 36%
Pf : 43°C

Analyse pour $C_8H_8O_2S$

| | C | H | O | S |
|---|---|---|---|---|
| Calculé | 57,12 | 4,79 | 19,02 | 19,06 |
| Trouvé | 57,23 | 4,78 | 19,20 | 19,16 |

EXEMPLE DE PREPARATION 2

Préparation de la 2-(β-hydroxyéthylthio)méthyl 1,4-benzoquinone

(Formule IV(A): $R=CH_2CH_2OH$):

1ère étape :

Préparation du 4-amino 2-[(β-hydroxyéthylthio)méthyl]phénol.

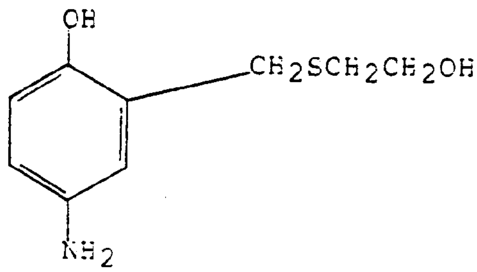

A une solution de 16 g de soude (NaCH) en pastilles dans 135 ml d'eau, on ajoute 0,085 mole (19,5 g) de 4-nitro 2-[(β-hydroxyéthylthio)méthyl]phénol, puis par portions, de façon à maintenir la température entre 70°C et 75°C, 55 g d'hydrosulfite de sodium. L'agitation est maintenue 20 minutes à 75°C après la fin de l'addition. Le produit attendu est précipité, par neutralisation du milieu réactionnel par l'acide acétique, après refroidissement. Après essorage, puis lavage à l'eau et séchage, le produit obtenu est recristallisé de l'acétonitrile. Il fond à 121°C.

L'analyse du produit obtenu donne les résultats suivants :

Analyse pour $C_9H_{13}NO_2S$

| | C | H | N | O | S |
|---|---|---|---|---|---|
| Calculé | 54,24 | 6,58 | 7,03 | 16,06 | 16,09 |
| Trouvé | 54,02 | 6,62 | 7,00 | 16,17 | 15,92 |

2ème étape :

On ajoute une solution de chlorure ferrique (0,65 g dans 50 ml d'eau) à une solution de 4-amino 2-[(β-hydroxyéthylthio)méthyl]phénol (0,4 g, 2 mmoles). On extrait aussitôt au dichlorométhane, sèche la phase organique et concentre. L'huile brune obtenue cristallise. Après recristallisation dans un mélange éther diisopropylique/hexane, on obtient des cristaux oranges :

Poids : 0,2 g
Rendement : 50%
Pf : 57°C

Analyse pour $C_9H_{10}O_3S$

| | C | H | O | S |
|---|---|---|---|---|
| Calculé | 54,53 | 5,08 | 24,21 | 16,17 |
| Trouvé | 54,31 | 5,07 | 23,94 | 16,56 |

EXEMPLE DE PREPARATION 3

Préparation de la 2-β-hydroxyéthylthio 1,4-benzoquinone.

(Formule IV(B):R=$CH_2CH_2OH$,$R_{21}$=H):

A une suspension de p-benzoquinone (80 g, 0,736 moles) dans l'éthanol absolu (500 ml), sous agitation et sous barbottage d'azote, on ajoute en une seule fois le 2-mercapto éthanol (25,9 ml, 0,368 mole). La température augmente de 22°C à 45°C en 30 secondes et le milieu devient homogène rouge brun. Au bout de quinze minutes, un précipité apparaît. On laisse sous agitation pendant 2 heures et filtre sur fritté n°4. Le précipité orangé rouge est lavé par 3x50 ml d'éthanol froid et séché. On obtient des cristaux orangés :

Poids : 53 g
Rendement : 78%
Pf : 108-109°C

Analyse pour $C_8H_8O_3S$

| | C | H | O | S |
|---|---|---|---|---|
| Calculé | 52,16 | 4,38 | 26,06 | 17,41 |
| Trouvé | 52,03 | 4,34 | 26,28 | 17,25 |

EXEMPLE DE PREPARATION 4

Préparation de la 2-hydroxyéthylthio 6-méthyl 1,4 benzoquinone.

(Formule IV(B):R=$CH_2CH_2OH$,$R_{21}$=6-$CH_3$):

Sous azote et sous agitation, on mélange dans l'éthanol absolu (70 ml) de la méthyl-p-benzoquinone (12,2 g, 0,1 mole) et du 2-mercapto éthanol (3,9 g, 0,05 mole). La température s'élève jusqu'à 50°C. On refroidit par un bain d'eau glacée et laisse sous agitation pendant 2 heures à température ambiante. La solution est concentrée sous vide et l'huile obtenue est chromatographiée sur Silice 60 (éluant : dichlorométhane). Une première fraction (3 g) est un mélange 50/50 par RMN du $^1H$ et $^{13}C$ de deux produits : la 2-hydroxyéthylthio 5-méthyl 1,4-benzoquinone et la 2-hydroxyéthylthio 6-méthyl 1,4-benzoquinone. Une deuxième fraction donne le produit attendu :

Poudre orangée
Poids : 0,5 g
Pf : 82°C

Analyse pour $C_9H_{10}O_3S$

| | C | H | O | S |
|---|---|---|---|---|
| Calculé | 54,53 | 5,06 | 24,21 | 16,17 |
| Trouvé | 54,33 | 4,94 | 24,38 | 16,05 |

EXEMPLE DE PREPARATION 5

Préparation de la 2,5-bis-β-hydroxyéthylthio 1,4-benzoquinone.

(Formule IV(C):R=$CH_2CH_2OH$):

On ajoute en une fois du mercapto-2 éthanol (3,5 ml, 0,05 mole) à une solution éthanolique de p-benzoquinone (10,81 g, 0,1 mole) et laisse sous agitation pendant une 1/2 heure. On ajoute à nouveau du 2-mercapto

éthanol (1,75 ml, 0,025 mole) et laisse sous agitation pendant 2 heures. On filtre et lave le précipité à l'éthanol absolu. On obtient des cristaux rouges foncés du dérivé attendu :

Poids : 6,7 g
Rendement : 34%
Pf : 120-122°C

Analyse pour $C_{10}H_{12}O_4S_2$

| | C | H | O | S |
|---|---|---|---|---|
| Calculé | 46,14 | 4,65 | 24,58 | 24,63 |
| Trouvé | 46,11 | 4,69 | 24,66 | 24,29 |

Les exemples suivants sont destinés à illustrer le procédé de teinture conforme à l'invention.

EXEMPLE 1

On imprègne une mèche de 1 g de cheveux naturels à 90% de blancs, par 5 ml d'une solution de 5,6-dihydroxyindole à 2,5% en milieu hydroéthanolique 90/10 pendant 15 minutes. On rince la mèche à l'eau courante et on l'essore. On imprègne à nouveau cette mèche par 5 ml d'une solution à 2% de 1,4-benzoquinone en milieu hydroéthanolique 50/50 pendant 8 minutes. On rince la mèche à l'eau courante et on fait un shampooing avec une solution aqueuse de laurylsulfate de sodium à 5%. On obtient une mèche dont la couleur est noire intense avec de légers reflets bleutés.

Le potentiel d'oxydoréduction du 5,6-dihydroxyindole, déterminé en milieu phosphate à ph 7, sur électrode de carbone vitreux par voltamètrie est $E_i$ = 90 mV.

Le potentiel d'oxydoréduction de la 1,4-benzoquinone, déterminé en milieu phosphate à pH 7 par polarographie, sur électrode de mercure par rapport à l'électrode au Calomel est $E_q$ = 10 mV. $\Delta E$ = 80 mV.

EXEMPLE 2

On imprègne, dans un premier temps, une mèche de 1 g de cheveux naturels gris (90% de blancs), par 5 ml d'une solution de 4-hydroxy 5-méthoxy indole à 2,5% en solution hydroéthanolique 50/50 pendant 15 minutes, à température ambiante. On rince la mèche à l'eau courante et on l'essore. On imprègne, dans un deuxième temps, cette même mèche, par 5 ml d'une solution de 1,4-benzoquinone à 2% en milieu hydroéthanolique 50/50, pendant 8 minutes. On lave à l'eau courante et on termine par un shampooing au laurylsulfate de sodium à 5%. On obtient une mèche intensément colorée dont la nuance est violette.

Les valeurs des potentiels d'oxydoréduction déterminées comme dans l'exemple 1 sont :

$$E_i = 110 \text{ mV} \; E_q = 10 \text{ mV} \; \Delta E = 100 \text{ mV}$$

EXEMPLE 3

On prend une mèche de 1 g de cheveux naturels gris (à 90% de blancs) et on l'imprègne pendant 15 minutes par une solution hydroéthanolique 60/40 de 2-méthyl 5,6-dihydroxyindole à 2,5%. On lave la mèche à l'eau courante puis on l'essore. On applique à nouveau 5 ml d'une solution de 1,4-benzoquinone à 2% en milieu hydroalcoolique 50/50, pendant 9 minutes. On lave la mèche à l'eau courante puis on effectue un shampooing avec du laurylsulfate de sodium à 5%. On obtient une mèche dont la couleur est noir-corbeau.

Les valeurs des potentiels d'oxydoréduction, déterminées comme dans l'exemple 1 sont les suivantes :

$$E_i = 45 \text{ mV} \; E_q = 10 \text{ mV} \; \Delta E = 35 \text{ mV}$$

EXEMPLE 4

On prépare une solution de 6-hydroxy 7-méthoxy indole à 2,5% en milieu hydroalcoolique 80/20. On applique 5 ml de cette solution sur une mèche de 1 g de cheveux naturels (gris à 90% de blancs), pendant 15

minutes. On rince la mèche à l'eau courante, puis on l'essore. On imprègne à nouveau cette mèche pendant 10 minutes, par 5 ml d'une solution hydroalcoolique 50/50 de 1,4-benzoquinone à 2%. On rince la mèche à l'eau et on termine par un shampooing au laurylsulfate de sodium à 5%. On obtient une mèche de couleur châtain.

Les valeurs des potentiels d'oxydoréduction déterminées comme dans l'exemple 1 sont les suivantes :

$$E_i = 160\ mV\ E_q = 10\ mV\ \Delta E = 150\ mV$$

EXEMPLE 5

On prépare une solution à 2,5% de 5-méthoxy 6-hydroxyindole dans une solution hydroalcoolique 70/30. On laisse poser cette solution sur une mèche de cheveux gris à 90% de cheveux blancs pendant 15 minutes, à température ambiante. On rince, essore, puis révèle la coloration par une solution hydroalcoolique 50/50 à 2% de 1,4-benzoquinone. Le temps de pose est de 8 minutes et après rinçage, shampooing, rinçage et séchage, on obtient une couleur noire.

Les valeurs des potentiels d'oxydoréduction déterminées comme dans l'exemple 1 sont :

$$E_i = 180\ mV\ E_q = 10\ mV\ \Delta E = 170\ mV$$

EXEMPLE 6

On prétraité une mèche de cheveux gris naturels, à 90% de cheveux blancs, pendant 15 minutes, par une solution hydroalcoolique 60/40 contenant 2,5% de 5-hydroxy 6-méthoxyindole. Le temps de pose est de 15 minutes et après rinçage et essorage, on révèle par une solution hydroalcoolique 50/50, à 2% de 1,4-benzoquinone. Le temps de pose est de 8 minutes et après rinçage, shampooing, rinçage à nouveau et séchage, on obtient une couleur châtain clair.

Les valeurs des potentiels d'oxydoréduction déterminées comme dans l'exemple 1 sont :

$$E_i = 230\ mV\ E_q = 10\ mV\ \Delta E = 220\ mV$$

EXEMPLE 7

On applique sur une mèche de cheveux gris naturels, à 90% de cheveux blancs, une solution hydroalcoolique 80/20, de 2,5% de 7-hydroxyindole. On laisse poser 15 minutes, rince, essore et applique une solution hydroalcoolique 50/50 contenant 2% de 1,4-benzoquinone. On laisse poser 15 minutes, lave, essore, sèche et on obtient une couleur violet foncé très intense.

Les valeurs des potentiels d'oxydoréduction déterminées comme dans l'exemple 1 sont :

$$E_i = 285\ mV\ E_q = 10\ mV\ \Delta E = 275\ mV$$

EXEMPLE 8

On traite une mèche de cheveux gris par une solution hydroalcoolique 80/20 contenant 2,5% de 6-hydroxyindole. Après un temps de pose de 15 minutes, on rince la mèche, l'essore et applique une composition à base d'eau/alcool 50/50 et 2% de 1,4-benzoquinone. On laisse poser 9 minutes, après quoi, on lave au shampooing, rince, sèche et obtient une couleur châtain doré.

Les valeurs des potentiels d'oxydoréduction déterminées comme dans l'exemple 1 sont :

$$E_i = 320\ mV\ E_q = 10\ mV\ \Delta E = 310\ mV$$

EXEMPLE 9

On traite une mèche de cheveux naturellement gris à 90% de cheveux blancs par une solution hydroalcoolique 60/40 de 1% de (5 ou 6)-acétoxy (6 ou 5)-hydroxyindole. On laisse poser pendant 15 minutes, après quoi, on rince, essore et applique une solution hydroalcoolique 50/50 de 2% de 1,4-benzoquinone. On laisse poser 8 minutes, puis lave, rince, essore et on obtient une couleur châtain doré.

Les valeurs des potentiels d'oxydoréduction déterminées comme dans l'exemple 1 sont :

$$E_i = 110\ mV\ OU\ 270\ mV\ E_q = 10\ mV$$
$$\Delta E = 100\ mV\ ou\ 260\ mV$$

EXEMPLE 10

Après avoir prétraité, pendant 15 minutes, une mèche de cheveux gris à 90% de cheveux blancs par une

solution hydroalcoolique 60/40 de 2,5% de 2,3-diméthyl 5,6-dihydroxyindole, on rince, essore et applique, pendant 8 minutes, une solution hydroalcoolique 50/50 de 2% de 1,4-benzoquinone. On opère comme précédemment et obtient une couleur acajou.

Les valeurs des potentiels d'oxydoréduction déterminées comme dans l'exemple 1 sont :

$E_i$ = 30 mV $E_q$ = 10 mV $\Delta E$ = 20 mV

EXEMPLE 11

Le procédé d'application est identique à celui de l'exemple 10, mais le colorant indolique est cette fois le 4-hydroxy 5-éthoxyindole. On obtient une couleur violet intense.

Les valeurs des potentiels d'oxydoréduction déterminées comme dans l'exemple 1 sont :

$E_i$ = 110 mV $E_q$ = 10 mV $\Delta E$ = 100 mV

EXEMPLE 12

En opérant comme dans l'exemple 10, avec le 1-méthyl 5,6-dihydroxyindole, on obtient une coloration noir corbeau intense.

Les valeurs des potentiels d'oxydoréduction déterminées comme dans l'exemple 1 sont :

$E_i$ = 105 mV $E_q$ = 10 mV $\Delta E$ = 95 mV

EXEMPLE 13

En partant d'une solution hydroalcoolique 50/50 à 2% de 2,3-diméthyl 4-hydroxy 7-méthoxy indole et en opérant avec la même composition révélatrice qu'à l'exemple 10 et suivant le même mode opératoire, on obtient une couleur acajou.

Les valeurs des potentiels d'oxydoréduction déterminées comme dans l'exemple 1 sont :

$E_i$ = 20 mV $E_q$ = 10 mV $\Delta E$ = 10 mV

EXEMPLE 14

Le procédé est le même que celui décrit à l'exemple 10 et les compositions identiques, hormis le fait que le colorant utilisé est le 2-méthyl 5,6-dihydroxyindole. On obtient ainsi, après révélation, une couleur châtain très foncé.

Les valeurs des potentiels d'oxydoréduction déterminées comme dans l'exemple 1 sont :

$E_i$ = 45 mV $E_q$ = 10 mV $\Delta E$ = 35 mV

EXEMPLE 15

On traite une mèche de cheveux naturellement gris par une solution hydroalcoolique 80/20 contenant 2,5% de 5,6-dihydroxyindole. Après un temps de pose de 15 minutes, rinçage et essorage, on révèle la couleur par la 2-méthyl 1,4-benzoquinone (toluquinone) dissoute à raison de 2% dans une solution hydroalcoolique 50/50. Après avoir rincé, lavé, rincé à nouveau et séché, on obtient une couleur noir intense avec des reflets bleutés.

Les valeurs des potentiels d'oxydoréduction déterminées comme dans l'exemple 1 sont :

$E_i$ = 90 mV $E_q$ = -45 mV $\Delta E$ = 135 mV

EXEMPLE 16

Suivant le même procédé qu'à l'exemple 15 et remplaçant la toluquinone par la 2,6-diméthyl 1,4-benzoquinone, on obtient une couleur châtain moyen avec des reflets violacés.

Les valeurs des potentiels d'oxydoréduction déterminées comme dans l'exemple 1 sont :

$E_i$ = 90 mV $E_q$ = -70 mV $\Delta E$ = 160 mV

EXEMPLE 17

Si dans l'exemple 16, on remplace la toluquinone par la même quantité de 2-méthoxy 1,4-benzoquinone, on obtient une couleur noir intense.

Les valeurs des potentiels d'oxydoréduction déterminées comme dans l'exemple 1 sont :

$E_i$ = 90 mV $E_q$ = -40 mV $\Delta E$ = 130 mV

EXEMPLE 18

On traite une mèche de cheveux gris naturels à 90% de blancs par une solution hydroéthanolique 50/50 contenant 2,5% de 5,6-dihydroxyindole. Après 15 minutes de temps de pose, on rince la mèche, l'essore et applique une composition hydroéthanolique 50/50 contenant 2% de tétrachloro-1,2 benzoquinone. On laisse poser 10 minutes, on rince, on lave au shampooing, on rince à nouveau et on sèche. On obtient une couleur gris foncé.

$E_i$ = 90 mV $E_q$ = -80 mV $\Delta E$ = 170 mV

EXEMPLE 19

La composition de prétraitement est la même que dans l'exemple 16 et celle de révélation est à base de 2,6-diméthyl 5-acétylamino 1,4-benzoquinone. On obtient une couleur châtain gris clair.

Les valeurs des potentiels d'oxydoréduction déterminées comme dans l'exemple 1 sont :

$E_i$ = 90 mV $E_q$ = -65 mV $\Delta E$ = 155 mV

EXEMPLE 20

Le prétraitement est le même que dans l'exemple 16. On le fait suivre d'une révélation par une solution hydroalcoolique 50/50 à 2% de 1,4-naphtoquinone. On laisse poser 8 minutes, rince, lave, rince à nouveau et sèche. On observe une couleur châtain clair.

Les valeurs des potentiels d'oxydoréduction déterminées comme dans l'exemple 1 sont :

$E_i$ = 90 mV $E_q$ = -185 mV $\Delta E$ = 275 mV

EXEMPLE 21

On opère comme dans l'exemple 16, la révélation étant effectuée à l'aide de la N-2,6-trichloro 1,4-benzoquinone-imine. La couleur obtenue est châtain foncé.

Les valeurs des potentiels d'oxydoréduction déterminées comme dans l'exemple 1 sont :

$E_i$ = 90 mV $\Delta E$ - 90 mV $E_q \geqq$ 180 mV

EXEMPLE 22

On prétraité tout d'abord à l'aide d'une solution hydroalcoolique 50/50 contenant 2% de 1,4-benzoquinone. Après un temps de pose de 15 minutes, on rince, essore et révèle par une solution hydroalcoolique 80/20 contenant 2,5% de 5,6-dihydroxyindole à pH 3. On laisse poser pendant 15 minutes, rince, lave au shampooing, rince à nouveau et sèche. On obtient une couleur châtain doré sur cheveux initialement gris à 90% de cheveux blancs.

Les valeurs des potentiels d'oxydoréduction déterminées comme dans l'exemple 1 sont :

$E_i$ = 90 mV $E_q$ = 10 mV $\Delta E$ = 80 mV

EXEMPLE 23

Composition (A)

| | | | |
|---|---|---|---|
| - 5,6-dihydroxyindole | | 2,5 | g |
| - Alcool éthylique | | 10,0 | g |
| - Hydroxypropylguar vendu sous la dénomination de JAGUAR HP 60 par la Société MEYHALL | | 1,0 | g |
| - Alkyléther de glycoside vendu à la concentration de 60% MA sous la dénomination de TRITON CG 110 par la Société SEPPIC | | 5,0 | g |
| - Conservateurs | | 0,15 | g |
| - pH spontané = 6,7 | | | |
| - Eau | qsp | 100,0 | g |

Composition (B)

| | | | |
|---|---|---|---|
| - 1,2-naphtoquinone | | 0,4 | g |
| - Monométhyléther de propylèneglycol | | 20,0 | g |
| - Lauryléther sulfate de sodium à 2 moles d'oxyde d'éthylène vendu sous la dénomination de SACTIPON 8533 par la Société LEVER | | 6,0 | g MA |
| - Nonylphénol à 9 moles d'oxyde d'éthylène vendu sous la dénomination de SINNOPAL NP 9 par la Société HENKEL | | 3,0 | g |
| - pH spontané = 3,7 | | | |
| - Eau | qsp | 100,0 | g |

Des cheveux gris à 90% de blancs sont traités pendant 15 minutes avec la composition (A).
Après rinçage, on applique pendant 10 minutes la composition (B).
On rince à nouveau et on sèche. On obtient alors des cheveux teints en gris foncé à reflets bleus.
Les valeurs des potentiels d'oxydoréduction déterminées comme dans l'exemple 1 sont :

$$E_i = 90 \text{ mV } E_q = -55 \text{ mV } \Delta E = 145 \text{ mV}$$

EXEMPLE 24

La composition (A) est identique à celle de l'exemple 23.

Composition (B1)

| | | | |
|---|---|---|---|
| - 1,2-naphtoquinone | | 0,05 | g |
| - Monobutyléther d'éthylèneglycol | | 30,0 | g |
| - pH spontané = 3,5 | | | |
| - Eau | qsp | 100,0 | g |

On applique la composition (A) 15 minutes sur des cheveux gris à 90% de blancs.
On rince puis on applique la composition (B1) pendant 10 minutes.
Après rinçage et séchage, les cheveux sont colorés dans une nuance bleu d'intensité moyenne.
Les valeurs des potentiels d'oxydoréduction déterminées comme dans l'exemple 1 sont :

$$E_i = 90\text{ mV } E_q = -55\text{ mV } \Delta E = 145\text{ mV}$$

EXEMPLE 25

La composition (A) est identique à celle de l'exemple 23.

Composition (B2)

| | | | |
|---|---|---|---|
| - Sel de potassium de l'acide 1,2-naphtoquinone 4-sulfonique | | 0,9 | g |
| - Alcool éthylique | | 10,0 | g |
| - pH spontané = 3,4 | | | |
| - Eau | qsp | 100,0 | g |

La composition (A) est appliquée 15 minutes sur des cheveux gris à 90% de blancs.
Après rinçage, les cheveux sont traités pendant 10 minutes avec la composition (B2).
On rince à nouveau les cheveux et on les sèche.
On obtient alors des cheveux teints en gris naturel très foncé.
Les valeurs des potentiels d'oxydoréduction déterminées comme dans l'exemple 1 sont :

$$E_i = 90\text{ mV } E_q = -20\text{ mV } \Delta E = 110\text{ mV}$$

EXEMPLE 26

La composition et le mode de prétraitement sont les mêmes que dans l'exemple 16. On les fait suivre d'une révélation par une solution hydroalcoolique 50/50 à 2% de 2-chloro 1,4-benzoquinone pendant 10 minutes. On rince, lave et sèche les cheveux.
La couleur obtenue est noir intense.
Les valeurs des potentiels d'oxydoréduction déterminées comme dans l'exemple 1 sont :

$$E_i = 90\text{ mV } E_q = 20\text{ mV } \Delta E = 70\text{ mV}$$

EXEMPLE 27

Suivant le même procédé qu'à l'exemple 15, mais en remplaçant la toluquinone par la 2-ß-hydroxyéthylthio 1,4-benzoquinone, on obtient une coloration noire.
Les valeurs des potentiels d'oxydoréduction déterminées comme dans l'exemple 1 sont :

$$E_i = 90\text{ mV } E_q = 25\text{ mV } \Delta E = 65\text{ mV}$$

EXEMPLE 28

Suivant le même procédé qu'à l'exemple 15, mais en remplaçant la toluquinone par la 2,5-bis-β-hydroxyéthylthio 1,4-benzoquinone, on obtient une teinte châtain foncé.

Les valeurs des potentiels d'oxydoréduction déterminées comme dans l'exemple 1 sont :

$$E_i = 90 \text{ mV } E_q = 30 \text{ mV } \Delta E = 60 \text{ mV}$$

EXEMPLE 29

Suivant le même procédé qu'à l'exemple 15, mais en remplaçant la toluquinone par la 2-β-carboxyéthylthio 1,4-benzoquinone, on obtient une teinte châtain foncé.

Les valeurs des potentiels d'oxydoréduction déterminées comme dans l'exemple 1 sont :

$$E_i = 90 \text{ mV } E_q = 40 \text{ mV } \Delta E = 50 \text{ mV}$$

EXEMPLE 30

Suivant le même procédé qu'à l'exemple 15, mais en remplaçant la toluquinone par la 2-méthylthiométhyl 1,4-benzoquinone, on obtient une coloration noire.

Les valeurs des potentiels d'oxydoréduction déterminées comme dans l'exemple 1 sont :

$$E_i = 90 \text{ mV } E_q = 45 \text{ mV } \Delta E = 45 \text{ mV}$$

EXEMPLE 31

Suivant le même procédé qu'à l'exemple 15, mais en remplaçant la toluquinone par la 2-β-hydroxyéthyl thiométhyl 1,4-benzoquinone, on obtient une coloration châtain foncé.

Les valeurs des potentiels d'oxydoréduction déterminées comme dans l'exemple 1 sont :

$$E_i = 90 \text{ mV } E_q = 55 \text{ mV } \Delta E = 35 \text{ mV}$$

EXEMPLE 32

Suivant le même procédé qu'à l'exemple 15, mais en remplaçant la toluquinone par la 2-β-carboxyéthoxy 1,4-benzoquinone, on obtient une coloration châtain foncé.

Les valeurs des potentiels d'oxydoréduction déterminées comme dans l'exemple 1 sont :

$$E_i = 90 \text{ mV } E_q = 15 \text{ mV } \Delta E = 105 \text{ mV}$$

EXEMPLE 33

On traite une mèche de cheveux gris à 90% de blancs par une solution hydroalcoolique 90/10 de 2,5% de 2,3-diméthyl 5-hydroxy 6-amino indole. On laisse en contact 15 minutes, rince, essore et applique une solution hydroalcoolique 50/50 contenant 2% de 1,2-naphtoquinone. Le temps de contact est de 15 minutes, on lave, essore, sèche et on obtient une teinte gris foncé.

Les valeurs des potentiels d'oxydoréduction déterminées comme dans l'exemple 1 sont :

$$E_i = 45 \text{ mV } E_q = 55 \text{ mV } \Delta E = 100 \text{ mV}$$

EXEMPLE 34

On traite une mèche de cheveux gris à 90% de blancs par une solution hydroalcoolique 90/10 de 2,5% de 2,3-diméthyl 5-amino 6-hydroxy indole. On laisse en contact 15 minutes, rince, essore et applique une solution hydroalcoolique 50/50 contenant 2% de 1,4-benzoquinone. Le temps de contact est de 15 minutes, on lave, essore, sèche et on obtient une coloration châtain moyen.

Les valeurs des potentiels d'oxydoréduction déterminées comme dans l'exemple 1 sont :

$$E_i = 10 \text{ mV } E_q = 10 \text{ mV } \Delta E = 0$$

EXEMPLE 35

Suivant le même procédé qu'à l'exemple 2, mais en remplaçant la 1,4-benzoquinone par la 2-β-hydroxyéthylthio 6-méthyl 1,4-benzoquinone, on obtient une coloration violette.

Les valeurs des potentiels d'oxydoréduction déterminées comme dans l'exemple 1 sont :

$E_i = 110$ mV $E_q = -50$ mV $\Delta E = 160$ mV

EXEMPLE 36

Suivant le même procédé qu'à l'exemple 2, mais en remplaçant la 1,4-benzoquinone par la 2-méthoxyméthyl 1,4-benzoquinone, on obtient une mèche colorée intensément en violet.

Les valeurs des potentiels d'oxydoréduction déterminées comme dans l'exemple 1 sont :

$E_i = 110$ mV $E_q = 25$ mV $\Delta E = 85$ mV

EXEMPLE 37

Suivant le même procédé qu'à l'exemple 5, mais en remplaçant la 1,4-benzoquinone par la 2-β, γ-dihydroxypropylthio 1,4-benzoquinone, on obtient une coloration gris foncé.

Les valeurs des potentiels d'oxydoréduction déterminées comme dans l'exemple 1 sont :

$E_i = 180$ mV $E_q = 30$ mV $\Delta E = 150$ mV

EXEMPLE 38

Suivant le même procédé qu'à l'exemple 15, mais en remplaçant la toluquinone par la 2-β-hydroxyéthoxy 1,4-benzoquinone, on obtient une coloration châtain foncé.

Les valeurs des potentiels d'oxydoréduction déterminées comme dans l'exemple 1 sont :

$E_i = 90$ mV $E_q = -50$ mV $\Delta E = 140$ mV

EXEMPLE 39

Suivant le même procédé qu'à l'exemple 15, mais en remplaçant la toluquinone par la N-méthyl-sulfonyl 1,4-benzoquinone monoimine, on obtient une coloration châtain foncé.

Les valeurs des potentiels d'oxydoréduction déterminées comme dans l'exemple 1 sont :

$E_i = 90$ mV $E_q = 65$ mV $\Delta E = 25$ mV

EXEMPLE 40

Suivant le même procédé qu'à l'exemple 15, mais en remplaçant la toluquinone par la N-phényl-sulfonyl 1,4-benzoquinone monoimine, on obtient une coloration châtain moyen.

Les valeurs des potentiels d'oxydoréduction déterminées comme dans l'exemple 1 sont :

$E_i = 90$ mV $E_q = 60$ mV $\Delta E = 30$ mV

**Revendications pour les Etats contractants suivants : AT, BE, CH, DE, FR, GB, GR, IT, LI, NL**

1. Procédé de teinture des fibres kératiniques caractérisé par le fait que l 'on applique sur ces fibres au moins une composition A contenant dans un milieu approprié pour la teinture au moins un mono- ou dihydroxyindole, l'application de la composition A étant précédée ou suivie par l'application d'une composition B contenant, dans un milieu approprié pour la teinture, au moins un dérivé quinonique choisi parmi les ortho- ou para-benzoquinones, les ortho- ou para-benzoquinones monoimines ou diimines, les 1,2- ou 1,4-naphtoquinones, les ortho- ou para-benzoquinones sulfonimides, les α,ω-alkylène bis-1,4-benzoquinones ou les 1,2- ou 1,4-naphtoquinones monoimines ou diimines, les mono- ou dihydroxyindoles et les dérivés quinoniques étant choisis de façon à ce que la différence de potentiel d'oxydoréduction $\Delta E$ entre le potentiel d'oxydoréduction $E_i$ des mono- ou dihydroxyindoles déterminé à pH 7 en milieu phosphate sur électrode de carbone vitreux par voltamétrie, et le potentiel d'oxydoréduction $E_q$ du dérivé quinonique déterminé à pH 7 en milieu phosphate par polarographie sur électrode de mercure par rapport à l'électrode au calomel saturé soit telle que :

   $$\Delta E = E_i - E_q \leq 320 \text{ mV.}$$

2. Procédé selon la revendication 1, caractérisé par le fait que les mono- ou dihydroxyindoles sont choisis parmi les composés répondant à la formule :

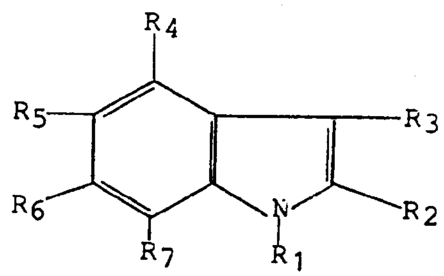

(I)

dans laquelle :

$R_1$ désigne hydrogène ou un groupement alkyle en $C_1$-$C_4$;

$R_2$ et $R_3$, identiques ou différents, représentent un atome d'hydrogène, un groupement alkyle en $C_1$-$C_4$, un groupement carboxyle, un groupement alcoxy ($C_1$-$C_4$)carbonyle, hydroxyalkyle ($C_1$-$C_4$), aminoalkyle ($C_1$-$C_4$);

$R_4$, $R_5$, $R_6$ et $R_7$, indépendamment l'un de l'autre, représentent un atome d'hydrogène, un radical alkyle en $C_1$-$C_4$, amino substitué ou non par un ou deux groupements alkyle ou hydroxyalkyle en $C_1$-$C_4$, acyle ($C_2$-$C_6$)amino, carboxyle, carboxyalkyle en $C_1$-$C_4$, alcoxy en $C_1$-$C_4$ carbonyle,

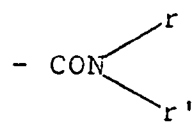

(r et r', indépendamment l'un de l'autre, désignant hydrogène, alkyle en $C_1$-$C_4$), halogène, hydroxyalkyle en $C_1$-$C_4$, amino alkyle en $C_1$-$C_4$, OH ou OZ, Z désignant un radical alkyle, linéaire ou ramifié en $C_1$-$C_{20}$, un groupement aralkyle, un groupement formyle, un groupement acyle en $C_2$-$C_{20}$, linéaire ou ramifié, un groupement alcénoyle en $C_3$-$C_{20}$, linéaire ou ramifié, un groupement $-SiR_{11}R_{12}R_{13}$, un groupement $-P(O)(OR_8)_2$, un groupement $R_8OSO_2$-; les radicaux $R_4$ et $R_5$ ou bien $R_5$ et $R_6$ ou bien $R_6$ et $R_7$ pouvant former, conjointement avec les atomes de carbone auxquels ils sont liés, un cycle contenant éventuellement un groupement carbonyle, un groupement thiocarbonyle, un groupement $>P(O)(OR_8)$ ou un groupement $>CR_9R_{10}$, sous réserve qu'au moins l'un des radicaux $R_4$ à $R_7$ représente un groupement OH;

$R_8$ et $R_9$ représentent un atome d'hydrogène ou un groupement alkyle en $C_1$-$C_4$;

$R_{10}$ représente un groupement alcoxy en $C_1$-$C_4$ ou un groupement mono- ou dialkyl ($C_1$-$C_4$) amino;

$R_{11}$, $R_{12}$ et $R_{13}$, identiques ou différents, représentent des groupements alkyle en $C_1$-$C_4$, linéaires ou ramifiés, et

les sels correspondants de métaux alcalins, alcalino-terreux, d'ammonium et d'amines de ces composés.

3. Procédé selon la revendication1 ou 2 caractérisé par le fait que les mono- ou dihydroxyindoles sont choisis parmi le :

- le 4-hydroxyindole
- le 4-hydroxy 5-methoxyindole
- le 4-hydroxy 5-éthoxyindole
- le 5-hydroxyindole
- le 2-carboxy 5-hydroxyindole
- le 5-hydroxy 6-méthoxyindole
- le 6-hydroxyindole
- le 6-hydroxy 7-méthoxyindole
- le 5-méthoxy 6-hydroxyindole
- le 2-carboxy 6-hydroxyindole
- le 2-éthoxycarbonyl 6-hydroxyindole
- le 7-hydroxyindole
- le 2,3-diméthyl 7-hydroxy 4-méthoxyindole
- le 5,6-dihydroxyindole
- le 1-méthyl 5,6-dihydroxyindole
- le 2-méthyl 5,6-dihydroxyindole

- le 3-méthyl 5,6-dihydroxyindole
- le 2,3-diméthyl 5,6-dihydroxyindole
- le (5 ou 6)-acétoxy (6 ou 5)-hydroxyindole
- le 2-éthoxycarbonyl 5,6-dihydroxyindole
- le 2-carboxy 5,6-dihydroxyindole.
- le 2,3-diméthyl 5-hydroxy 6-amino indole
- le 2,3-diméthyl 5-amino 6-hydroxyindole.

**4.** Procédé selon l'une quelconque des revendications 1 à 3, caractérisé par le fait que les dérivés quinoniques sont choisis parmi les composés répondant aux formules (II) et (II') :

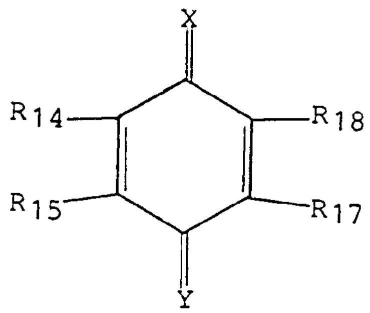

(II)

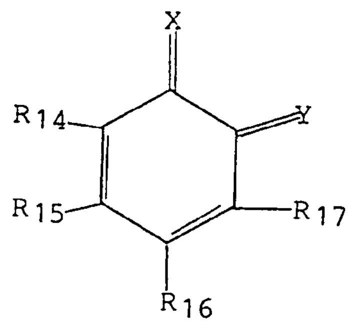

(II')

dans lesquelles :

X désigne oxygène ou un groupement $NR_{19}$;

Y désigne oxygène ou un groupement $NR_{20}$;

$R_{19}$ et $R_{20}$, identiques ou différents, désignant hydrogène, halogène, un radical alkyle, hydroxyalkyle en $C_1$-$C_4$, un radical alkyl($C_1$-$C_4$)sulfonyle ou un radical phénylsulfonyle;

$R_{14}$, $R_{15}$, $R_{16}$, $R_{17}$ et $R_{18}$ désignent, indépendamment l'un de l'autre, hydrogène, un radical alkyle en $C_1$-$C_4$, un groupement carboxyle, alkyl($C_1$-$C_4$)carbonyle, alcoxy($C_1$-$C_4$) carbonyle, alcoxy($C_1$-$C_4$)méthyle, alkyl ($C_1$-$C_4$)thiométhyle, hydroxyalkyl($C_1$-$C_4$)thiométhyle, hydroxyalkyl($C_1$-$C_4$)sulfinyle,

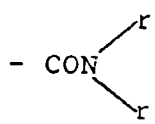

(r et r' désignant, indépendamment l'un de l'autre, hydrogène ou alkyle en $C_1$-$C_4$), carboxyalkyle, halogène, hydroxyalkyle en $C_1$-$C_4$, amino substitué ou non par un ou deux groupements alkyle ou hydroxyalkyle en $C_1$-$C_4$, acyl($C_2$-$C_6$)amino, $SO_3M$ où M désigne hydrogène, K ou Na, un groupement sulfoxyde, sulfone ou sulfonamide éventuellement substitué, ou bien un radical $OZ_1$ dans lequel $Z_1$ peut être hydrogène, alkyle en $C_1$-$C_4$, hydroxyalkyle ($C_1$-$C_4$), carboxyalkyle ($C_1$-$C_4$), phényle éventuellement substitué par alcoxy en $C_1$-$C_4$, ou encore un radical $-SZ_2$ dans lequel $Z_2$ est un groupement alkyle en $C_1$-$C_4$, hydroxyalkyle en $C_1$-$C_4$, dihydroxyalkyle en $C_2$-$C_4$, carboxyalkyle en $C_1$-$C_4$;

$R_{14}$ et $R_{15}$ pouvant former avec les atomes de carbone auxquels ils sont rattachés, le groupement cyclique suivant :

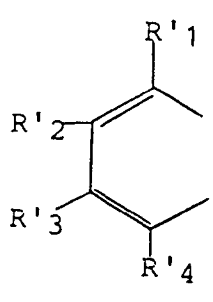

(III)

dans lequel :

$R'_1$, $R'_2$, $R'_3$ et $R'_4$ ont les significations indiquées ci-dessus pour $R_{14}$, $R_{15}$, $R_{16}$, $R_{17}$, $R_{18}$, lorsqu'ils ne forment pas de cycle;

**5.** Procédé selon l'une quelconque des revendications 1 à 4, caractérisé par le fait que les dérivés quinoniques sont choisis parmi les benzoquinones de formules :

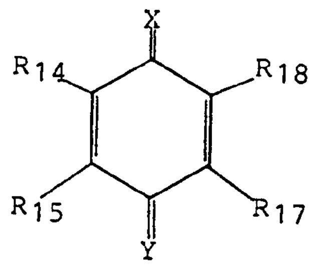

(II)

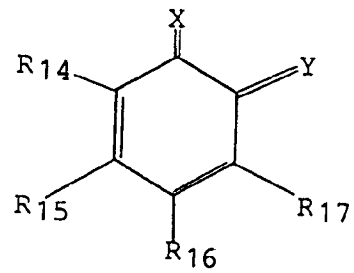

(II')

dans lesquelles :

$R_{14}$, $R_{15}$, $R_{16}$, $R_{17}$, $R_{18}$ désignent, indépendamment l'un de l'autre, hydrogène, alkyle inférieur en $C_1$-$C_4$, alcoxy inférieur en $C_1$-$C_4$, halogène, acylamino en $C_2$-$C_6$, $SO_3M$, alcoxy($C_1$-$C_4$)méthyle carboxyalkyle ($C_1$-$C_4$), alcoxy($C_1$-$C_4$)carbonyle, dialkylamino, $OZ_1$, dans lequel $Z_1$ représente carboxyalkyle ($C_1$-$C_4$), hydroxyalkyle($C_1$-$C_4$), $SZ_2$, dans lequel $Z_2$ représente hydroxyalkyle($C_1$-$C_4$), dihydroxyalkyle($C_1$-$C_4$), carboxyalkyle($C_1$-$C_4$), alkyle($C_1$-$C_4$);

X désigne oxygène ou le groupement $NR_{19}$;

Y désigne oxygène ou le groupement $NR_{20}$,

$R_{19}$ et $R_{20}$ indépendamment l'un de l'autre, désignent hydrogène, halogène, alkyle inférieur en $C_1$-$C_4$, méthylsulfonyle ou phénylsulfonyle;

**6.** Procédé selon l'une quelconque des revendications 1 à 4, caractérisé par le fait que les dérivés quinoniques sont choisis parmi les composés de formules :

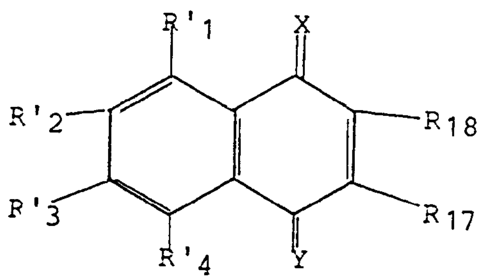

(V)

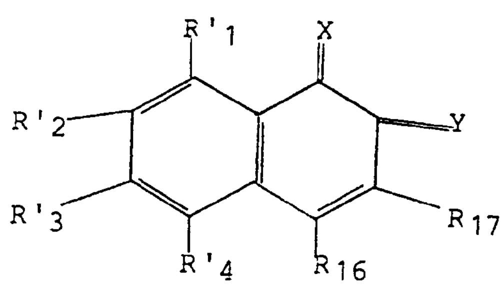

(VI)

dans lesquelles :

$R'_1$,$R'_2$,$R'_3$,$R'_4$,$R_{16}$,$R_{17}$,$R_{18}$ ont les significations indiquées dans les revendications 4 et 5; et

X et Y ont les mêmes significations que celles indiquées dans la revendication 5.

**7.** Procédé selon l'une quelconques des revendications 1 à 5, caractérisé par le fait que les dérivés quinoniques sont choisis parmi les composés suivants : 1,4-benzoquinone, 2-méthoxy 1,4-benzoquinone, 2-méthyl 1,4-benzoquinone, 2,6-diméthyl 1,4-benzoquinone, 2,3,5-trichloro 6-méthyl 1,4-benzoquinone, 2-acétylamino 1,4-benzoquinone,2-acétylamino 3,5-diméthyl 1,4-benzoquinone, 2,6-diméthyl 5-acétylamino 1,4-benzoquinone, tétrachloro 1,2-benzoquinone, 2-chloro 1,4-benzoquinone, 2,3-diméthoxy 1,4-benzoquinone, 2-β-carboxyéthoxy 1,4-benzoquinone, 2-méthoxyméthyl 1,4-benzoquinone, 2-β-hydroxyéthyl 1,4-benzoquinone, 2-β-hydroxyéthyltio 1,4-benzoquinone, 2,5-bis-β-hydroxyéthylthio 1,4-benzoquinone, 2-β, γ-dihydroxypropylthio 1,4-benzoquinone, 2-β-carboxyéthylthio 1,4-benzoquinone, 2-carboxyméthyl 1,4-benzoquinone, 2-β-hydroxyéthylthio 6-méthyl 1,4-benzoquinone, 2-méthoxycarbonyl 3-méthoxy 1,4-benzoquinone, 2-méthoxycarbonyl 1,4-benzoquinone, 2-méthylthio 1,4-benzoquinone, 2-diméthylamino 1,4-benzoquinone, 2-acétylamino 5-méthoxy 1,4-benzoquinone, 2-(β-hydroxyéthylthio)méthyl 1,4-benzoquinone, 2-(méthylthio)méthyl 1,4-benzoquinone, 4,5-diméthoxy 1,2-benzoquinone, 4-méthyl 5-chloro 1,2-benzoquinone, 4,5-diméthyl 1,2-benzoquinone, 2,3-diméthyl 1,4-benzoquinone, 2-β-hydroxyéthoxy 1,4-benzoquinone, N-méthyl sulfonyl 1,4-benzoquinone monoimine, N-phényl sulfonyl 1,4-benzoquinone monoimine, 1,4-naphtoquinone, 1,2-naphtoquinone, acide 1,2-naphtoquinone 4-sulfonique, 2,3-dichloro 1,4-naphtoquinone, N,2,6-trichloro 1,4-benzoquinoneimine.

**8.** Procédé selon l'une quelconque des revendications 1 à 7, caractérisé par le fait que la composition A contient le 5,6-dihydroxyindole et la composition B contient un dérivé quinonique choisi parmi la 1,4-benzoquinone, la 2-méthyl 1,4-benzoquinone, la 2,6-diméthyl 1,4-benzoquinone, la 2-méthoxy 1,4-benzoquinone, la 2-chloro 1,4-benzoquinone, la 2,3,5-trichloro 6-méthyl 1,4-benzoquinone, la 2-acétylamino 1,4-benzoquinone, la 2-acétylamino 3-méthoxy 1,4-benzoquinone, la 2,6-diméthyl 5-acétyl-amino 1,4-benzoquinone, la 2,3-diméthoxy 1,4-benzoquinone, la 2-méthoxyméthyl 1,4-benzoquinone, la 2-β-hydroxyéthyl 1,4-benzoquinone, la 2-β γ-dihydroxypropylthio 1,4-benzoquinone, la 2-β-carboxyéthylthio 1,4-benzoquinone, la 2-carboxyméthyl 1,4-benzoquinone, la 1,4-naphtoquinone, la N-2,6-trichloro 1,4-benzoquinoneimine, la 1,2-naphtoquinone, l'acide 1,2-naphtoquinone 4-sulfonique.

**9.** Procédé selon l'une quelconque des revendications 1 à 8 caractérisé par le fait que le mono- ou le dihydroxyindole est présent dans la composition A en une concentration variant entre 0,01 et 0,3 mole/litre.

**10.** Procédé selon l'une quelconque des revendications 1 à 8, caractérisé par le fait que le dérivé quinonique est présent dans la composition B dans des concentrations comprises entre 0,005 et 1 mole/litre.

**11.** Procédé selon l'une quelconque des revendications 1 à 10, caractérisé par le fait que le pH des compositions A et B est indépendamment l'un de l'autre, compris entre 2 et 10.

**12.** Procédé selon la revendication 11, caractérisé par le fait que le pH de la composition B est acide.

**13.** Procédé selon l'une quelconque des revendications 1 à 12, caractérisé par le fait que les compositions A et B comprennent un milieu aqueux constitué par de l'eau ou un mélange d'eau et d'un solvant.

**14.** Procédé selon l'une quelconque des revendications 1 à 12, caractérisé par le fait que les compositions A et B sont constitutées par un milieu solvant anhydre.

**15.** Procédé selon l'une quelconque des revendications 1 à 14, caractérisé par le fait que les compositions A et B contiennent indépendamment l'une de l'autre des agents tensio-actifs anioniques, cationiques, nonioniques, amphotères ou leurs mélanges, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

**16.** Procédé selon l'une quelconque des revendications 1 à 15, caractérisé par le fait que les compositions A et/ou B contiennent d'autres colorants choisis parmi les colorants directs, les colorants d'oxydation, les coupleurs ou les colorants d'oxydation dits "rapides".

**17.** Procédé selon l'une quelconque des revendications 1 à 16, caractérisé par le fait que la composition A et/ou B contient également des colorants quinoniques de la famille des benzoquinones des benzoquinonimines ou diimines, des naphtoquinones, des naphtoquinonimines, des naphtoquinonediimines ou des indoles quinones dont les potentiels sont tels que $\Delta E$ soit supérieur à 320 mV.

**18.** Dispositif à plusieurs compartiments ou kit de teinture, caractérisé par le fait qu'il comprend dans un premier compartiment la composition A telle que définie dans l'une quelconque des revendications 1 à 17, et dans un second compartiment une composition B telle que définie dans l'une quelconque des revendications 1 à 17.

**19.** Dispositif selon la revendication 18, caractérisé par le fait que l'une au moins des deux compositions A et B comportent un milieu solvant anhydre et un troisième compartiment contenant un milieu aqueux pour la teinture et destiné à être mélangé tout juste avant l'emploi avec le contenu de l'un ou l'autre des deux

compartiments contenant les compositions anhydres A et B.

**20.** Composé nouveau répondant à la formule :

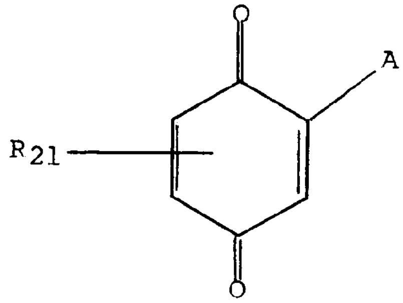

(IV)

dans laquelle A représente les groupements $CH_2SR$, dans lesquels R désigne un groupement alkyle en $C_1$-$C_4$ ou hydroxyalkyle en $C_1$-$C_4$; $R_{21}$ représente un atome d'hydrogène.

**21.** Composé nouveau répondant à la formule :

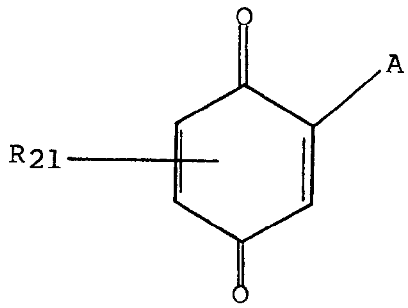

(IV)

dans laquelle A représente le groupement SR, dans lequel R désigne un groupement hydroxyalkyle en $C_1$-$C_4$; $R_{21}$ représente un atome d'hydrogène, un radical alkyle en $C_1$-$C_4$ ou le groupement SR, R ayant la signification mentionnée ci-dessus.

**22.** Utilisation du composé selon la revendication 20 ou 21, dans des compositions tinctoriales pour fibres kératiniques.

**23.** Procédé de préparation des composés de formule (IV), dans laquelle A désigne $CH_2SR$ et $R_{21}$ hydrogène, caractérisé par le fait que l'on procède à l'oxydation d'un paraaminophénol de formule :

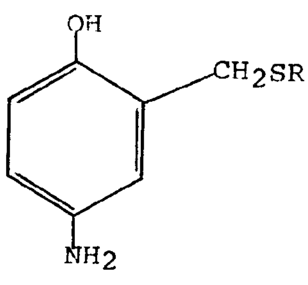

(VII)

avec du chlorure ferrique ou un sulfate ferrique, dans laquelle R a les significations indiquées dans la revendication 20.

**Revendications pour l'Etat contractant suivant : ES**

**1.** Procédé de teinture des fibres kératiniques caractérisé par le fait que l'on applique sur ces fibres au moins une composition A contenant dans un milieu approprié pour la teinture au moins un mono- ou dihydroxyindole, l'application de la composition A étant précédée ou suivie par l'application d'une composition B contenant, dans un milieu approprié pour la teinture, au moins un dérivé quinonique choisi parmi les ortho- ou para-benzoquinones, les ortho- ou para-benzoquinones monoimines ou diimines, les 1,2- ou 1,4-naphtoquinones, les ortho- ou para-benzoquinones sulfonimides, les $\alpha,\omega$-alkylène bis-1,4-benzoquinones ou les 1,2- ou 1,4-naphtoquinones monoimines ou diimines, les mono- ou dihydroxyindoles et les dérivés quinoniques étant choisis de façon à ce que la différence de potentiel d'oxydoréduction $\Delta E$ entre le potentiel d'oxydoréduction $E_i$ des mono- ou dihydroxyindoles déterminé à pH 7 en milieu phosphate sur électrode de carbone vitreux par voltamétrie, et le potentiel d'oxydoréduction $E_q$ du dérivé quinonique déterminé à pH 7 en milieu phosphate par polarographie sur électrode de mercure par rapport à l'électrode au calomel saturé soit telle que :

$$\Delta E = E_i - E_q \leqq 320 \text{ mV}.$$

2. Procédé selon la revendication 1, caractérisé par le fait que les mono- ou dihydroxyindoles sont choisis parmi les composés répondant à la formule :

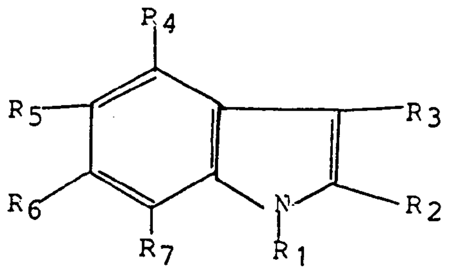

(I)

dans laquelle :

$R_1$ désigne hydrogène ou un groupement alkyle en $C_1$-$C_4$;

$R_2$ et $R_3$, identiques ou différents, représentent un atome d'hydrogène, un groupement alkyle en $C_1$-$C_4$, un groupement carboxyle, un groupement alcoxy ($C_1$-$C_4$)carbonyle, hydroxyalkyle ($C_1$-$C_4$), aminoalkyle ($C_1$-$C_4$);

$R_4$, $R_5$, $R_6$ et $R_7$, indépendamment l'un de l'autre, représentent un atome d'hydrogène, un radical alkyle en $C_1$-$C_4$, amino substitué ou non par un ou deux groupements alkyle ou hydroxyalkyle en $C_1$-$C_4$, acyle ($C_2$-$C_6$)amino, carboxyle, carboxyalkyle en $C_1$-$C_4$, alcoxy en $C_1$-$C_4$ carbonyle

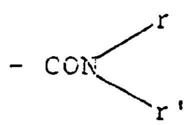

(r et r', indépendamment l'un de l'autre, désignant hydrogène, alkyle en $C_1$-$C_4$), halogène, hydroxyalkyle en $C_1$-$C_4$, amino alkyle en $C_1$-$C_4$, OH ou OZ, Z désignant un radical alkyle, linéaire ou ramifié en $C_1$-$C_{20}$, un groupement aralkyle, un groupement formyle, un groupement acyle en $C_2$-$C_{20}$, linéaire ou ramifié, un groupement alcénoyle en $C_3$-$C_{20}$, linéaire ou ramifié, un groupement $-SiR_{11}R_{12}R_{13}$, un groupement $-P(O)(OR_8)_2$, un groupement $R_8OSO_2$-; les radicaux $R_4$ et $R_5$ ou bien $R_5$ et $R_6$ ou bien $R_6$ et $R_7$ pouvant former, conjointement avec les atomes de carbone auxquels ils sont liés, un cycle contenant éventuellement un groupement carbonyle, un groupement thiocarbonyle, un groupement $>P(O)(OR_8)$ ou un groupement $>OR_9R_{10}$, sous réserve qu'au moins l'un des radicaux $R_4$ à $R_7$ représente un groupement OH;

$R_8$ et $R_9$ représentent un atome d'hydrogène ou un groupement alkyle en $C_1$-$C_4$;

$R_{10}$ représente un groupement alcoxy en $C_1$-$C_4$ ou un groupement mono- ou dialkyl ($C_1$-$C_4$) amino;

$R_{11}$, $R_{12}$ et $R_{13}$, identiques ou différents, représentent des groupements alkyle en $C_1$-$C_4$, linéaires ou ramifiés, et

les sels correspondants de métaux alcalins, alcalino-terreux, d'ammonium et d'amines de ces composés.

3. Procédé selon la revendication 1 ou 2 caractérisé par le fait que les mono- ou dihydroxyindoles sont choisis parmi le :

- le 4-hydroxyindole
- le 4-hydroxy 5-méthoxyindole

- le 4-hydroxy 5-éthoxyindole
- le 5-hydroxyindole
- le 2-carboxy 5-hydroxyindole
- le 5-hydroxy 6-méthoxyindole
- le 6-hydroxyindole
- le 6-hydroxy 7-méthoxyindole
- le 5-méthoxy 6-hydroxyindole
- le 2-carboxy 6-hydroxyindole
- le 2-éthoxycarbonyl 6-hydroxyindole
- le 7-hydroxyindole
- le 2,3-diméthyl 7-hydroxy 4-méthoxyindole
- le 5,6-dihydroxyindole
- le 1-méthyl 5,6-dihydroxyindole
- le 2-méthyl 5,6-dihydroxyindole
- le 3-méthyl 5,6-dihydroxyindole
- le 2,3-diméthyl 5,6-dihydroxyindole
- le (5 ou 6)-acétoxy (6 ou 5)-hydroxyindole
- le 2-éthoxycarbonyl 5,6-dihydroxyindole
- le 2-carboxy 5,6-dihydroxyindole.
- le 2,3-diméthyl 5-hydroxy 6-amino indole
- le 2,3-diméthyl 5-amino 6-hydroxyindole.

4. Procédé selon l'une quelconque des revendications 1 à 3, caractérisé par le fait que les dérivés quinoniques sont choisis parmi les composés répondant aux formules (II) et (II') :

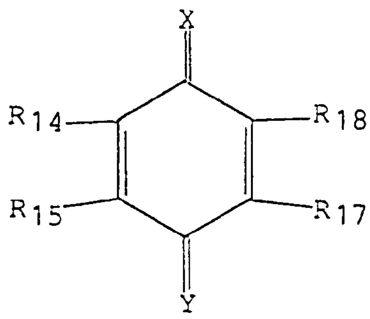

(II)

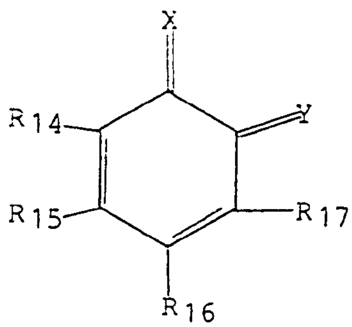

(II')

dans lesquelles :

X désigne oxygène ou un groupement $NR_{19}$;

Y désigne oxygène ou un groupement $NR_{20}$;

$R_{19}$ et $R_{20}$, identiques ou différents, désignant hydrogène, halogène, un radical alkyle, hydroxyalkyle en $C_1$-$C_4$, un radical alkyl ($C_1$-$C_4$)sulfonyle ou un radical phénylsulfonyle;

$R_{14}$, $R_{15}$, $R_{16}$, $R_{17}$ et $R_{18}$ désignent, indépendamment l'un de l'autre, hydrogène, un radical alkyle en $C_1$-$C_4$, un groupement carboxyle, alkyl($C_1$-$C_4$)carbonyle, alcoxy($C_1$-$C_4$)carbonyle, alcoxy($C_1$-$C_4$)méthyle, alkyl($C_1$-$C_4$)thiométhyle, hydroxyalkyl($C_1$-$C_4$)thiométhyle, hydroxyalkyl ($C_1$-$C_4$)sulfinyle

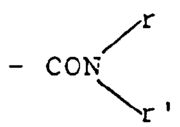

(r et r' désignant, indépendamment l'un de l'autre, hydrogène ou alkyle en $C_1$-$C_4$), carboxyalkyle, halogène, hydroxyalkyle en $C_1$-$C_4$, amino substitué ou non par un ou deux groupements alkyle ou hydroxyalkyle en $C_1$-$C_4$, acyl($C_2$-$C_6$)amino, $SO_3M$ où M désigne hydrogène, K ou Na, un groupement sulfoxyde, sulfone ou sulfonamide éventuellement substitué, ou bien un radical $OZ_1$ dans lequel $Z_1$ peut être hydrogène, alkyle en $C_1$-$C_4$, hydroxyalkyle ($C_1$-$C_4$), carboxyalkyle ($C_1$-$C_4$), phényle éventuellement substitué par alcoxy

en $C_1$-$C_4$, ou encore un radical -$SZ_2$ dans lequel $Z_2$ est un groupement alkyle en $C_1$-$C_4$, hydroxayalkyle en $C_1$-$C_4$, dihydroxyalkyle en $C_2$-$C_4$, carboxyalkyle en $C_1$-$C_4$;

$R_{14}$ et $R_{15}$ pouvant former avec les atomes de carbone auxquels ils sont rattachés, le groupement cyclique suivant :

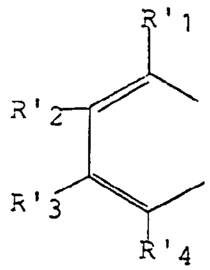

(III)

dans lequel :

$R'_1$, $R'_2$, $R'_3$ et $R'_4$ ont les significations indiquées ci-dessus pour $R_{14}$, $R_{15}$, $R_{16}$, $R_{17}$, $R_{18}$, lorsqu'ils ne forment pas de cycle;

**5.** Procédé selon l'une quelconque des revendications 1 à 4, caractérisé par le fait que les dérivés quinoniques sont choisis parmi les benzoquinones de formules :

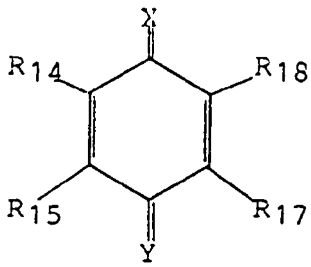

(II)

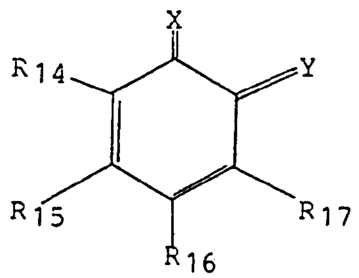

(II')

dans lesquelles :

$R_{14}$, $R_{15}$, $R_{16}$, $R_{17}$, $R_{18}$ désignent, indépendamment l'un de l'autre, hydrogène, alkyle inférieur en $C_1$-$C_4$, alcoxy inférieur en $C_1$-$C_4$, halogène acylamino en $C_2$-$C_6$, $SO_3M$, alcoxy($C_1$-$C_4$)méthyle, carboxyalkyle ($C_1$-$C_4$), alcoxy($C_1$-$C_4$)carbonyle, dialkylamino, $OZ_1$, dans lequel $Z_1$ représente carboxyalkyle ($C_1$-$C_4$), hydroxyalkyle($C_1$-$C_4$), $SZ_2$, dans lequel $Z_2$ représente hydroxyalkyle($C_1$-$C_4$), dihydroxyalkyle ($C_1$-$C_4$), carboxyalkyle($C_1$-$C_4$), alkyle($C_1$-$C_4$);

X désigne oxygène ou le groupement $NR_{19}$;

Y désigne oxygène ou le groupement $NR_{20}$, $R_{19}$ et $R_{20}$, indépendamment l'un de l'autre, désignent hydrogène, halogène, alkyle inférieur en $C_1$-$C_4$, méthylsulfonyle ou phénylsulfonyle;

**6.** Procédé selon l'une quelconque des revendications 1 à 4, caractérisé par le fait que les dérivés quinoniques sont choisis parmi les composés de formules :

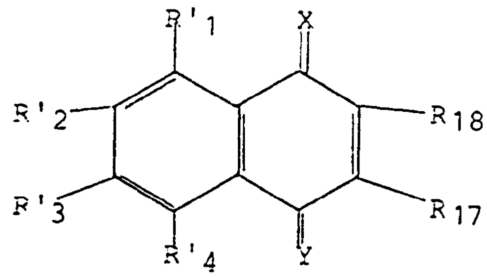

(V)

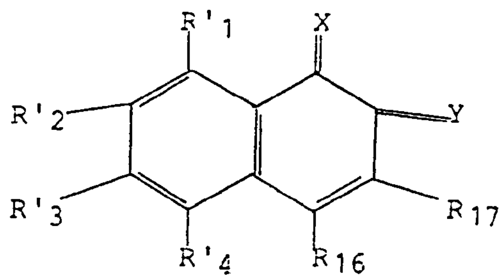

(VI)

dans lesquelles :

$R'_1$,$R'_2$,$R'_3$,$R'_4$,$R_{16}$,$R_{17}$,$R_{18}$ ont les significations indiquées dans les revendications 4 et 5; et X et Y ont les mêmes significations que celles indiquées dans la revendication 5.

7. Procédé selon l'une quelconque des revendications 1 à 5, caractérisé par le fait que les dérivés quinoniques sont choisis parmi les composés suivants : 1,4-benzoquinone, 2-méthoxy 1,4-benzoquinone, 2-méthyl, 1,4-benzoquinone, 2,6-diméthyl 1,4-benzoquinone, 2,3,5-trichloro 6-méthyl 1,4-benzoquinone 2-acétylamino 1,4-benzoquinone,2-acétylamino 3,5-diméthyl 1,4-benzoquinone, 2,6-diméthyl 5-acétylamino 1,4-benzoquinone, tétrachloro 1,2-benzoquinone, 2-chloro 1,4-benzoquinone, 2,3-diméthoxy 1,4-benzoquinone, 2-β-carboxyéthoxy 1,4-benzoquinone, 2-méthoxyméthyl 1,4-benzoquinone, 2-β-hydroxyéthyl 1,4-benzoquinone, 2-β-hydroxyéthylthio 1,4-benzoquinone, 2,5-bis-β-hydroxyéthylthio 1,4-benzoquinone, 2-β, γ-dihydroxypropylthio 1,4-benzoquinone, 2-β-carboxyéthylthio 1,4-benzoquinone, 2-carboxyméthyl 1,4-benzoquinone, 2-β-hydroxyéthylthio 6-méthyl 1,4-benzoquinone, 2-méthoxycarbonyl 3-méthoxy 1,4-benzoquinone, 2-méthoxycarbonyl 1,4-benzoquinone, 2-méthylthio 1,4-benzoquinone, 2-diméthylamino 1,4-benzoquinone, 2-acétylamino 5-méthoxy 1,4-benzoquinone, 2-(β-hydroxyéthylthio)méthyl 1,4-benzoquinone, 2-(méthylthio)méthyl 1,4-benzoquinone, 4,5-diméthoxy 1,2-benzoquinone, 4-méthyl 5-chloro 1,2-benzoquinone, 4,5-diméthyl 1,2-benzoquinone, 2,3-diméthyl 1,4-benzoquinone, 2-β-hydroxyéthoxy 1,4-benzoquinone, N-méthyl sulfonyl 1,4-benzoquinone monoimine, N-phenyl sulfonyl 1,4 benzoquinone monoimine, 1,4-naphtoquinone, 1,2-naphtoquinone, acide 1,2-naphtoquinone 4-sulfonique, 2,3-dichloro 1,4-naphtoquinone, N,2,6-trichloro 1,4-benzoquinoneimine.

8. Procédé selon l'une quelconque des revendications 1 à 7, caractérisé par le fait que la composition A contient le 5,6-dihydroxyindole et la composition B contient un dérivé quinonique choisi parmi la 1,4-benzoquinone, la 2-méthyl 1,4-benzoquinone, la 2,6-diméthyl 1,4-benzoquinone, la 2-méthoxy 1,4-benzoquinone, la 2-chloro 1,4-benzoquinone, la 2,3,5-trichloro 6-méthyl 1,4-benzoquinone, la 2-acétylamino 1,4-benzoquinone, la 2-acétylamino 3-méthoxy 1,4-benzoquinone, la 2,6-diméthyl 5-acétylamino 1,4-benzoquinone, la 2,3-diméthoxy 1,4-benzoquinone, la 2-méthoxyméthyl 1,4-benzoquinone, la 2-β-hydroxyéthyl 1,4-benzoquinone, la 2-β-γ-dihydroxypropylthio 1,4-benzoquinone, la 2-β-carboxyéthylthio 1,4-benzoquinone, la 2-carboxyméthyl 1,4-benzoquinone, la 1,4-naphtoquinone, la N-2,6-trichloro 1,4-benzoquinoneimine, la 1,2-naphtoquinone, l'acide 1,2-naphtoquinone 4-sulfonique.

9. Procédé selon l'une quelconque des revendications 1 à 8 caractérisé par le fait que le mono- ou le dihydroxyindole est présent dans la composition A en une concentration variant entre 0,01 et 0,3 mole/litre.

10. Procédé selon l'une quelconque des revendications 1 à 8, caractérisé par le fait que le dérivé quinonique est présent dans la composition B dans des concentrations comprises entre 0,005 et 1 mole/litre.

11. Procédé selon l'une quelconque des revendications 1 à 10, caractérisé par le fait que le pH des compositions A et B est indépendamment l'un de l'autre, compris entre 2 et 10.

12. Procédé selon la revendication 11, caractérisé par le fait que le pH de la composition B est acide.

13. Procédé selon l'une quelconque des revendications 1 à 12, caractérisé par le fait que les compositions A et B comprennent un milieu aqueux constitué par de l'eau ou un mélange d'eau et d'un solvant.

14. Procédé selon l'une quelconque des revendications 1 à 12, caractérisé par le fait que les compositions A et B sont constituées par un milieu solvant anhydre.

15. Procédé selon l'une quelconque des revendications 1 à 14, caractérisé par le fait que les compositions A et B contiennent indépendamment l'une de l'autre des agents tensio-actifs anioniques, cationiques, non-ioniques, amphotères ou leurs mélanges, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

16. Procédé selon l'une quelconque des revendications 1 à 15, caractérisé par le fait que les compositions A et/ou B contiennent d'autres colorants choisis parmi les colorants directs, les colorants d'oxydation, les coupleurs ou les colorants d'oxydation dits "rapides".

17. Procédé selon l'une quelconque des revendications 1 à 16, caractérisé par le fait que la composition A et/ou B contient également des colorants quinoniques de la famille des benzoquinones des benzoquinonimines ou diimines, des naphtoquinones, des naphtoquinonimines, des naphtoquinonediimines ou des indoles quinones dont les potentiels sont tels que $\Delta E$ soit supérieur à 320mV.

18. Dispositif à plusieurs compartiments ou kit de teinture, caractérisé par le fait qu'il comprend dans un premier compartiment la composition A telle que définie dans l'une quelconque des revendications 1 à 17, et dans un second compartiment une composition B telle que définie dans l'une quelconque des revendications 1 à 17.

19. Dispositif selon la revendication 18, caractérisé par le fait que l'une au moins des deux compositions A et B comportent un milieu solvant anhydre et un troisième compartiment contenant un milieu aqueux pour la teinture et destiné à être mélangé tout juste avant l'emploi avec le contenu de l'un ou l'autre des deux compartiments contenant les compositions anhydres A et B.

20. Procédé de préparation des composés de formule (IV) :

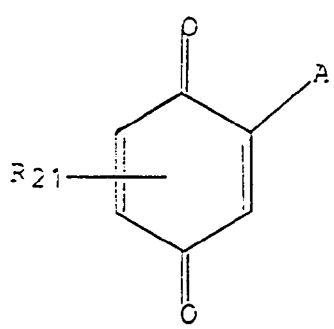

(IV)

dans laquelle A désigne $CH_2SR$, $R_{21}$ désigne hydrogène, et R désigne un groupement alkyle en $C_1$-$C_4$ ou hydroxyalkyle en $C_1$-$C_4$, caractérisé par le fait que l'on procède à l'oxydation d'un paraaminophénol de formule :

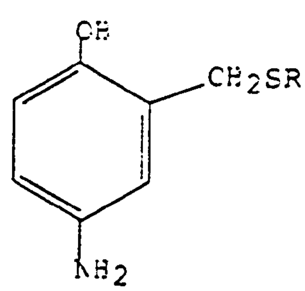

(VII)

avec du chlorure ferrique ou un sulfate ferrique.

21. Procédé de préparation des composés de formule (IV) :

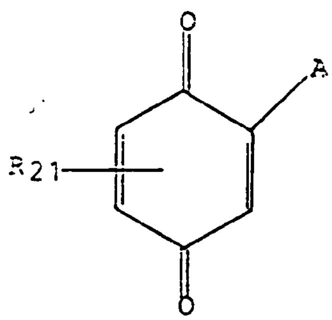

(IV)

dans laquelle A désigne SR, $R_{21}$ représentant hydrogène SR ou alkyle en $C_1$-$C_4$, et R désigne un groupement hydroxyalkyle en $C_1$-$C_4$, caractérisé par le fait que

si $R_{21}$ désigne alkyle en $C_1$-$C_4$ ou hydrogène :

on fait réagir environ 2 moles d'un dérivé quinonique de formule :

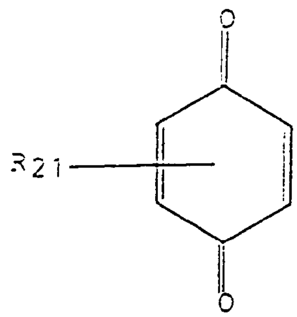

(X)

avec environ un mole de thiol de formule RSH.

si $R_{21}$ désigne SR :

on fait réagir environ 3 moles d'un thiol de formule RSH avec environ 4 moles de p-benzoquinone en milieu éthanol/eau ou éthanol.

22. Procédé de préparation d'un "kit" à plusieurs compartiments destiné à être utilisé pour la teinture des fibres kératiniques, caractérisé par le fait que l'on prépare une composition (A) en introduisant dans un milieu approprié pour la teinture, au moins un mono- ou polyhydroxyindole de formule :

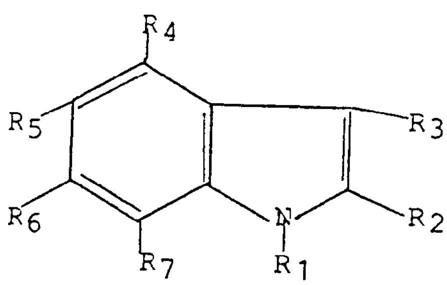

(I)

dans laquelle :

$R_1$ désigne hydrogène ou un groupement alkyle en $C_1$-$C_4$;

$R_2$ et $R_3$, identiques ou différents représentent un atome d'hydrogène, un groupement alkyle en $C_1$-$C_4$, un groupement carboxyle, un groupement alcoxy($C_1$-$C_4$)carbonyle, hydroxyalkyle($C_1$-$C_4$), aminoalkyle($C_1$-$C_4$);

$R_4$, $R_5$, $R_6$ et $R_7$, indépendamment l'un de l'autre, représentent un atome d'hydrogène, un radical alkyle en $C_1$-$C_4$, amino substitué ou non par un ou deux groupements alkyle ou hydroxyalkyle en $C_1$-$C_4$, acyle($C_2$-$C_6$)amino, carboxyle, carboxyalkyle en $C_1$-$C_4$, alcoxy en $C_1$-$C_4$ carbonyle,

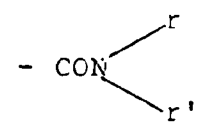

(r et r′, indépendamment l'un de l'autre, désignant hydrogène, alkyle en $C_1$-$C_4$), halogène, hydroxyalkyle en $C_1$-$C_4$, amino alkyle en $C_1$-$C_4$, OH ou OZ, Z désignant un radical alkyle , linéaire ou ramifié en $C_1$-$C_{20}$, un groupement aralkyle, un groupement formyle, un groupement acyle en $C_2$-$C_{20}$, linéaire ou ramifié, un groupement alcénoyle en $C_3$-$C_{20}$, linéaire ou ramifié, un groupement -$SiR_{11}R_{12}R_{13}$, un groupement -$P(O)(OR_8)_2$, un groupement $R_8OSO_2$–; les radicaux $R_4$ et $R_5$ ou bien $R_5$ et $R_6$ ou bien $R_6$ et $R_7$ pouvant former, conjointement avec les atomes de carbone auxquels ils sont liés, un cycle contenant éventuellement un groupement carbonyle, un groupement thiocarbonyle, un groupement $>P(O)(OR_8)$ ou un groupement $>CR_9R_{10}$, sous réserve qu'au moins l'un des radicaux $R_4$ à $R_7$ représente un groupement OH;

$R_8$ et $R_9$ représentent un atome d'hydrogène ou un groupement alkyle en $C_1$-$C_4$;

$R_{10}$ représente un groupement alcoxy en $C_1$-$C_4$ ou un groupement mono- ou dialkyl ($C_1$-$C_4$) amino;

$R_{11}$, $R_{12}$ et $R_{13}$, identiques ou différents, représentent des groupements alkyle en $C_1$-$C_4$, linéaires ou ramifiés, et

les sels correspondants de métaux alcalins, alcalino-terreux, d'ammonium et d'amines de ces composés, dans des proportions comprises entre 0,01 et 0,3 mole/litre, cette composition (A) étant introduite dans un premier compartiment;

que l'on prépare une composition (B) en introduisant dans un milieu approprié pour la teinture, au moins un dérivé quinonique choisi parmi les ortho- ou para-benzoquinones, les ortho- ou para-benzoquinones monoimines ou diimines, les 1,2- ou 1,4-naphtoquinones, les ortho- ou para-benzoquinones sulfonimides, les α,ω-alkylène bis-1,4-benzoquinones ou les 1,2- ou 1,4-naphtoquinones monoimines ou diimines, les mono- ou dihydroxyindoles et les dérivés quinoniques étant choisis de façon à ce que la différence de potentiel d'oxydoréduction ΔE entre le potentiel d'oxydoréduction $E_i$ des mono- ou dihydroxyindoles déterminé à pH 7 en milieu phosphate sur électrode de carbone vitreux par voltamétrie, et le potentiel d'oxydoréduction $E_q$ du dérivé quinonique déterminé à pH 7 en milieu phosphate par polarographie sur électrode de mercure par rapport à l'électrode au calomel saturé soit telle que :

$$\Delta E = E_i - E_q \leqq 320 \text{ mV}.$$

dans des concentrations comprises entre 0,005 et 1 mole/litre, cette composition (B) étant introduite dans un second compartiment.

**Claims for the following Contracting States : AT, BE, CH, DE, FR, GB, GR, IT, LI, NL**

1. Process for dyeing keratinous fibres characterised in that at least one composition A containing, in a medium suitable for dyeing, at least one mono- or dihydroxyindole is applied to these fibres, the application of the composition A being preceded or followed by the application of a composition B containing, in a medium suitable for dyeing, at least one quinone derivative chosen from ortho- or para-benzoquinones, ortho- or para-benzoquinonemonoimines or diimines, 1,2- or 1,4-naphthoquinones, ortho- or parabenzoquinonesulphonimides, α,ω-alkylene-bis-1,4-benzoquinones or 1,2- or 1,4-naphthoquinonemonoimines or diimines, mono- or dihydroxyindoles, and the quinone derivatives being chosen so that the difference in oxidation-reduction potential ΔE between the oxidation-reduction potential $E_i$ of the mono- or dihydroxyindoles, determined at pH 7 in a phosphate medium by voltametry using a vitrous carbon electrode, and the oxidation-reduction potential Eq of the quinone derivative, determined at pH 7 in a phosphate medium by polarography using a mercury electrode with reference to a saturated calomel electrode, is such that:

$$\Delta E = E_i - E_q \leq 320 \text{ mV}.$$

2. Process according to Claim 1, characterised in that the mono- or dihydroxyindoles are chosen from compounds of the formula:

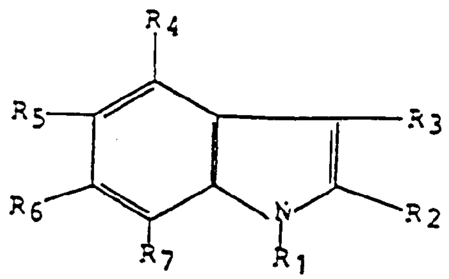

(I)

in which:

$R_1$ denotes hydrogen or a $C_1$-$C_4$ alkyl group;

$R_2$ and $R_3$, which are identical or different, represent a hydrogen atom, a $C_1$-$C_4$ alkyl group, a carboxyl group, a ($C_1$-$C_4$ alkoxy)carbonyl group, a hydroxy($C_1$-$C_4$ alkyl) or an amino($C_1$-$C_4$ alkyl);

$R_4$, $R_5$, $R_6$ and $R_7$, independently of each other, represent a hydrogen atom, a $C_1$-$C_4$ alkyl radical, an amino which is unsubstituted or substituted by one or two $C_1$-$C_4$ alkyl or hydroxy($C_1$-$C_4$ alkyl), ($C_2$-$C_6$ acyl)amino, carboxyl, carboxy ($C_1$-$C_4$ alkyl), or ($C_1$-$C_4$ alkoxy)carbonyl groups, a group

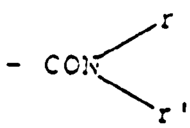

( r and r', independently of each other, denoting hydrogen, $C_1$-$C_4$ alkyl), a halogen, hydroxy($C_1$-$C_4$ alkyl), amino ( $C_1$-$C_4$ alkyl ), OH or OZ groups, Z denoting a linear or branched $C_1$-$C_{20}$ alkyl radical, an aralkyl group, a formyl group, a linear or branched $C_2$-$C_{20}$ acyl group, a linear or branched $C_3$-$C_{20}$ alkenoyl group, a group $-SiR_{11}R_{12}R_{13}$, a group $-P(O)(OR_5)_2$, or a group $R_5OSO_2$–; it being possible for the radicals $R_4$ and $R_5$ or alternatively $R_5$ and $R_6$ or alternatively $R_6$ and $R_7$ to form, together with the carbon atoms to which they are attached, a ring possibly containing a carbonyl group, a thiocarbonyl group, a group $>P(O)(OR_8)$ or a group $>CR_9R_{10}$, provided that at least one of the radicals $R_4$ to $R_7$ represents an OH group;

$R_8$ and $R_9$ represent a hydrogen atom or a $C_1$-$C_4$ alkyl group;

$R_{10}$ represents a $C_1$-$C_4$ alkoxy group or a ($C_1$-$C_4$ mono- or dialkyl )amino group;

$R_{11}$, $R_{12}$ and $R_{13}$, which are identical or different, represent linear or branched $C_1$-$C_4$ alkyl groups, and the corresponding alkali metal, alkaline-earth metal, ammonium and amine salts of these compounds.

**3.** Process according to Claim 1 or 2, characterised in that the mono- or dihydroxyindoles are chosen from:

– 4-hydroxyindole
– 4-hydroxy-5-methoxyindole
– 4-hydroxy-5-ethoxyindole
– 5-hydroxyindole
– 2-carboxy-5-hydroxyindole
– 5-hydroxy-6-methoxyindole
– 6-hydroxyindole
– - 6-hydroxy-7-methoxyindole
– 5-methoxy-6-hydroxyindole
– 2-carboxy-6-hydroxyindole
– 2-ethoxycarbonyl-6-hydroxyindole
– 7-hydroxyindole
– 2,3-dimethyl-7-hydroxy-4-methoxyindole
– 5,6-dihydroxyindole
– 1-methyl-5,6-dihydroxyindole
– 2-methyl-5,6-dihydroxyindole
– 3-methyl-5,6-dihydroxyindole
– 2,3-dimethyl-5,6-dihydroxyindole
– (5 or 6)-acetoxy-(6 or 5)-hydroxyindole

- 2-ethoxycarbonyl-5,6-dihydroxyindole
- 2-carboxy-5,6-dihydroxyindole
- 2,3-dimethyl-5-hydroxy-6-aminoindole
- 2,3-dimethyl-5-amino-6-hydroxyindole.

**4.** Process according to any one of Claims 1 to 3, characterised in that the quinone derivatives are chosen from the compounds of formulae (II) and (II′):

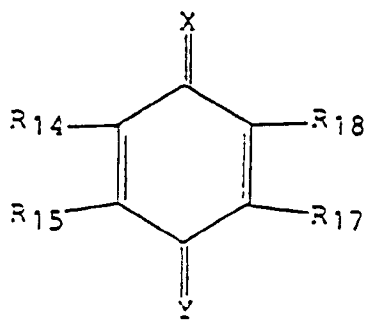

(II)

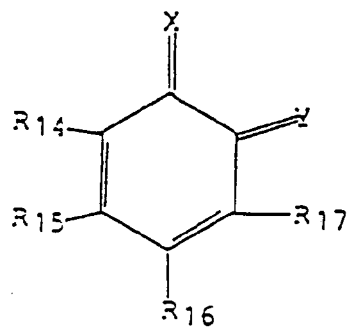

(II')

in which:

X denotes oxygen or a group $NR_{19}$;

Y denotes oxygen or a group $NR_{20}$;

$R_{19}$ and $R_{20}$, which are identical or different, denoting hydrogen, halogen, a $C_1$-$C_4$ alkyl or hydroxy($C_1$-$C_4$ alkyl) radical, a ($C_1$-$C_4$ alkyl)sulphonyl radical or a phenylsulphonyl radical;

$R_{14}$, $R_{15}$, $R_{16}$, $R_{17}$ and $R_{18}$ denote, independently of each other, hydrogen, a $C_1$-$C_4$ alkyl radical, a carboxyl, ($C_1$-$C_4$ alkyl)carbonyl, ($C_1$-$C_4$ alkoxy)carbonyl, ($C_1$-$C_4$ alkoxy)methyl, ($C_1$-$C_4$ alkyl)thiomethyl, hydroxy($C_1$-$C_4$ alkyl)thiomethyl or hydroxy($C_1$-$C_4$ alkyl)sulphinyl group, a group

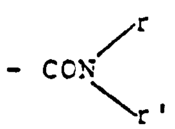

(r and r′ denoting, independently of each other, hydrogen or $C_1$-$C_4$ alkyl), a carboxyalkyl, halogen, hydroxy-($C_1$-$C_4$ alkyl)group, an amino group which is unsubstituted or substituted by one or two $C_1$-$C_4$ alkyl or hydroxy-($C_1$-$C_4$ alkyl)groups, a ($C_2$-$C_6$ acyl) amino group, a group $SO_3M$ where M denotes hydrogen, K or Na, an optionally substituted sulphoxide, sulphone or sulphonamide group, or alternatively a radical $OZ_1$ in which $Z_1$ may be hydrogen, $C_1$-$C_4$ alkyl, hydroxy ($C_1$-$C_4$ alkyl), carboxy-($C_1$-$C_4$ alkyl), phenyl which is optionally substituted by $C_1$-$C_4$ alkoxy, or alternatively a radical $-SZ_2$ in which $Z_2$ is a $C_1$-$C_4$ alkyl, hydroxy($C_1$-$C_4$ alkyl), dihydroxy($C_2$-$C_4$ alkyl) or carboxy($C_1$-$C_4$ alkyl) group;

it being possible for $R_{14}$ and $R_{15}$ to form, with the carbon atoms to which they are attached, the following cyclic group:

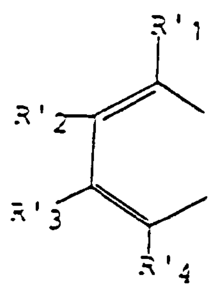

(III)

in which:

$R'_1$, $R'_2$, $R'_3$ and $R'_4$ have the meanings given above for $R_{14}$, $R_{15}$, $R_{16}$, $R_{17}$ and $R_{18}$, when they are not forming a ring.

5. Process according to any one of Claims 1 to 4, characterised in that the quinone derivatives are chosen from benzoquinones of formulae:

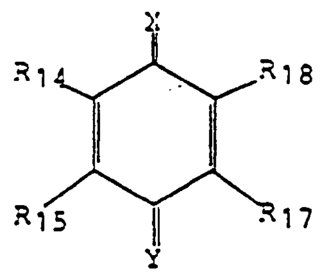

(II)

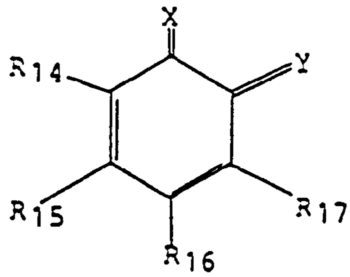

(II')

in which:

$R_{14}$, $R_{15}$, $R_{16}$, $R_{17}$ and $R_{18}$ denote, independently of each other, hydrogen, lower $C_1$-$C_4$ alkyl, lower $C_1$-$C_4$ alkoxy, halogen, ($C_2$-$C_6$ acyl) amino, $SO_3M$, ($C_1$-$C_4$ alkoxy) methyl, carboxy ($C_1$-$C_4$ alkyl), ($C_1$-$C_4$ alkoxy)carbonyl, dialkylamino, $OZ_1$, in which $Z_1$ represents carboxy ($C_1$-$C_4$ alkyl), hydroxy ($C_1$-$C_4$ alkyl), $SZ_2$, in which $Z_2$ represents hydroxy($C_1$-$C_4$ alkyl), dihydroxy($C_1$-$C_4$ alkyl), carboxy($C_1$-$C_4$ alkyl) or $C_1$-$C_4$ alkyl;

X denotes oxygen or the group $NR_{19}$;

Y denotes oxygen or the group $NR_{20}$, $R_{19}$ and $R_{20}$, independently of each other, denote hydrogen, halogen, lower $C_1$-$C_4$ alkyl, methylsulphonyl or phenylsulphonyl.

6. Process according to any one of Claims 1 to 4, characterised in that the quinone derivatives are chosen from the compounds of formulae:

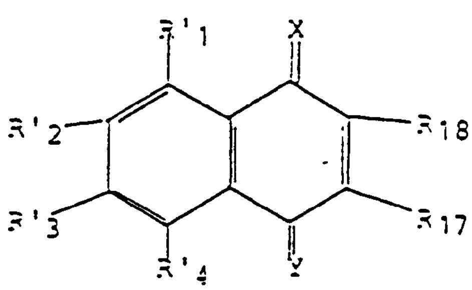

(V)

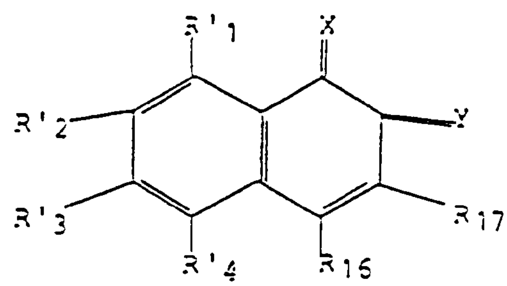

(VI)

in which:

$R'_1$, $R'_2$, $R'_3$, $R'_4$, $R_{16}$, $R_{17}$ and $R_{18}$ have the meanings given in Claims 4 and 5; and

X and Y have the same meanings as those given in Claim 5.

7. Process according to any one of Claims 1 to 5, characterised in that the quinone derivatives are chosen from the following compounds: 1,4-benzoquinone, 2-methoxy-1,4-benzoquinone, 2-methyl-1,4-benzoquinone, 2,6-dimethyl -1,4-benzoquinone, 2,3,5-trichloro-6-methyl- ,4-benzoquinone,2-acetylamino-1,4-benzoquinone,2-acetylamino -3,5-dimethyl-1,4-benzoquinone, 2,6-dimethyl-5 -acetylamino-1,4-benzoquinone, tetrachloro-1,2-benzoquinone, 2-chloro-1,4-benzoquinone, 2,3-dimethoxyl-1,4-benzoquinone, 2-β-carboxyethoxy-1,4-benzoquinone, 2-methoxymethyl-1,4-benzoquinone, 2-β-hydroxyethyl- 1,4-benzoquinone, 2-β-hydroxyethylthio-1,4-benzoquinone, 2,5-bis-β-hydroxyethylthio-1,4-benzoquinone, 2-β,γ-di-hydroxypropylthio -1,4-benzoquinone, 2-β-carboxyethylthio-1,4-benzoquinone, 2-carboxymethyl-1,4-benzoquinone, 2-β-hydroxyethylthio-6-methyl-1,4-benzoquinone, 2-methoxycarbonyl-3-methoxy-1,4-benzoquinone, 2-methoxycarbonyl -1,4-benzoquinone, 2-methylthio-1,4-benzoquinone, 2-dimethylamino-1,4-benzoquinone, 2-acetylamino-5 -methoxy-1,4-benzoquinone, 2-(β-hydroxyethylthio)methyl- 1,4-benzoquinone, 2-(methylthio)methyl-1,4-benzoquinone, 4,5-dimethoxy-1,2-benzoquinone, 4-methyl-5-chloro- ,2-benzoquinone, 4,5-dimethyl-1,2-benzoquinone, 2,3-dimethyl -1,4-benzoquinone, 2-β-hydroxyethoxy-1,4-benzoquinone, N-(methylsulphonyl-1,4-benzoquinone)monoimine, N-(phenylsulphonyl-1,4-benzoquinone)monoimine, 1,4-naphthoquinone, 1,2-naphthoquinone, 1,2-naphthoquinone-4 -sulphonic acid, 2,3-dichloro-1,4-naphthoquinone and N-(2,6-trichloro-1,4-benzoquinone)imine.

**8.** Process according to any one of Claims 1 to 7, characterised in that the composition A contains 5,6-dihydroxyindole and the composition B contains a quinone derivative chosen from 1,4-benzoquinone, 2-methyl- ,4-benzoquinone, 2,6-dimethyl-1,4-benzoquinone, 2-methoxy-1,4-benzoquinone, 2-chloro-1,4-benzoquinone, 2,3,5-trichloro-6-methyl-1,4-benzoquinone, 2-acetylamino- 1,4-benzoquinone, 2-acetylamino-3-methoxy-1,4-benzoquinone, 2,6-dimethyl-5-acetylamino-1,4-benzoquinone, 2,3-dimethoxy-1,4-benzoquinone, 2-methoxymethyl- 1,4-benzoquinone, 2-β-hydroxyethyl-1,4-benzoquinone, 2-β,γ-dihydroxypropylthio-1,4-benzoquinone, 2-β-carboxyethylthio -1,4-benzoquinone, 2-carboxymethyl-1,4-benzoquinone, 1,4-naphthoquinone, N-(2,6-trichloro-1,4-benzoquinone)imine, 1,2-naphthoquinone, 1,2-naphthoquinone-4 -sulphonic acid.

**9.** Process according to any one of Claims 1 to 8, characterised in that the mono- or dihydroxyindole is present in the composition A in a concentration ranging between 0.01 and 0.3 mol/litre.

**10.** Process according to any one of Claims 1 to 8, characterised in that the quinone derivative is present in the composition B in concentrations of between 0.005 and 1 mol/litre.

**11.** Process according to any one of Claims 1 to 10, characterised in that the pH of the compositions A and B is, independently of each other, between 2 and 10.

**12.** Process according to Claim 11, characterised in that the pH of the composition B is acid.

**13.** Process according to any one of Claims 1 to 12, characterised in that the compositions A and B comprise an aqueous medium consisting of water or a mixture of water and a solvent.

**14.** Process according to any one of Claims 1 to 12, characterised in that the compositions A and B consist of an anhydrous solvent medium.

**15.** Process according to any one of Claims 1 to 14, characterised in that the compositions A and B contain, independently of each other, anionic, cationic, nonionic or amphoteric surface-active agents or mixtures thereof, thickening agents, perfumes, sequestering agents, film-forming agents, treatment agents, dispersing agents, conditioning agents, preserving agents, opacifying agents, keratinous fibre-swelling agents.

**16.** Process according to any one of Claims 1 to 15, characterised in that the compositions A and/or B contain other dyes chosen from direct dyes, oxidation dyes, couplers or so-called "rapid" oxidation dyes.

**17.** Process according to any one of Claims 1 to 16, characterised in that the composition A and/or B also contains quinone dyes of the benzoquinone, benzoquinone-imine or diimine, naphthoquinone, naphthoquinoneimine, naphthoquinonediimine or indolequinone family, whose potentials are such that $\Delta E$ is greater than 320 mV.

**18.** Multicompartment device or dyeing kit, characterised in that it comprises, in a first compartment, the composition A as defined in any one of Claims 1 to 17, and, in a second compartment, a composition B as defined in any one of Claims 1 to 17.

**19.** Device according to Claim 18, characterised in that at least one of the two compositions A and B comprise an anhydrous solvent medium and a third compartment containing an aqueous medium for the dyeing and intended to be mixed immediately before use with the content of one or the other of the two compartments containing the anhydrous compositions A and B.

**20.** Novel compound of the formula:

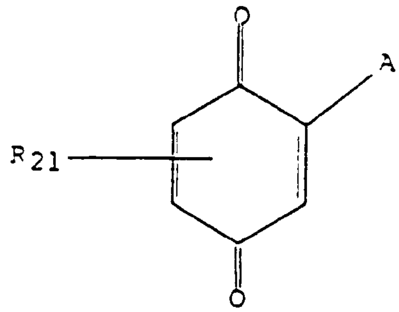

(IV)

in which A represents the groups $CH_2SR$, in which R denotes a $C_1$-$C_4$ alkyl or hydroxy($C_1$-$C_4$ alkyl) group; $R_{21}$ represents a hydrogen atom.

**21.** Novel compound of the formula:

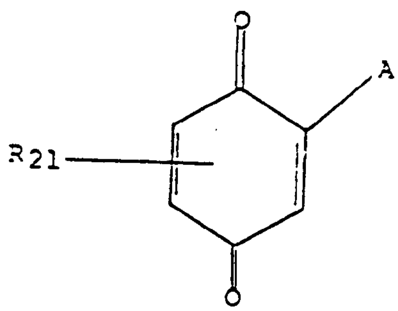

(IV)

in which A represents a group SR, in which R denotes a hydroxy ($C_1$-$C_4$ alkyl) group; $R_{21}$ represents a hydrogen atom, a $C_1$-$C_4$ alkyl radical or the group SR, R having the meaning given above.

**22.** Use of the compound according to Claim 20 or 21, in dyeing compositions for keratinous fibres.

**23.** Process for preparing the compounds of formula (IV), in which A denotes $CH_2SR$ and $R_{21}$ denotes hydrogen, characterised in that the oxidation of a para-aminophenol of formula:

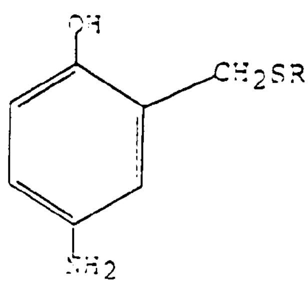

(VII)

is carried out using ferric chloride or ferric sulphate, in which R has the meanings given in Claim 20.

**Claims for the following Contracting State : ES**

**1.** Process for dyeing keratinous fibres characterised in that at least one composition A containing, in a medium suitable for dyeing, at least one mono- or dihydroxyindole is applied to these fibres, the application of the composition A being preceded or followed by the application of a composition B containing, in a medium suitable for dyeing, at least one quinone derivative chosen from ortho- or para-benzoquinones, ortho- or para-benzoquinonemonoimines or diimines, 1,2- or 1,4-naphthoquinones, ortho- or para-benzoquinone sulphonimides, $\alpha,\omega$-alkylene-bis-1,4-benzoquinones or 1,2- or 1,4-naphthoquinone monoimines

or diimines, mono- or dihydroxyindoles, and the quinone derivatives being chosen such that the difference in oxidation-reduction potential $\Delta E$ between the oxidation-reduction potential $E_i$ of the mono- or dihydroxyindoles, determined at pH 7 in a phosphate medium by voltametry using a vitrous carbon electrode and the oxidation-reduction potential Eq of the quinone derivative, determined at pH 7 in a phosphate medium by polarography using a mercury electrode with reference to a saturated calomel electrode, is such that:

$$\Delta E = E_i - E_q \leq 320 \text{ mV}.$$

2. Process according to Claim 1, characterised in that the mono- or dihydroxyindoles are chosen from compounds of the formula:

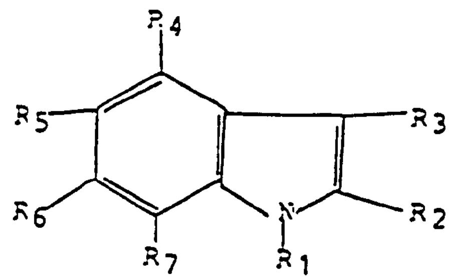

(I)

in which:

$R_1$ denotes hydrogen or a $C_1$-$C_4$ alkyl group;

$R_2$ and $R_3$, which are identical or different, represent a hydrogen atom, a $C_1$-$C_4$ alkyl group, a carboxyl group, a ($C_1$-$C_4$ alkoxy)carbonyl group, a hydroxy($C_1$-$C_4$ alkyl) or an amino($C_1$-$C_4$ alkyl);

$R_4$, $R_5$, $R_6$ and $R_7$, independently of each other, represent a hydrogen atom, a $C_1$-$C_4$ alkyl radical, an amino which is unsubstituted or substituted by one or two $C_1$-$C_4$ alkyl or hydroxy($C_1$-$C_4$ alkyl), ($C_2$-$C_6$ acyl)amino, carboxyl, $C_1$-$C_4$ carboxyalkyl, or ($C_1$-$C_4$ alkoxy)carbonyl groups,

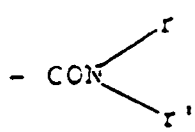

(r and r′, independently of each other, denote hydrogen, $C_1$-$C_4$ alkyl), halogen, hydroxy($C_1$-$C_4$)alkyl, amino($C_1$-$C_4$ alkyl), OH or OZ, Z denoting a linear or branched $C_1$-$C_{20}$ alkyl radical, an aralkyl group, a formyl group, a linear or branched $C_2$-$C_{20}$ acyl group, a linear or branched $C_3$-$C_{20}$ alkenoyl group, a group-$SiR_{11}R_{12}R_{13}$, a group $-P(O)(OR_5)_2$, or a group $R_8OSO_2$–; it being possible for the radicals $R_4$ and $R_5$ or alternatively $R_5$ and $R_6$ or alternatively $R_6$ and $R_7$ to form, together with the carbon atoms to which they are attached, a ring possibly containing a carbonyl group, a thiocarbonyl group, a group $>P(O)(OR_8)$ or a group $>CR_9R_{10}$, provided that at least one of the radicals $R_4$ to $R_7$ represents an OH group;

$R_8$ and $R_9$ represent a hydrogen atom or a $C_1$-$C_4$ alkyl group;

$R_{10}$ represents a $C_1$-$C_4$ alkoxy group or a ($C_1$-$C_4$ mono- or dialkyl )amino group;

$R_{11}$, $R_{12}$ and $R_{13}$ , which are identical or different, represent linear or branched $C_1$-$C_4$ alkyl groups, and the corresponding alkali metal, alkaline-earth metal, ammonium and amine salts of these compounds.

3. Process according to Claim 1 or 2, characterised in that the mono- or dihydroxyindoles are chosen from:
- 4-hydroxyindole
- 4-hydroxy-5-methoxyindole
- 4-hydroxy-5-ethoxyindole
- 5-hydroxyindole
- 2-carboxy-5-hydroxyindole
- 5-hydroxy-6-methoxyindole
- 6-hydroxyindole
- 6-hydroxy-7-methoxyindole

- 5-methoxy-6-hydroxyindole
- 2-carboxy-6-hydroxyindole
- 2-ethoxycarbonyl-6-hydroxyindole
- 7-hydroxyindole
- 2,3-dimethyl-7-hydroxy-4-methoxyindole
- 5,6-dihydroxyindole
- 1-methyl-5,6-dihydroxyindole
- 2-methyl-5,6-dihydroxyindole
- 3-methyl-5,6-dihydroxyindole
- 2,3-dimethyl-5,6-dihydroxyindole
- (5 or 6)-acetoxy-(6 or 5)-hydroxyindole
- 2-ethoxycarbonyl-5,6-dihydroxyindole
- 2-carboxy-5,6-dihydroxyindole
- 2,3-dimethyl-5-hydroxy-6-aminoindole
- 2,3-dimethyl-5-amino-6-hydroxyindole.

**4.** Process according to any one of Claims 1 to 3, characterised in that the quinone derivatives are chosen from the compounds of formulae (II) and (II'):

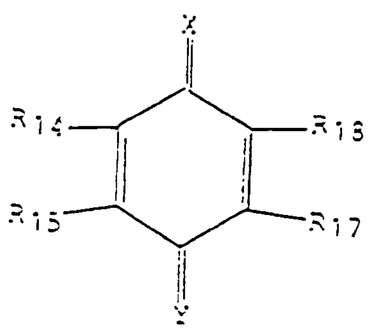

(II)

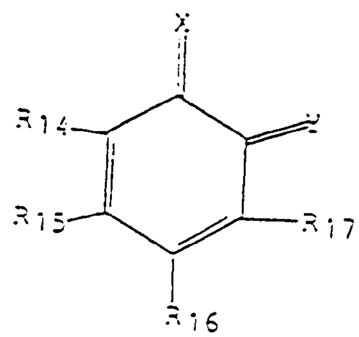

(II')

in which:

X denotes oxygen or a group $NR_{19}$;

Y denotes oxygen or a group $NR_{20}$;

$R_{19}$ and $R_{20}$, which are identical or different, denote hydrogen, halogen, a $C_1$-$C_4$ alkyl or hydroxy($C_1$-$C_4$ alkyl) radical, a ($C_1$-$C_4$ alkyl)sulphonyl radical or a phenylsulphonyl radical;

$R_{14}$, $R_{15}$, $R_{16}$, $R_{17}$ and $R_{18}$ denote, independently of each other, hydrogen, a $C_1$-$C_4$ alkyl radical, a carboxyl, ($C_1$-$C_4$ alkyl)carbonyl, ($C_1$-$C_4$ alkoxy)carbonyl, ($C_1$-$C_4$ alkoxy)methyl, ($C_1$-$C_4$ alkyl)thiomethyl, hydroxy($C_1$-$C_4$ alkyl)thiomethyl or hydroxy($C_1$-$C_4$ alkyl)sulphinyl group,

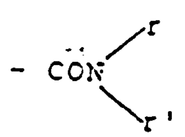

(r and r' denoting, independently of each other, hydrogen, $C_1$-$C_4$ alkyl), a carboxyalkyl, halogen, $C_1$-$C_4$ hydroxy alkyl group, an amino group which is unsubstituted or substituted by one or two $C_1$-$C_4$ alkyl or hydroxy ($C_1$-$C_4$ alkyl) groups, a ($C_2$-$C_6$ acyl)amino group, a group $SO_3M$ where M denotes hydrogen, K or Na, an optionally substituted sulphoxide, sulphone or sulphonamide group, or alternatively a radical $OZ_1$ in which $Z_1$ may be hydrogen, $C_1$-$C_4$ alkyl, hydroxy($C_1$-$C_4$ alkyl), carboxy($C_1$-$C_4$ alkyl), phenyl which is optionally substituted by $C_1$-$C_4$ alkoxy, or alternatively a radical $-SZ_2$ in which $Z_2$ is a $C_1$-$C_4$ alkyl, hydroxy($C_1$-$C_4$ alkyl), dihydroxy($C_2$-$C_4$ alkyl) or carboxy($C_1$-$C_4$ alkyl) group;

it being possible for $R_{14}$ and $R_{15}$ to form, with the carbon atoms to which they are attached, the following cyclic group:

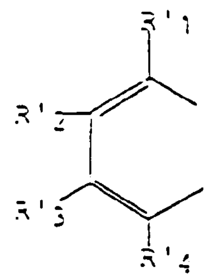

(III)

in which:

$R'_1$, $R'_2$, $R'_3$ and $R'_4$ have the meanings given above for $R_{14}$, $R_{15}$, $R_{16}$, $R_{17}$ and $R_{18}$, when they are not forming a ring.

5. Process according to any one of Claims 1 to 4, characterised in that the quinone derivatives are chosen from benzoquinones of formulae:

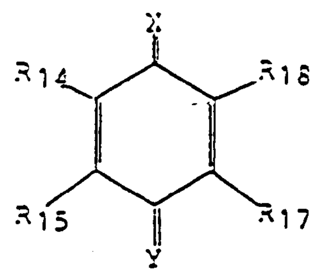

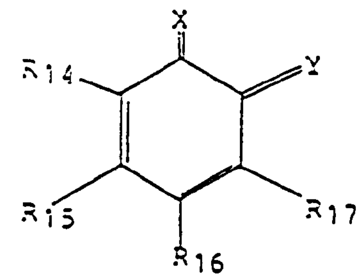

(II) (II')

in which:

$R_{14}$, $R_{15}$, $R_{16}$, $R_{17}$ and $R_{18}$ denote, independently of each other, hydrogen, lower $C_1$-$C_4$ alkyl, lower $C_1$-$C_4$ alkoxy, halogen, $C_2$-$C_6$ acylamino, $SO_3M$, ($C_1$-$C_4$ alkoxy) methyl, carboxy ($C_1$-$C_4$ alkyl), ($C_1$-$C_4$ alkoxy)carbonyl, dialkylamino, $OZ_1$, in which $Z_1$ represents carboxy($C_1$-$C_4$ alkyl), hydroxy($C_1$-$C_4$ alkyl), $SZ_2$, in which $Z_2$ represents hydroxy($C_1$-$C_4$ alkyl), dihydroxy($C_1$-$C_4$ alkyl), carboxy($C_1$-$C_4$ alkyl) or $C_1$-$C_4$ alkyl;

X denotes oxygen or the group $NR_{19}$;

Y denotes oxygen or the group $NR_{20}$, $R_{19}$ and $R_{20}$, independently of each other, denote hydrogen, halogen, lower $C_1$-$C_4$ alkyl, methylsulphonyl or phenylsulphonyl.

6. Process according to any one of Claims 1 to 4, characterised in that the quinone derivatives are chosen from the compounds of formulae:

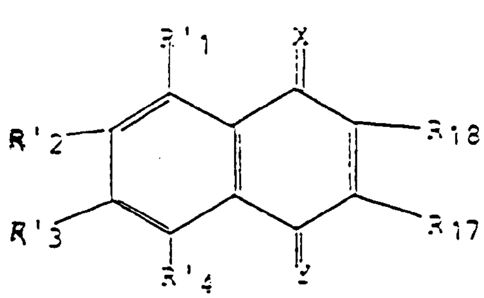

(V)

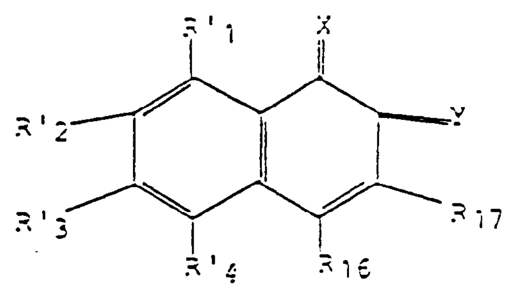

(VI)

in which:

$R'_1$, $R'_2$, $R'_3$, $R'_4$, $R_{16}$, $R_{17}$ and $R_{18}$ have the meanings given in Claims 4 and 5; and
X and Y have the same meanings as those given in Claim 5.

7. Process according to any one of Claims 1 to 5, characterised in that the quinone derivatives are chosen from the following compounds: 1,4-benzoquinone, 2-methoxy-1,4-benzoquinone, 2-methyl-1,4-benzoquinone, 2,6-dimethyl -1,4-benzoquinone, 2,3,5-trichloro-6-methyl- 1,4-benzoquinone,2-acetylamino-1,4-benzoquinone,2-acetylamino -3,5-dimethyl-1,4-benzoquinone, 2,6-dimethyl-5 -acetylamino-1,4-benzoquinone, tetrachloro-1,2-benzoquinone, 2-chloro-1,4-benzoquinone, 2,3-dimethoxy-1-4-benzoquinone, 2-β-carboxyethoxy-1,4-benzoquinone, 2-methoxymethyl-1,4-benzoquinone, 2-β-hydroxyethyl- 1,4-benzoquinone, 2-β-hydroxyethylthio-1,4-benzoquinone, 2,5-bis-β-hydroxyethylthio-1,4-benzoquinone, 2-β,γ-dihydroxypropylthio -1,4-benzoquinone, 2-β-carboxyethylthio-1,4-benzoquinone, 2-carboxymethyl-1,4-benzoquinone, 2-β-hydroxyethylthio-6-methyl-1,4-benzoquinone, 2-methoxycarbonyl -3-methoxy-1,4-benzoquinone, 2-methoxycarbonyl -1,4-benzoquinone, 2-methylthio-1,4-benzoquinone, 2-dimethylamino-1,4-benzoquinone, 2-acetylamino-5 -methoxy-1,4-benzoquinone, 2-(β-hydroxyethylthio)methyl-1,4-benzoquinone, 2-(methylthio)methyl-1,4-benzoquinone, 4,5-dimethoxy-1,2-benzoquinone, 4-methyl-5-chloro- 1,2-benzoquinone, 4,5-dimethyl-1,2-benzoquinone, 2,3-dimethyl -1,4-benzoquinone, 2-β-hydroxyethoxy-1,4-benzoquinone, N-methylsulphonyl-1,4-benzoquinone monoimine, N-phenylsulphonyl-1,4-benzoquinone monoimine, 1,4-naphthoquinone, 1,2-naphthoquinone, 1,2-naphthoquinone-4-sulphonic acid,2,3-dichloro-1,4-naphthoquinone and N-2,6-trichloro-1,4-benzoquinone imine.

8. Process according to any one of Claims 1 to 7, characterised in that the composition A contains 5,6-dihydroxyindole and the composition B contains a quinone derivative chosen from 1,4-benzoquinone, 2-methyl- 1,4-benzoquinone, 2,6-dimethyl-1,4-benzoquinone, 2-methoxy-1,4-benzoquinone, 2-chloro-1,4-benzoquinone, 2,3,5-trichloro-6-methyl-1,4-benzoquinone, 2-acetylamino- 1,4-benzoquinone, 2-acetylamino-3-methoxy-1,4-benzoquinone, 2,6-dimethyl-5-acetylamino-1,4-benzoquinone, 2,3-dimethoxy-1,4-benzoquinone, 2-methoxymethyl- 1,4-benzoquinone, 2-β-hydroxyethyl-1,4-benzoquinone, 2-β,γ-dihydroxypropylthio-1,4-benzoquinone, 2-β-carboxyethylthio -1,4-benzoquinone, 2-carboxymethyl-1,4-benzoquinone, 1,4-naphthoquinone, N-2,6-trichloro-1,4-benzoquinone imine, 1,2-naphthoquinone, 1,2-naphthoquinone-4 -sulphonic acid.

9. Process according to any one of Claims 1 to 8, characterised in that the mono- or dihydroxyindole is present in the composition A in a concentration ranging between 0.01 and 0.3 mol/litre.

10. Process according to any one of Claims 1 to 8, characterised in that the quinone derivative is present in the composition B in concentrations of between 0.005 and 1 mol/litre.

11. Process according to any one of Claims 1 to 10, characterised in that the pH of the compositions A and B is, independently of each other, between 2 and 10.

12. Process according to Claim 11, characterised in that the pH of the composition B is acid.

13. Process according to any one of Claims 1 to 12, characterised in that the compositions A and B comprise an aqueous medium consisting of water or a mixture of water and a solvent.

14. Process according to any one of Claims 1 to 12, characterised in that the compositions A and B consist of an anhydrous solvent medium.

15. Process according to any one of Claims 1 to 14, characterised in that the compositions A and B contain, independently of each other, anionic, cationic, nonionic or amphoteric surface-active agents or mixtures thereof, thickening agents, perfumes, sequestering agents, film-forming agents, treatment agents, dispersing agents, conditioning agents, preserving agents, opacifying agents, keratinous fibre-swelling agents.

16. Process according to any one of Claims 1 to 15, characterised in that the compositions A and/or B contain other dyes chosen from direct dyes, oxidation dyes, couplers or so-called "rapid" oxidation dyes.

17. Process according to any one of Claims 1 to 16, characterised in that the composition A and/or B also contains quinone dyes of the benzoquinone, benzoquinone imine or diimine, naphthoquinone, naphthoquinone imine, naphthoquinone diimine or indole quinone family, whose potentials are such that $\Delta E$ is greater than 320 mV.

18. Multicompartment device or dyeing kit, characterised in that it comprises, in a first compartment, the composition A as defined in any one of Claims 1 to 17, and, in a second compartment, a composition B

as defined in any one of Claims 1 to 17.

**19.** Device according to Claim 18, characterised in that at least one of the two compositions A and B comprise an anhydrous solvent medium and a third compartment containing an aqueous medium for the dyeing and intended to be mixed immediately before use with the content of one or the other of the two compartments containing the anhydrous compositions A and B.

**20.** Process for preparing the compounds of formula (IV):

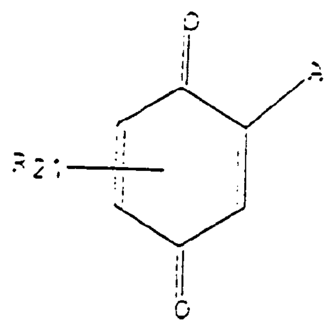

(IV)

in which A denotes $CH_2SR$, $R_{21}$ denotes hydrogen, and R denotes a $C_1$-$C_4$ alkyl or hydroxy($C_1$-$C_4$ alkyl) group, characterised in that the oxidation of a para-aminophenol of formula:

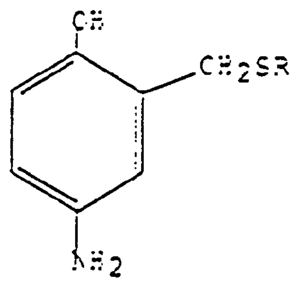

(VII)

is carried out using ferric chloride or ferric sulphate.

21. Process for preparing the compounds of formula (IV):

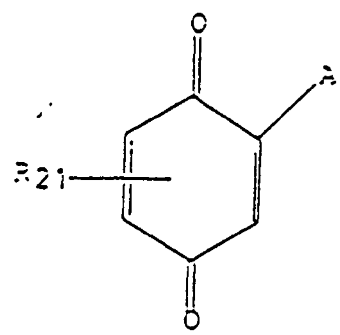

(IV)

in which A denotes SR, $R_{21}$ representing hydrogen, SR or $C_1$-$C_4$ alkyl, and R denotes a hydroxy($C_1$-$C_4$ alkyl) group, characterised in that if $R_{21}$ denotes $C_1$-$C_4$ alkyl or hydrogen:

approximately 2 moles of a quinone derivative of formula:

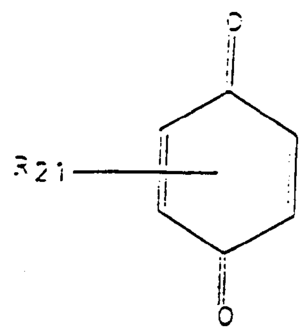

(X)

are reacted with approximately one mole of a thiol of formula RSH,

if $R_{21}$ denotes SR:

approximately 3 moles of a thiol of formula RSH are reacted with approximately 4 moles of p-benzoquinone in an ethanol/water or ethanol medium.

**21.** Process for preparing a multicompartment "kit" intended to be used for dyeing keratinous fibres, characterised in that a composition (A) is prepared by introducing, into a medium suitable for dyeing, at least one mono- or polyhydroxyindole of formula:

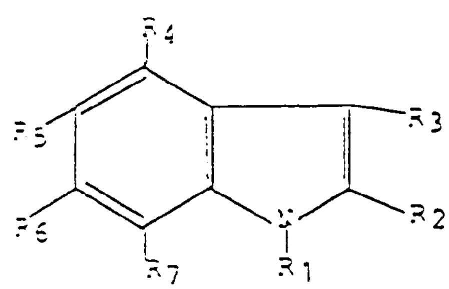

(I)

in which:

$R_1$ denotes hydrogen or a $C_1$-$C_4$ alkyl group;

$R_2$ and $R_3$, which are identical or different, represent a hydrogen atom, a $C_1$-$C_4$ alkyl group, a carboxyl group, a ($C_1$-$C_4$ alkoxy)carbonyl group, a hydroxy($C_1$-$C_4$ alkyl) or an amino($C_1$-$C_4$ alkyl);

$R_4$, $R_5$, $R_6$ and $R_7$, independently of each other, represent a hydrogen atom, a $C_1$-$C_4$ alkyl radical, an amino which is unsubstituted or substituted by one or two $C_1$-$C_4$ alkyl or hydroxy($C_1$-$C_4$ alkyl), ($C_2$-$C_6$ acyl)amino, carboxyl, carboxy($C_1$-$C_4$ alkyl), or ($C_1$-$C_4$ alkoxy)carbonyl groups, a group

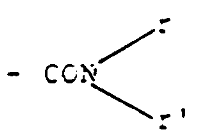

(r and r', independently of each other, denoting hydrogen, $C_1$-$C_4$ alkyl), a halogen, hydroxy($C_1$-$C_4$ alkyl), amino($C_1$-$C_4$ alkyl), OH or OZ groups, Z denoting a linear or branched $C_1$-$C_{20}$ alkyl radical, an aralkyl group, a formyl group, a linear or branched $C_2$-$C_{20}$ acyl group, a linear or branched $C_3$-$C_{20}$ alkenoyl group, a group $-SiR_{11}R_{12}R_{13}$, a group $-P(O)(OR_5)_2$, or a group $R_8OSO_2$-; it being possible for the radicals $R_4$ and $R_5$ or alternatively $R_5$ and $R_6$ or alternatively $R_6$ and $R_7$ to form, together with the carbon atoms to which they are attached, a ring possibly containing a carbonyl group, a thiocarbonyl group, a group $>P(O)(OR_5)$ or a group $>CR_9R_{10}$, provided that at least one of the radicals $R_4$ to $R_7$ represents an OH group;

$R_8$ and $R_9$ represent a hydrogen atom or a $C_1$-$C_4$ alkyl group;

$R_{10}$ represents a $C_1$-$C_4$ alkoxy group or a ($C_1$-$C_4$ mono- or dialkyl)amino group;

$R_{11}$, $R_{12}$ and $R_{13}$, which are identical or different, represent linear or branched $C_1$-$C_4$ alkyl groups,

and

the corresponding alkali metal, alkaline-earth metal, ammonium and amine salts of these compounds, in proportions of between 0.01 and 0.3 mol/litre, this composition (A) being introduced in a first compartment;

in that the composition (B) is prepared by introducing, into a medium suitable for dyeing, at least one quinone derivative chosen from ortho- or para-benzoquinones, ortho- or para-benzoquinonemonoimines or diimines, 1,2- or 1,4-naphthoquinones, ortho- or parabenzoquinonesulphonimides, α,ω-alkylene-bis-1,4-benzoquinones or 1,2- or 1,4-naphthoquinonemonoimines or diimines, mono- or dihydroxyindoles and the quinone derivatives being chosen so that the difference in oxidation-reduction potential ΔE between the oxidation-reduction potential $E_i$ of the mono- or dihydroxyindoles, determined at pH 7 in a phosphate medium by voltametry using a vitrous carbon electrode, and the oxidation-reduction potential Eq of the quinone derivative, determined at pH 7 in a phosphate medium by polarography using a mercury electrode with reference to a saturated calomel electrode, is such that:

$$\Delta E = E_i - E_q \leq 320 \text{ mV}$$

in concentrations of between 0.005 and 1 mol/litre, this composition (B) being introduced in a second compartment.

**Patentansprüche für folgende Vertragsstaaten : AT, BE, CH, DE, FR, GB, GR, IT, LI, NL**

1. Verfahren zum Färben von Keratinfasern, dadurch gekennzeichnet, daß man auf die Fasern wenigstens ein Mittel A aufträgt, welches in einem zum Färben geeigneten Medium wenigstens ein Mono- oder Dihydroxyindol enthält, wobei man das Mittel A vor oder nach Auftragen eines Mittels B anwendet, welches in einem zum Färben geeigneten Medium wenigstens ein Chinonderivat enthält, das ausgewählt ist unter o- und p-Benzochinonen, o- und p-Benzochinonmonoiminen oder -diiminen, 1,2- oder 1,4-Naphthochinonen, o- oder p-Benzochinonsulfonimiden, α,ω-Alkylen-bis-1,4-benzochinonen oder 1,2- oder 1,4-Naphtochinonmonoiminen oder -diiminen, wobei die Mono- oder Dihydroxyindole und die Chinonderivate so ausgewählt werden, daß die Redoxpotentialdifferenz ΔE zwischen dem bei pH 7 in einem Phosphatmilieu mit einer Glaskohlenstoffelektrode durch Voltametrie bestimmten Redoxpotential $E_i$ der Mono- oder Dihydroxyindole und dem bei pH 7 in einem Phosphatmilieu durch Polarographie mit einer Quecksilberelektrode gegen eine gesättigte Kalomelelektrode bestimmten Redoxpotential $E_q$ der Chinonderivate folgender Bedingung genügt:

$$\Delta E = E_i - E_q \leq 320 \text{ mV}.$$

2. Verfahren nach Anspruch 1, dadurch gekennzeichnet, daß die Mono- oder Dihydroxyindole ausgewählt sind unter Verbindungen der Formel:

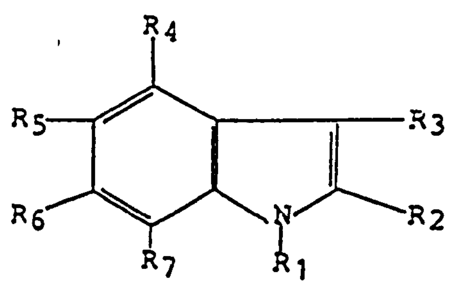

(I)

worin $R_1$ ein Wasserstoffatom oder eine $C_1$-$C_4$-Alkylgruppe bedeutet;

$R_2$ und $R_3$, die gleich oder verschieden sein können, ein Wasserstoffatom, eine $C_1$-$C_4$-Alkylgruppe, Carboxylgruppe, $C_1$-$C_4$-Alkoxycarbonylgruppe, Hydroxy-$C_1$-$C_4$-alkylgruppe oder Amino-$C_1$-$C_4$-alkylgruppe bedeuten;

$R_4$, $R_5$, $R_6$ und $R_7$ unabhängig voneinander für ein Wasserstoffatom, $C_1$-$C_4$-Alkyl, eine gegebenenfalls durch eine oder zwei $C_1$-$C_4$-Alkyl- oder-Hydroxyalkylgruppen substituierte Aminogruppe, $C_2$-$C_6$-Acylamino, Carboxyl, Carboxy-$C_1$-$C_4$-alkyl, $C_1$-$C_4$-Alkoxycarbonyl,

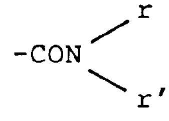

(worin r und r' unabhängig voneinander für ein Wasserstoffatom oder eine $C_1$-$C_4$-Alkylgruppe stehen), Halogen, $C_1$-$C_4$-Hydroxyalkyl, $C_1$-$C_4$-Aminoalkyl, OH oder OZ steht, wobei Z für eine geradkettige oder verzweigte $C_1$-$C_{20}$-Alkylgruppe , Aralkylgruppe, Formylgruppe, geradkettige oder verzweigte $C_2$-$C_{20}$-Acylgruppe, geradkettige oder verzweigte $C_3$-$C_{20}$-Alkenylgruppe, für $-SiR_{11}R_{12}R_{13}$, $-P(O)(OR_8)_2$ oder $R_8OSO_2$- steht, wobei die Reste $R_4$ und $R_5$ oder $R_5$ und $R_6$ oder $R_6$ und $R_7$ zusammen mit den Kohlenstoffatomen, an die sie gebunden sind, einen Ring bilden können, der gegebenenfalls eine Carbonylgruppe, eine Thiocarbonylgruppe, die Gruppen $>P(O)(OR_8)$ oder $>CR_9R_{10}$ enthält, unter der Bedingung, daß wenigstens einer der Reste $R_4$ bis $R_7$ für OH steht;
$R_8$ und $R_9$ ein Wasserstoffatom oder eine $C_1$-$C_4$-Alkylgruppe bedeuten;
$R_{10}$ eine $C_1$-$C_4$-Alkoxygruppe oder eine Mono- oder Di-$C_1$-$C_4$-alkylaminogruppe bedeutet;
$R_{11}$, $R_{12}$ und $R_{13}$, die gleich oder verschieden sein können, eine gradkettige oder verzweigte $C_1$-$C_4$-Alkylgruppe bedeuten und
den entsprechenden Alkalimetall-, Erdalkalimetall-, Ammonium- und Aminsalzen dieser Verbindungen.

**3.** Verfahren nach Anspruch 1 oder 2, dadurch gekennzeichnet, daß die Mono- oder Dihydroxyindole ausgewählt sind unter:
4-Hydroxyindol,
4-Hydroxy-5-methoxyindol,
4-Hydroxy-5-ethoxyindol,
5-Hydroxyindol,
2-Carboxy-5-hydroxyindol,
5-Hydroxy-6-methoxyindol,
6-Hydroxyindol,
6-Hydroxy-7-methoxyindol,
5-Methoxy-6-hydroxyindol,
2-Carboxy-6-hydroxyindol,
2-Ethoxycarbonyl-6-hydroxyindol,
7-Hydroxyindol,
2,3-Dimethyl-7-hydroxy-4-methoxyindol,
5,6-Dihydroxyindol,
1-Methyl-5,6-dihydroxyindol,
2-Methyl-5,6-dihydroxyindol,
3-Methyl-5,6-dihydroxyindol,
2,3-Dimethyl-5,6-dihydroxyindol,
(5 oder 6)-Acetoxy-(6 oder 5)-hydroxyindol,
2-Ethoxycarbonyl-5,6-dihydroxyindol,
2-Carboxy-5,6-dihydroxyindol,
2,3-Dimethyl-5-hydroxy-6-aminoindol,
2,3-Dimethyl-5-amino-6-hydroxyindol.

**4.** Verfahren nach einem der Ansprüche 1 bis 3, dadurch gekennzeichnet, daß die Chinonderivate ausgewählt sind unter Verbindungen der Formeln (II) und (II'):

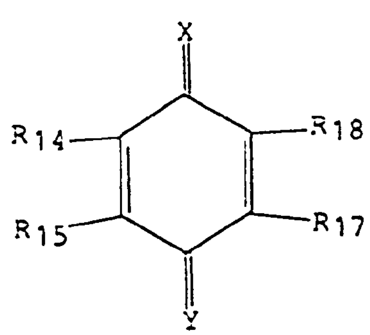

(II)

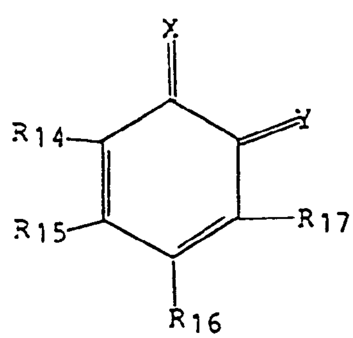

(II')

worin X für ein Sauerstoffatom oder die Gruppe $NR_{19}$ steht;

Y für ein Sauerstoffatom oder die Gruppe $NR_{20}$ steht;

$R_{19}$ und $R_{20}$, die gleich oder verschieden sein können, ein Wasserstoff- oder Halogenatom, eine Alkylgruppe, $C_1$-$C_4$-Hydroxyalkylgruppe, $C_1$-$C_4$-Alkylsulfonylgruppe oder Phenylsulfonylgruppe stehen;

$R_{14}$, $R_{15}$, $R_{16}$, $R_{17}$ und $R_{18}$ unabhängig voneinander für ein Wasserstoffatom, $C_1$-$C_4$-Alkyl, Carboxyl, $C_1$-$C_4$-Alkylcarbonyl, $C_1$-$C_4$-Alkoxycarbonyl, $C_1$-$C_4$-Alkoxymethyl, $C_1$-$C_4$-Alkylthiomethyl, Hydroxy-$C_1$-$C_4$-alkylthiomethyl, Hydroxy-$C_1$-$C_4$-alkylsulfinyl,

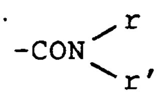

(worin r und r′ unabhängig voneinander für ein Wasserstoffatom oder eine $C_1$-$C_4$-Alkylgruppe stehen), Carboxyalkyl, Halogen, $C_1$-$C_4$-Hydroxyalkyl, eine gegebenenfalls durch eine oder zwei $C_1$-$C_4$-Alkyl- oder -Hydroxyalkylgruppen substituierte Aminogruppe, $C_2$-$C_6$-Acylamino, $SO_3M$, wobei M ein Wasserstoffatom, K oder Na bedeutet, eine Sulfoxid-, Sulfon- oder gegebenenfalls substituierte Sulfonamidgruppe, für $OZ_1$, worin $Z_1$ ein Wasserstoffatom, $C_1$-$C_4$-Alkyl, Hydroxy-$C_1$-$C_4$-alkyl, Carboxy-$C_1$-$C_4$-alkyl, gegebenenfalls durch $C_1$-$C_4$-Alkoxy substituiertes Phenyl bedeutet, oder für $-SZ_2$ steht, worin $Z_2$ für $C_1$-$C_4$-Alkyl, $C_1$-$C_4$-Hydroxyalkyl, $C_2$-$C_4$-Dihydroxyalkyl oder Carboxy-$C_1$-$C_4$-alkyl steht;

$R_{14}$ und $R_{15}$ zusammen mit den Kohlenstoffatomen, an die sie gebunden sind, den folgenden Ring bilden können:

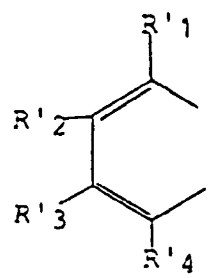

(III)

worin $R'_1$, $R'_2$, $R'_3$ und $R'_4$ die oben für $R_{14}$, $R_{15}$, $R_{16}$, $R_{17}$ und $R_{18}$ angegebenen Bedeutungen besitzen, wenn diese keinen Ring bilden.

**5.** Verfahren nach einem der Ansprüche 1 bis 4, dadurch gekennzeichnet, daß die Chinonderivate ausgewählt sind unter Benzochinonen der Formeln:

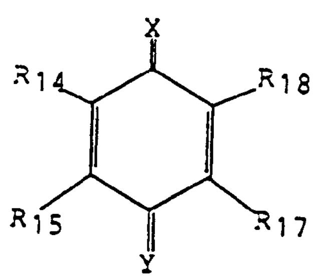

(II)

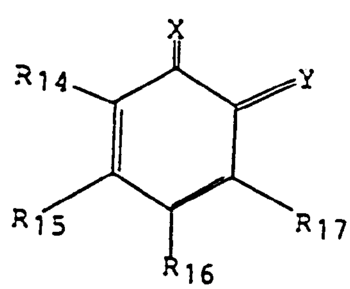

(II')

worin $R_{14}$, $R_{15}$, $R_{16}$, $R_{17}$ und $R_{18}$ unabhängig voneinander für ein Wasserstoffatom, $C_1$-$C_4$-Niedrigalkyl, $C_1$-$C_4$-Niedrigalkoxy, Halogen, $C_2$-$C_6$-Acylamino, $SO_3M$, $C_1$-$C_4$-Alkoxymethyl, Carboxy-$C_1$-$C_4$-alkyl, $C_1$-$C_4$-Alkoxycarbonyl, Dialkylamino, $OZ_1$, worin $Z_1$ Carboxy-$C_1$-$C_4$-alkyl, $C_1$-$C_4$-Hydroxyalkyl bedeutet, oder $SZ_2$ stehen, worin $Z_2$ für $C_1$-$C_4$-Hydroxyalkyl, $C_1$-$C_4$-Dihydroxyalkyl, Carboxy-$C_1$-$C_4$-Alkyl oder $C_1$-$C_4$-Alkyl steht;

X für ein Sauerstoffatom oder die Gruppe $NR_{19}$ steht;

Y für ein Sauerstoffatom oder die Gruppe $NR_{20}$ steht,
$R_{19}$ und $R_{20}$ unabhängig voneinander ein Wasserstoff- oder Halogenatom, $C_1$-$C_4$-Niedrigalkyl, Methylsulfonyl oder Phenylsulfonyl bedeuten.

6. Verfahren nach einem der Ansprüche 1 bis 4, dadurch gekennzeichnet, daß die Chinonderivate ausgewählt sind unter Verbindungen der Formeln:

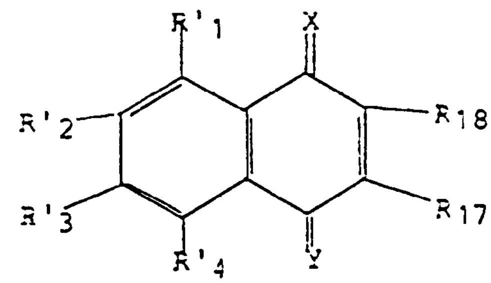

(V)

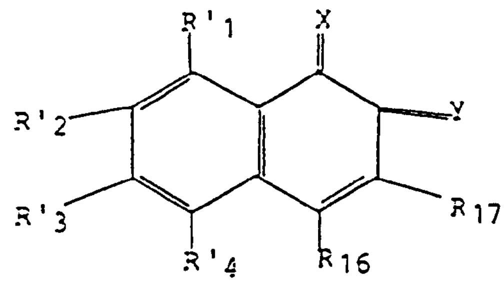

(VI)

worin $R'_1$, $R'_2$, $R'_3$, $R'_4$, $R_{16}$, $R_{17}$ und $R_{18}$ die in den Ansprüchen 4 und 5 angegebenen Bedeutungen besitzen und
X und Y die in Anspruch 5 angegebenen Bedeutungen besitzen.

7. Verfahren nach einem der Ansprüche 1 bis 5, dadurch gekennzeichnet, daß die Chinonderivate ausgewählt sind unter folgenden Verbindungen:
1,4-Benzochinon, 2-Methoxy-1,4-benzochinon, 2-Methyl- 1,4-benzochinon, 2,6-Dimethyl-1,4-benzochinon, 2,3,5-Trichlor -6-methyl-1,4-benzochinon, 2-Acetylamino-1,4-benzochinon, 2-Acetylamino-3,5-dimethyl-1,4-benzochinon, 2,6-Dimethyl-5-acetylamino-1,4-benzochinon, tetrachlor-1,2-benzochinon, 2-Chlor-1,4-benzochinon, 2,3-Dimethoxy- 1,4-benzochinon, 2-β-Carboxyethoxy-1,4-benzochinon, 2-Methoxymethyl-1,4-benzochinon, 2-β-Hydroxyethyl-1,4-benzochinon, 2-β-Hydroxyethylthio-1,4-benzochinon, 2,5-Bis-β-hydroxyethylthio-1,4-benzochinon, 2-β,γ-Dihydroxypropylthio -1,4-benzochinon, 2-β-Carboxyethylthio-1,4-benzochinon, 2-Carboxymethyl-1,4-benzochinon, 2-β-Hydroxyethylthio -6-methyl-1,4-benzochinon, 2-Methoxycarbonyl -3-methoxy-1,4-benzochinon, 2-Methoxycarbonyl- 1,4-benzochinon, 2-Methylthio-1,4-benzochinon, 2-Dimethylamino -1,4-benzochinon, 2-Acetylamino-5-methoxy- 1,4-benzochinon, 2-(β-Hydroxyethylthio)-methyl-1,4-benzochinon, 2-(Methylthio)-methyl-1,4-benzochinon, 4,5-Dimethoxy-1,2-benzochinon, 4-Methyl-5-chlor-1,2-benzochinon, 4,5-Dimethyl-1,2-benzochinon, 2,3-Dimethyl-1,4-benzochinon, 2-β-Hydroxyethoxy-1,4-benzochinon, N-Methylsulfonyl-1,4-benzochinon-monoimin, N-Phenyl-sulfonyl -1,4-benzochinon-monoimin, 1,4-Naphthochinon, 1,2-Naphthochinon, 1,2-Naphthochinon-4-sulfonsäure, 2,3-Dichlor-1,4-naphthochinon, N,2,6-Trichlor-1,4-benzochinonimin.

8. Verfahren nach einem der Ansprüche 1 bis 7, dadurch gekennzeichnet, daß das Mittel A 5,6-Dihydroxyindol enthält und das Mittel B ein Chinonderivat enthält, das ausgewählt ist unter 1,4-Benzochinon, 2-Methyl-1,4-benzochinon 2,6-Dimethyl-1,4-benzochinon, 2-Methoxy- 1,4-benzochinon, 2-Chlor-1,4-benzochinon, 2,3,5-Trichlor -6-methyl-1,4-benzochinon, 2-Acetylamino-1,4-benzochinon, 2-Acetylamino-3-methoxy-1,4-benzochinon, 2,6-Dimethyl -5-acetylamino-1,4-benzochinon, 2,3-Dimethoxy- 1,4-benzochinon, 2-Methoxymethyl-1,4-benzochinon, 2-β-Hydroxyethyl-1,4-benzochinon, 2-β,-γ-Dihydroxypropylthio -1,4-benzochinon, 2-β-Carboxyethylthio-1,4-benzochinon, 2-Carboxymethyl-1,4-benzochinon, 1,4-Naphthochinon, N-2,6-Trichlor-1,4-benzochinonimin, 1,2-Naphthochinon, 1,2-Naphthochinon-4-sulfonsäure.

9. Verfahren nach einem der Ansprüche 1 bis 8, dadurch gekennzeichnet, daß das Mono- oder Dihydroxyindol im Mittel A in einer Konzentration zwischen 0,01 und 0,3 Mol/l enthalten ist.

10. Verfahren nach einem der Ansprüche 1 bis 8, dadurch gekennzeichnet, daß das Chinonderivat im Mittel B in einer Konzentration zwischen 0,005 und 1 Mol/l enthalten ist.

11. Verfahren nach einem der Ansprüche 1 bis 10, dadurch gekennzeichnet, daß der pH-Wert der Mittel A und B unabhängig voneinander zwischen 2 und 10 liegt.

12. Verfahren nach Anspruch 11, dadurch gekennzeichnet, daß der pH-Wert des Mittels B sauer ist.

**13.** Verfahren nach einem der Ansprüche 1 bis 12, dadurch gekennzeichnet, daß die Mittel A und B ein wäßriges Milieu umfassen, das aus Wasser oder einer Mischung von Wasser und einem Lösungsmittel besteht.

**14.** Verfahren nach einem der Ansprüche 1 bis 12, dadurch gekennzeichnet, daß die Mittel A und B in einem wasserfreien Lösungsmittelmilieu vorliegen.

**15.** Verfahren nach einem der Ansprüche 1 bis 14, dadurch gekennzeichnet, daß die Mittel A und B anionische, kationische, nicht-ionische, amphotere Tenside oder Mischungen davon, Verdickungsmittel, Parfüms, Sequestrier-mittel, filmbildende Mittel, Behandlungsmittel, Dispergiermittel, Konditioniermittel, Konservierungsmittel, opakmachende Mittel oder Mittel zur Quellung von Keratinfasern enthalten.

**16.** Verfahren nach einem der Ansprüche 1 bis 15, dadurch gekennzeichnet, daß das Mittel A und/oder B weitere Farbstoffe enthält, die ausgewählt sind unter Direktfarbstoffen, Oxidationsfarbstoffen, Kupplern oder "Schnell"-Oxidationsfarbstoffen.

**17.** Verfahren nach einem der Ansprüche 1 bis 16, dadurch gekennzeichnet, daß das Mittel A und/oder B außerdem Chinonfarbstoffe der Familie der Benzochinone, Benzochinonimine oder -diimine, Naphthochinone, Naphthochinonimine, Naphthochinondiimine oder Indolchinone enthält, deren Potential $\Delta E$ größer als 320 mV ist.

**18.** Vorrichtung aus mehreren Abteilen oder Färbekit, dadurch gekennzeichnet, daß in einem ersten Abteil das Mittel A wie in einem der Ansprüche 1 bis 17 definiert und in einem zweiten Abteil das Mittel B wie in einem der Ansprüche 1 bis 17 definiert, enthalten ist.

**19.** Vorrichtung nach Anspruch 18, dadurch gekennzeichnet, daß wenigstens eines der Mittel A und B ein wasserfreies Lösungsmittelmilieu umfaßt und ein drittes Abteil ein wäßriges Milieu zum Färben enthält, das dazu bestimmt ist, unmittelbar vor Gebrauch mit dem Inhalt eines der beiden Abteile vermischt zu werden, welches die wasserfreien Mittel A und B enthält.

**20.** Neue Verbindungen der Formel:

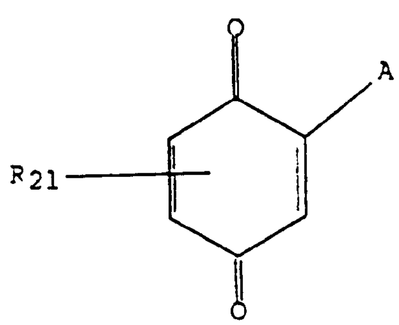

(IV)

worin A die Gruppe $CH_2SR$ bedeutet, wobei R eine $C_1$-$C_4$-Alkyl- oder $C_1$-$C_4$-Hydroxyalkylgruppe bedeutet; und $R_{21}$ ein Wasserstoffatom bedeutet.

**21.** Neue Verbindungen der Formel:

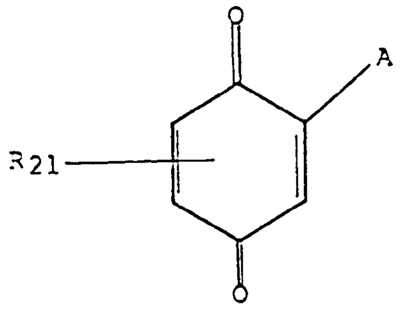

(IV)

worin A die Gruppe SR bedeutet, wobei R eine $C_1$-$C_4$-Hydroxyalkylgruppe bedeutet; $R_{21}$ ein Wasserstoffatom, eine $C_1$-$C_4$-Alkylgruppe oder die Gruppe SR bedeutet, wobei R die oben angegebene Bedeutung besitzt.

22. Verwendung der Verbindungen der Ansprüche 20 oder 21 in Färbemitteln für Keratinfasern.

23. Verfahren zur Herstellung der Verbindungen der Formel (IV), worin A für $CH_2SR$ steht und $R_{21}$ ein Wasserstoffatom bedeutet, dadurch gekennzeichnet, daß man ein p-Aminophenol der Formel:

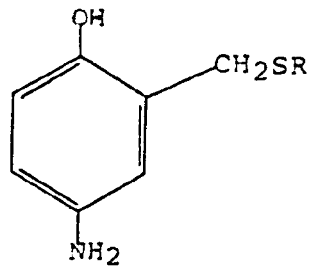

(VII)

worin R die im Anspruch 20 angegebenen Bedeutungen besitzt, mit Eisen(III)-chlorid oder Eisen(III)-sulfat oxidiert.

**Patentanspruche für folgenden Vertragsstaat :ES**

1. Verfahren zum Färben von Keratinfasern, dadurch gekennzeichnet, daß man auf die Fasern wenigstens ein Mittel A aufträgt, welches in einem zum Färben geeigneten Medium wenigstens ein Mono- oder Dihydroxyindol enthält, wobei man das Mittel A vor oder nach Auftragen eines Mittels B anwendet, welches in einem zum Farben geeigneten Medium wenigstens ein Chinonderivat enthält, das ausgewählt ist unter o- und p-Benzochinonen, o- und p-Benzochinonmonoiminen oder -diiminen, 1,2- oder 1,4-Naphthochinonen, o- oder p-Benzochinonsulfonimiden, $\alpha,\omega$-Alkylen-bis-1,4-benzochinonen oder 1,2- oder 1,4-Naphtochinonmonoiminen oder -diiminen, wobei die Mono- oder Dihydroxyindole und die Chinonderivate so ausgewählt werden, daß die Redoxpotentialdifferenz $\Delta E$ zwischen dem bei pH 7 in einem Phosphatmilieu mit einer Glaskohlenstoffelektrode durch Voltametrie bestimmten Redoxpotential Ei der Mono- oder Dihydroxyindole und dem bei pH 7 in einem Phosphatmilieu durch Polarographie mit einer Quecksilberelektrode gegen eine gesättigte Kalomelelektrode bestimmten Redoxpotential $E_q$ der Chinonderivate folgender Bedingung genügt:

$$\Delta E = E_i - E_q \leq 320 \text{ mV}.$$

2. Verfahren nach Anspruch 1, dadurch gekennzeichnet, daß die Mono- oder Dihydroxyindole ausgewählt sind unter Verbindungen der Formel:

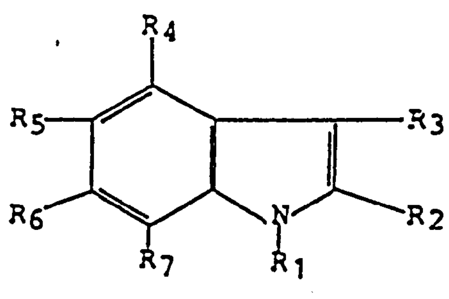

(I)

worin $R_1$ ein Wasserstoffatom oder eine $C_1$-$C_4$-Alkylgruppe bedeutet;
$R_2$ und $R_3$, die gleich oder verschieden sein können, ein Wasserstoffatom, eine $C_1$-$C_4$-Alkylgruppe, Carboxylgruppe, $C_1$-$C_4$-Alkoxycarbonylgruppe, Hydroxy-$C_1$-$C_4$-alkylgruppe oder Amino-$C_1$-$C_4$-alkylgruppe bedeuten;
$R_4$, $R_5$, $R_6$ und $R_7$ unabhängig voneinander für ein Wasserstoffatom, $C_1$-$C_4$-Alkyl, eine gegebenenfalls durch eine oder zwei $C_1$-$C_4$-Alkyl- oder-Hydroxyalkylgruppen substituierte Aminogruppe, $C_2$-$C_6$-Acylamino, Carboxyl, Carboxy-$C_1$-$C_4$-alkyl, $C_1$-$C_4$-Alkoxycarbonyl,

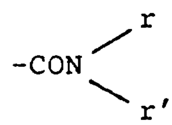

(worin r und r′ unabhängig voneinander für ein Wasserstoffatom oder eine $C_1$-$C_4$-Alkylgruppe stehen), Halogen, $C_1$-$C_4$-Hydroxyalkyl, $C_1$-$C_4$-Aminoalkyl, OH oder OZ steht, wobei Z für eine geradkettige oder verzweigte $C_1$-$C_{20}$-Alkylgruppe, Aralkylgruppe, Formylgruppe, geradkettige oder verzweigte $C_2$-$C_{20}$-Acylgruppe, geradkettige oder verzweigte $C_3$-$C_{20}$-Alkenylgruppe, für $-SiR_{11}R_{12}R_{13}$, $-P(O)(OR_8)_2$ oder $R_8OSO_2$– steht, wobei die Reste $R_4$ und $R_5$ oder $R_5$ und $R_6$ oder $R_6$ und $R_7$ zusammen mit den Kohlenstoffatomen, an die sie gebunden sind, einen Ring bilden können, der gegebenenfalls eine Carbonylgruppe, eine Thiocarbonylgruppe, die Gruppen $>P(O)(OR_8)$ oder $>CR_9R_{10}$ enthält, unter der Bedingung, daß wenigstens einer der Reste $R_4$ bis $R_7$ für OH steht;
$R_8$ und $R_9$ ein Wasseistoffatom oder eine $C_1$-$C_4$-Alkylgruppe bedeuten;
$R_{10}$ eine $C_1$-$C_4$-Alkoxygruppe oder eine Mono- oder Di-$C_1$-$C_4$-alkylaminogruppe bedeutet;
$R_{11}$, $R_{12}$ und $R_{13}$, die gleich oder verschieden sein können, eine gradkettige oder verzweigte $C_1$-$C_4$-Alkylgruppe bedeuten und
den entsprechenden Alkalimetall-, Erdalkalimetall-, Ammonium- und Aminsalzen dieser Verbindungen.

**3.** Verfahren nach Anspruch 1 oder 2, dadurch gekennzeichnet, daß die Mono- oder Dihydroxyindole ausgewählt sind unter:
4-Hydroxyindol,
4-Hydroxy-5-methoxyindol,
4-Hydroxy-5-ethoxyindol,
5-Hydroxyindol,
2-Carboxy-5-hydroxyindol,
5-Hydroxy-6-methoxyindol,
6-Hydroxyindol,
6-Hydroxy-7-methoxyindol,
5-Methoxy-6-hydroxyindol,
2-Carboxy-6-hydroxyindol,
2-Ethoxycarbonyl-6-hydroxyindol,
7-Hydroxyindol,
2,3-Dimethyl-7-hydroxy-4-methoxyindol,
5,6-Dihydroxyindol,
1-Methyl-5,6-dihydroxyindol,
2-Methyl-5,6-dihydroxyindol,
3-Methyl-5,6-dihydroxyindol,
2,3-Dimethyl-5,6-dihydroxyindol,
(5 oder 6)-Acetoxy-(6 oder 5)-hydroxyindol,
2-Ethoxycarbonyl-5,6-dihydroxyindol,
2-Carboxy-5,6-dihydroxyindol,
2,3-Dimethyl-5-hydroxy-6-aminoindol,
2,3-Dimethyl-5-amino-6-hydroxyindol.

**4.** Verfahren nach einem der Ansprüche 1 bis 3, dadurch gekennzeichnet, daß die Chinonderivate ausgewählt sind unter Verbindungen der Formeln (II) und (II′):

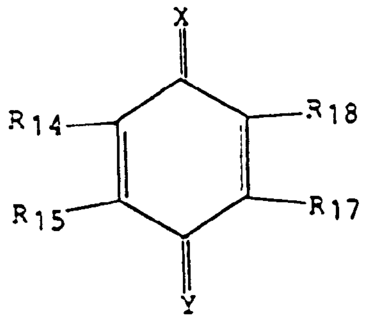

(II)

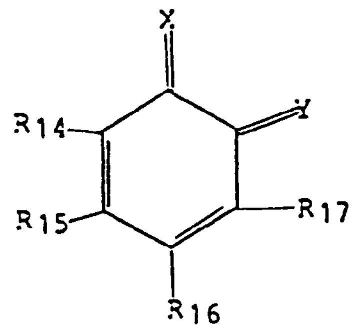

(II')

worin X für ein Sauerstoffatom oder die Gruppe $NR_{19}$ steht;
Y für ein Sauerstoffatom oder die Gruppe $NR_{20}$ steht;
$R_{19}$ und $R_{20}$, die gleich oder verschieden sein können, ein Wasserstoff- oder Halogenatom, eine Alkylgruppe, $C_1$-$C_4$-Hydroxyalkylgruppe, $C_1$-$C_4$-Alkylsulfonylgruppe oder Phenylsulfonylgruppe stehen;
$R_{14}$, $R_{15}$, $R_{16}$, $R_{17}$ und $R_{18}$ unabhängig voneinander für ein Wasserstoffatom, $C_1$-$C_4$-Alkyl, Carboxyl, $C_1$-$C_4$-Alkylcarbonyl, $C_1$-$C_4$-Alkoxycarbonyl, $C_1$-$C_4$-Alkoxymethyl, $C_1$-$C_4$-Alkylthiomethyl, Hydroxy-$C_1$-$C_4$-alkylthiomethyl, Hydroxy-$C_1$-$C_4$-alkylsulfinyl,

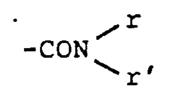

(worin r und r′ unabhängig voneinander für ein Wasserstoffatom oder eine $C_1$-$C_4$-Alkylgruppe stehen), Carboxyalkyl, Halogen, $C_1$-$C_4$-Hydroxyalkyl, eine gegebenenfalls durch eine oder zwei $C_1$-$C_4$-Alkyl- oder -Hydroxyalkylgruppen substituierte Aminogruppe, $C_2$-$C_6$-Acylamino, $SO_3M$, wobei M ein Wasserstoffatom, K oder Na bedeutet, eine Sulfoxid-, Sulfon- oder gegebenenfalls substituierte Sulfonamidgruppe, für $OZ_1$, worin $Z_1$ ein Wasserstoffatom, $C_1$-$C_4$-Alkyl, Hydroxy-$C_1$-$C_4$-alkyl, Carboxy-$C_1$-$C_4$-alkyl, gegebenenfalls durch $C_1$-$C_4$-Alkoxy substituiertes Phenyl bedeutet, oder für $-SZ_2$ steht, worin $Z_2$ für $C_1$-$C_4$-Alkyl, $C_1$-$C_4$-Hydroxyalkyl, $C_2$-$C_4$-Dihydroxyalkyl oder Carboxy-$C_1$-$C_4$-alkyl steht;
$R_{14}$ und $R_{15}$ zusammen mit den Kohlenstoffatomen, an die sie gebunden sind, den folgenden Ring bilden können:

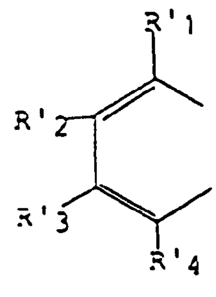

(III)

worin $R'_1$, $R'_2$, $R'_3$ und $R'_4$ die oben für $R_{14}$, $R_{15}$, $R_{16}$, $R_{17}$ und $R_{18}$ angegebenen Bedeutungen besitzen, wenn diese keinen Ring bilden.

**5.** Verfahren nach einem der Ansprüche 1 bis 4, dadurch gekennzeichnet, daß die Chinonderivate ausgewählt sind unter Benzochinonen der Formeln:

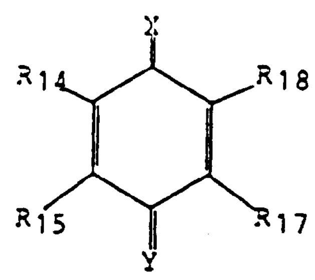

(II)

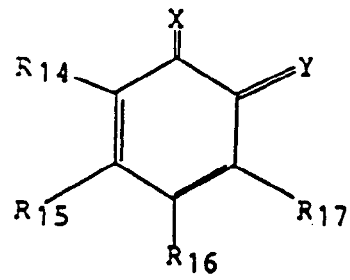

(II')

worin $R_{14}$, $R_{15}$, $R_{16}$, $R_{17}$ und $R_{18}$ unabhängig voneinander für ein Wasserstoffatom, $C_1$-$C_4$-Niedrigalkyl, $C_1$-$C_4$-Niedrigalkoxy, Halogen, $C_2$-$C_6$-Acylamino, $SO_3M$, $C_1$-$C_4$-Alkoxymethyl, Carboxy-$C_1$-$C_4$-alkyl, $C_1$-$C_4$-Alkoxycarbonyl, Dialkylamino, $OZ_1$, worin $Z_1$ Carboxy-$C_1$-$C_4$-alkyl, $C_1$-$C_4$-Hydroxyalkyl bedeutet, oder $SZ_2$ stehen, worin $Z_2$ für $C_1$-$C_4$-Hydroxyalkyl, $C_1$-$C_4$-Dihydroxyalkyl, Carboxy-$C_1$-$C_4$-Alkyl oder $C_1$-$C_4$-Alkyl steht;

X für ein Sauerstoffatom oder die Gruppe $NR_{19}$ steht;

Y für ein Sauerstoffatom oder die Gruppe $NR_{20}$ steht,

$R_{19}$ und $R_{20}$ unabhängig voneinander ein Wasserstoff- oder Halogenatom, $C_1$-$C_4$-Niedrigalkyl, Methylsulfonyl oder Phenylsulfonyl bedeuten.

6. Verfahren nach einem der Ansprüche 1 bis 4, dadurch gekennzeichnet, daß die Chinonderivate ausgewählt sind unter Verbindungen der Formeln:

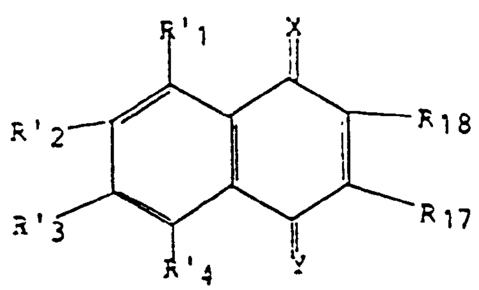

(V)

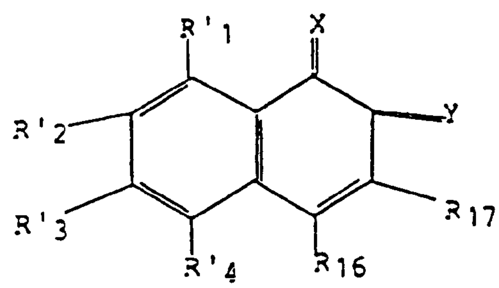

(VI)

worin $R'_1$, $R'_2$, $R'_3$, $R'_4$, $R_{16}$, $R_{17}$ und $R_{18}$ die in den Ansprüchen 4 und 5 angegebenen Bedeutungen besitzen und

X und Y die in Anspruch 5 angegebenen Bedeutungen besitzen.

7. Verfahren nach einem der Ansprüche 1 bis 5, dadurch gekennzeichnet, daß die Chinonderivate ausgewählt sind unter folgenden Verbindungen:

1,4-Benzochinon, 2-Methoxy-1,4-benzochinon, 2-Methyl- 1,4-benzochinon, 2,6-Dimethyl-1,4-benzochinon, 2,3,5-Trichlor -6-methyl-1,4-benzochinon, 2-Acetylamino-1,4-benzochinon, 2-Acetylamino-3,5-dimethyl-1,4-benzochinon, 2,6-Dimethyl-5-acetylamino-1,4-benzochinon, tetrachlor-1,2-benzochinon, 2-Chlor-1,4-benzochinon, 2,3-Dimethoxy- 1,4-benzochinon, 2-β-Carboxyethoxy-1,4-benzochinon, 2-Methoxymethyl-1,4-benzochinon, 2-β-Hydroxyethyl-1,4-benzochinon, 2-β-Hydroxyethylthio-1,4-benzochinon, 2,5-Bis-β-hydroxyethylthio-1,4-benzochinon, 2-β,γ-Dihydroxypropylthio -1,4-benzochinon, 2-β-Carboxyethylthio-1,4-benzochinon, 2-Carboxymethyl-1,4-benzochinon, 2-β-Hydroxyethylthio -6-methyl-1,4-benzochinon, 2-Methoxycarbonyl -3-methoxy-1,4-benzochinon, 2-Methoxycarbonyl- 1,4-benzochinon, 2-Methylthio-1,4-benzochinon, 2-Dimethylamino -1,4-benzochinon, 2-Acetylamino-5-methoxy- 1,4-benzochinon, 2-(β-Hydroxyethylthio)-methyl-1,2-benzochinon, 2-(Methylthio)-methyl-1,4-benzochinon, 4,5-Dimethoxy-1,2-benzochinon, 4-Methyl-5-chlor-1,4-benzochinon, 4,5-Dimethyl-1,2-benzochinon, 2,3-Dimethyl-1,4-benzochinon, 2-β-Hydroxyethoxy-1,4-benzochinon, N-Methylsulfonyl-1,4-benzochinon-monoimin, N-Phenyl-sulfonyl -1,4-benzochinon-monoimin, 1,4-Naphthochinon, 1,2-Naphthochinon, 1,2-Naphthochinon-4-sulfonsäure, 2,3-Dichlor-1,4-naphthochinon, N,2,6-Trichlor-1,4-benzochinonimin.

8. Verfahren nach einem der Ansprüche 1 bis 7, dadurch gekennzeichnet, daß das Mittel A 5,6-Dihydroxyindol enthält und das Mittel B ein Chinonderivat enthält, das ausgewählt ist unter 1,4-Benzochinon, 2-Methyl-1,4-benzochinon, 2,6-Dimethyl-1,4-benzochinon, 2-Methoxy- 1,4-benzochinon, 2-Chlor-1,4-benzochinon, 2,3,5-Trichlor -6-methyl-1,4-benzochinon, 2-Acetylamino-1,4-benzochinon, 2-Acetylamino-3-methoxy-1,4-benzochinon, 2,6-Dimethyl -5-acetylamino-1,4-benzochinon, 2,3-Dimethoxy- 1,4-benzochinon, 2-Methoxymethyl-1,4-benzochinon, 2-β-Hydroxyethyl-1,4-benzochinon, 2-β,-γ-Dihydroxypropylthio - 1,4-benzochinon, 2-β-Carboxyethylthio-1,4-benzochinon, 2-Carboxymethyl-1,4-benzochinon, 1,4-Naphthochinon, N-2,6-Trichlor-1,4-benzochinonimin, 1,2-Naphthochinon, 1,2-Naphthochinon-4-sulfonsäure.

9. Verfahren nach einem der Ansprüche 1 bis 8, dadurch gekennzeichnet, daß das Mono- oder Dihydroxyindol im Mittel A in einer Konzentration zwischen 0,01 und 0,3 Mol/l enthalten ist.

10. Verfahren nach einem der Ansprüche 1 bis 8, dadurch gekennzeichnet, daß das Chinonderivat im Mittel B in einer Konzentration zwischen 0,005 und 1 Mol/l enthalten ist.

11. Verfahren nach einem der Ansprüche 1 bis 10, dadurch gekennzeichnet, daß der pH-Wert der Mittel A und B unabhängig voneinander zwischen 2 und 10 liegt.

12. Verfahren nach Anspruch 11, dadurch gekennzeichnet, daß der pH-Wert des Mittels B sauer ist.

13. Verfahren nach einem der Ansprüche 1 bis 12, dadurch gekennzeichnet, daß die Mittel A und B ein wäßriges Milieu umfassen, das aus Wasser oder einer Mischung von Wasser und einem Lösungsmittel besteht.

14. Verfahren nach einem der Ansprüche 1 bis 12, dadurch gekennzeichnet, daß die Mittel A und B in einem wasserfreien Lösungsmittelmilieu vorliegen.

15. Verfahren nach einem der Ansprüche 1 bis 14, dadurch gekennzeichnet, daß die Mittel A und B anionische,kationische , nicht-ionische, amphotere Tenside oder Mischungen davon, Verdickungsmittel, Parfüms, Sequestrier-mittel, filmbildende Mittel, Behandlungsmittel, Dispergiermittel, Konditioniermittel, Konservïerungsmittel, opakmachende Mittel oder Mittel zur Quellung von Keratinfasern enthalten.

16. Verfahren nach einem der Ansprüche 1 bis 15, dadurch gekennzeichnet, daß das Mittel A und/oder B weitere Farbstoffe enthält, die ausgewählt sind unter Direktfarbstoffen, Oxidationsfarbstoffen, Kupplern oder "Schnell"-Oxidationsfarbstoffen.

17. Verfahren nach einem der Ansprüche 1 bis 16, dadurch gekennzeichnet, daß das Mittel A und/oder B außerdem Chinonfarbstoffe der Familie der Benzochinone, Benzochinonimine oder -diimine, Naphthochinone, Naphthochinonimine, Naphthochinondiimine oder Indolchinone enthält, deren Potential ΔE größer als 320 mV ist.

18. Vorrichtung aus mehreren Abteilen oder Färbekit, dadurch gekennzeichnet, daß in einem ersten Abteil das Mittel A wie in einem der Ansprüche 1 bis 17 definiert und in einem zweiten Abteil das Mittel B wie in einem der Ansprüche 1 bis 17 definiert, enthalten ist.

19. Vorrichtung nach Anspruch 18, dadurch gekennzeichnet, daß wenigstens eines der Mittel A und B ein wasserfreies Lösungsmittelmilieu umfaßt und ein drittes Abteil ein wäßriges Milieu zum Färben enthält, das dazu bestimmt ist, unmittelbar vor Gebrauch mit dem Inhalt eines der beiden Abteile vermischt zu werden, welches die wasserfreien Mittel A und B enthält.

20. Verfahren zur Herstellung der Verbindungen der Formel (IV):

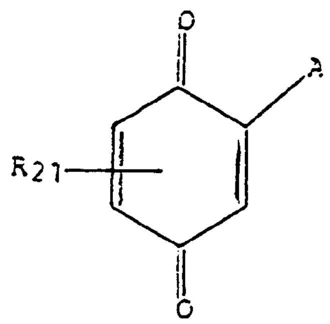

(IV)

worin A für $CH_2SR$ steht, $R_{21}$ ein Wasserstoffatom bedeutet und R für eine $C_1$-$C_4$-Alkylgruppe oder $C_1$-$C_4$-Hydroxyalkylgruppe steht, dadurch gekennzeichnet, daß man ein p-Aminophenol der Formel:

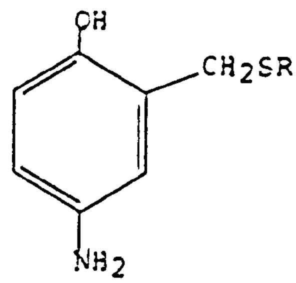

(VII)

mit Eisen(III)-chlorid oder Eisen(III)-sulfat oxidiert.

**21.** Verfahren zur Herstellung der Verbindungen der Formel (IV):

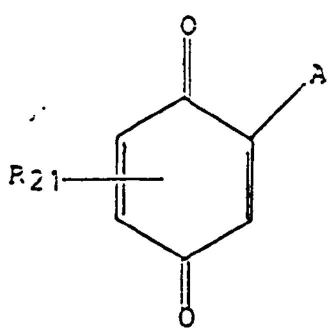

(IV)

worin A für SR steht, $R_{21}$ für ein Wasserstoffatom, SR, oder $C_1$-$C_4$-Alkyl steht und R eine $C_1$-$C_4$-Hydroxyalkylgruppe bedeutet, dadurch gekennzeichnet, daß man wenn $R_{21}$ eine $C_1$-$C_4$-Alkylgruppe oder ein Wasserstoffatom bedeutet:
etwa 2 Mol eines Chinonderivates der Formel:

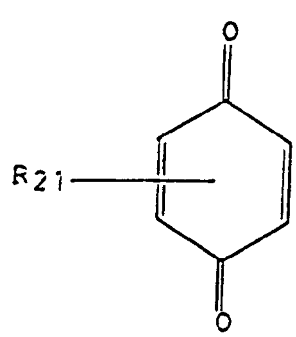

(X)

mit etwa 1 Mol eines Thiols der Formel RSH umsetzt, oder wenn $R_{21}$ für SR steht: etwa 3 Mol eines Thiols der Formel RSH mit etwa 4 Mol p-Benzochinon in Ethanol/Wasser oder Ethanol umsetzt.

**22.** Verfahren zur Herstellung eines "Kits" aus mehreren Abteilen zum Färben von Keratinfasern, dadurch gekennzeichnet, daß man ein Mittel A herstellt, indem man in ein zum Färben geeignetes Milieu wenigstens ein Mono- oder Polyhydroxyindol der Formel:

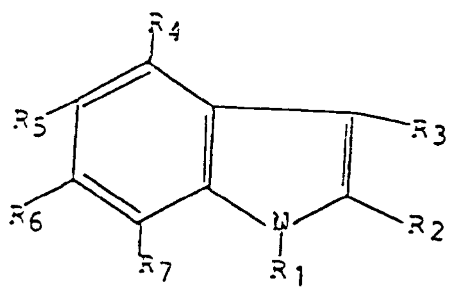

(I)

worin $R_1$ ein Wasserstoffatom oder eine $C_1$-$C_4$-Alkylgruppe bedeutet;
R2 und $R_3$, die gleich oder verschieden sein können, ein Wasserstoffatom, eine $C_1$-$C_4$-Alkylgruppe, Carboxylgruppe, $C_1$-$C_4$-Alkoxycarbonylgruppe, Hydroxy-$C_1$-$C_4$-alkylgruppe oder Amino-$C_1$-$C_4$-alkylgruppe bedeuten;
$R_4$, $R_5$, $R_6$ und $R_7$ unabhängig voneinander für ein Wasserstoffatom, $C_1$-$C_4$-Alkyl, eine gegebenenfalls durch eine oder zwei $C_1$-$C_4$-Alkyl- oder-Hydroxyalkylgruppen substituierte Aminogruppe, $C_2$-$C_6$-Acylamino, Carboxyl, Carboxy-$C_1$ -$C_4$-alkyl, $C_1$-$C_4$-Alkoxycarbonyl,

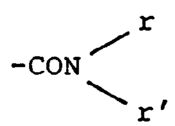

(worin r und r′ unabhängig voneinander für ein Wasserstoffatom oder eine $C_1$-$C_4$-Alkylgruppe stehen), Halogen, $C_1$-$C_4$-Hydroxyalkyl, $C_1$-$C_4$-Aminoalkyl, OH oder OZ steht, wobei Z für eine geradkettige oder verzweigte $C_1$-$C_{20}$-Alkylgruppe, Aralkylgruppe, Formylgruppe, geradkettige oder verzweigte $C_2$-$C_{20}$-Acylgruppe, geradkettige oder verzweigte $C_3$-$C_{20}$-Alkenoylgruppe,für -$SiR_{11}R_{12}R_{13}$, -$P(O)(OR_8)_2$ oder $R_6OSO_2$- steht, wobei die Reste $R_4$ und $R_5$ oder $R_5$ und $R_6$ oder $R_6$ und $R_7$ zusammen mit den Kohlenstoffatomen, an die sie gebunden sind, einen Ring bilden können, der gegebenenfalls eine Carbonylgruppe, eine Thiocarbonylgruppe, die Gruppen >$P(O)(OR_8)$ oder >$CR_9R_{10}$ enthält, unter der Bedingung, daß wenigstens einer der Reste $R_4$ bis $R_7$ für OH steht;
$R_8$ und $R_9$ ein Wasserstoffatom oder eine $C_1$-$C_4$-Alkylgruppe bedeuten;
$R_{10}$ eine $C_1$-$C_4$-Alköxygruppe oder eine Mono- oder Di-$C_1$-$C_4$-alkylaminogruppe bedeutet;
$R_{11}$, $R_{12}$ und $R_{13}$, die gleich oder verschieden sein können, eine gradkettige oder verzweigte $C_1$-$C_4$-Alkylgruppe bedeuten und
den entsprechenden Alkalimetall-, Erdalkalimetall-, Ammonium- und Aminsalzen dieser Verbindungen, in einer Menge von 0,01 bis 0,3 Mol/l gibt und das Mittel A in eines der Abteile gibt;
daß man ein Mittel B herstellt, indem man in ein zum Färben geeignetes Milieu wenigstens ein Chinonderivat gibt, das ausgewählt ist unter o- oder p-Benzochinonen, o- oder p- Benzochinonmonoiminen oder -diiminen, 1,2-oder 1,4-Naphthochinonen, o- oder p-Benzochinonsulfonimiden, α,ω-Alkylen-bis-1,4-benzochinonen oder 1,2- oder 1,4-Naphthochinonmonoiminen oder -diiminen, wobei die Mono- oder Dihydroxyindole und die Chinonderivate so ausgewählt werden, daß die Redoxpotentialdifferenz ΔE zwischen dem bei pH 7 in einem Phosphatmilieu mit einer Glaskohlenstoffelektrode durch Voltametrie bestimmten Redoxpotential $E_i$ der Mono- oder Dihydroxyindole und dem bei pH 7 in einem Phosphatmilieu durch Polarographie mit einer Quecksilberelektrode gegen eine gesättigte Kalomelelektrode bestimmten Redoxpotential $E_q$ der Chinonderivate folgender Bedingung genügt:

$$\Delta E = E_i - E_q \leq 320 \text{ mV},$$

in einer Konzentration zwischen 0,005 und 1 Mol/l gibt, und das Mittel B in das zweite Abteil gibt.